(19) Europäisches Patentamt
European Patent Office
Office européen des brevets

(11) EP 2 121 689 B1

# (12) EUROPÄISCHE PATENTSCHRIFT

(45) Veröffentlichungstag und Bekanntmachung des Hinweises auf die Patenterteilung:
**31.07.2013 Patentblatt 2013/31**

(21) Anmeldenummer: **08707767.3**

(22) Anmeldetag: **19.02.2008**

(51) Int Cl.:
***C07D 471/10*** (2006.01) ***C07D 495/10*** (2006.01)
***C07D 491/107*** (2006.01) ***A61K 31/407*** (2006.01)
***A61P 25/00*** (2006.01)

(86) Internationale Anmeldenummer:
**PCT/EP2008/001270**

(87) Internationale Veröffentlichungsnummer:
**WO 2008/101659 (28.08.2008 Gazette 2008/35)**

## (54) SPIROCYCLISCHE CYCLOHEXAN-DERIVATE

SPIROCYCLIC CYCLOHEXANE DERIVATIVES

DÉRIVÉS DE CYCLOHEXANE SPIROCYCLIQUES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **22.02.2007 DE 102007009235**

(43) Veröffentlichungstag der Anmeldung:
**25.11.2009 Patentblatt 2009/48**

(73) Patentinhaber: **Grünenthal GmbH**
**52078 Aachen (DE)**

(72) Erfinder:
- **SCHUNK, Stefan**
  **52080 Aachen (DE)**
- **ZEMOLKA, Saskia**
  **52066 Aachen (DE)**
- **SAUNDERS, Derek**
  **52072 Aachen (DE)**
- **GRUSS, Michael**
  **52062 Aachen (DE)**
- **GRAUBAUM, Heinz**
  **12557 Berlin (DE)**

(74) Vertreter: **Bülle, Jan**
**Kutzenberger Wolff & Partner**
**Theodor-Heuss-Ring 23**
**50668 Köln (DE)**

(56) Entgegenhaltungen:
**WO-A-2004/043967 WO-A-2006/108565**
**WO-A-2008/009415**

Bemerkungen:
Die Akte enthält technische Angaben, die nach dem Eingang der Anmeldung eingereicht wurden und die nicht in dieser Patentschrift enthalten sind.

EP 2 121 689 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft spirocyclische Cyclohexan-Derivate, Verfahren zu deren Herstellung, Arzneimittel enthaltend diese Verbindungen und die Verwendung von spirocyclischen Cyclohexan-Derivaten zur Herstellung von Arzneimitteln.

Das Heptadekapeptid Nociceptin ist ein endogener Ligand des ORL1(Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den $\mu$, $\kappa$ und $\delta$ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf $\mu$-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen aber gerade im Bereich der Schmerztherapie aber auch anderen der genannten Indikationen Opioidrezeptoren wie der $\mu$-Rezeptor, aber auch die anderen Subtypen dieser Opioidrezeptoren, nämlich $\delta$ und $\kappa$ eine große Rolle. Entsprechend ist es günstig, wenn die Verbindung auch Wirkung an diesen Opioidrezeptoren zeigen.

In WO 2004043967 werden spirocyclische Cyclohexan-Derivate offenbart, die eine hohe Affinität zum ORL1-Rezeptor, aber auch zum $\mu$-Opioid-Rezeptor aufweisen. WO 2004043967 beschreibt generisch auch eine Gruppe, bei denen $R^3$ Alkyl oder Cycloalkyl bedeutet. Es werden jedoch keine Beispielverbindungen offenbart, die Bestandteil dieser Subgruppe sind.

Löslichkeit ist ein wichtige Eigenschaft für die Bioverfügbarkeit und ein entscheidender Faktor bezüglich der Wirksamkeit und damit auch für den Erfolg einer Arzneimittelentwicklung. Um die Löslichkeit zu erhöhen werden aufwändige Prozesse verwendet wie beispielsweise die Herstellung von Mikro- bzw Nanopartikeln (z. B. Exp. Op. Dug Disc. 2007, 2, 145), einfacher und planbarer ist jedoch die Entwicklung von Verbindungen, die bei gleicher Wirksamkeit eine höhere Löslichkeit aufweisen.

Ein Nachteil der in WO 2004043967 offenbarten Beispielverbindungen ist die geringe Löslichkeit der Verbindungen.

Aufgabe der vorliegenden Erfindung war es, Arzneimittel zur Verfügung zu stellen, die auf das Nociceptin/ORL1-Rezeptor-System wirken und eine höhere Löslichkeit aufweisen als die in WO 2004043967 offenbarten Verbindungen.

Überraschenderweise wurde nun gefunden, dass bestimmte Verbindungen, die zwar generisch in WO 2004043967 beschrieben sind, jedoch nicht anhand von Beispielverbindungen offenbart wurden, eine höhere Löslichkeit aufweisen als die dort offenbarten Beispielverbindungen

Gegenstand der Erfindung sind daher spirocyclische Cyclohexan-Derivate der allgemeinen Formel I,

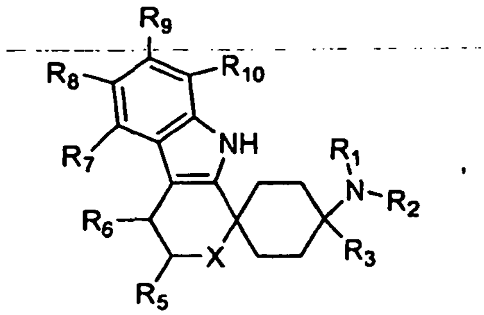

I

worin

$R^1$ und $R^2$, unabhängig voneinander für H; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl, unsubstituiert oder einfach oder mehrfach substituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{11}CH_2CH_2$ oder $(CH_2)_{3-6}$ stehen, wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;

$R^3$ für $C_{1-8}$-Alkyl, jeweils gesättigt oder ungesättigt, verzweigt oder unverzweigt, einfach oder mehrfach substituiert oder unsubstituiert;

$R^5$ für =O; H; $C_{1-5}$-Alkyl, gesättigt oder ungesättigt, verzweigt oder unverzweigt, unsubstituiert oder einfach oder mehrfach substituiert; $COOR^{13}$, $CONR^{13}$, $OR^{13}$; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

oder $R^5$ und $R^6$ gemeinsam $(CH_2)_n$ mit n = 2, 3, 4, 5 oder 6 bedeuten, wobei einzelne Wasserstoffatome auch durch F, Cl, Br, I, $NO_2$, $CF_3$, $OR^{13}$, CN oder $C_{1-5}$-Alkyl ersetzt sein können;

$R^7$, $R^8$, $R^9$ und $R^{10}$ unabhängig voneinander für H, F, Cl, Br, I, $NO_2$, $CF_3$, $OR^{13}$, $SR^{13}$, $SO_2R^{13}$, $SO_2OR^{13}$, $NHC(=O)NR^{14}R^{15}$, $SO_2NR^{14}R^{15}$, CN, $COOR^{13}$, $NR^{14}R^{15}$; $C_{1-5}$-Alkyl, $C_{3-8}$-Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, stehen;

wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substi-

tuiert, bedeutet;

$R^{14}$ und $R^{15}$ unabhängig voneinander H; $C_{1-5}$-Alkyl , jeweils gesättigt oder ungesättigt, verzweigt oder unverzweigt, unsubstituiert oder einfach oder mehrfach substituiert; oder $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeuten;

oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$-Alkyl gesättigt oder ungesättigt, verzweigt oder unverzweigt, unsubstituiert oder einfach oder mehrfach substituiert, bedeutet;

X für O, S, SO, $SO_2$ oder $NR^{17}$ steht;

$R^{17}$ für H; $C_{1-5}$-Alkyl, gesättigt oder ungesättigt, verzweigt oder unverzweigt; $COR^{12}$ oder $SO_2R^{12}$,

wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $OR^{13}$; $NR^{14}R^{15}$ bedeutet;

worin "Alkyl substituiert" oder "Cycloalkyl substituiert" Alkyl oder Cycloalkyl substituiert mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ bedeutet und

"Aryl substituiert" oder "Heteroaryl substituiert" Aryl oder Heteroaryl substituiert mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ bedeutet, mit Ausnahme der Verbindung 2',3',4',9'-Tetrahydro-N,N-dimethyl-4-butyl-spiro[cyclohexan-1,1'(1'H)-pyrido[3,4-b]indol]-4-amin.

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen,

Bei der Zusammenfassung verschiedener Reste, beispielsweise $R^7$, $R^8$, $R^9$ und $R^{10}$ sowie der Zusammenfassung von Resten an deren Substituenten, wie z. B. $OR^{13}$, $SR^{13}$, $SO2R^{13}$ oder $COOR^{13}$, kann ein Substituent, z.B. $R^{13}$, für zwei oder mehrere Reste, beispielsweise $R^7$, $R^8$, $R^9$ und $R^{10}$, innerhalb einer Substanz unterschiedliche Bedeutungen annehmen.

Die erfindungsgemäßen Verbindungen zeigen gute Bindung an den ORL1-Rezeptor, aber auch an andere Opioidrezeptoren.

Die Ausdrücke"$C_{1-8}$-Alkyl" "$C_{1-5}$-Alkyl" und "$C_{1-3}$-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4, 5, 6, 7 oder 8 C-Atomen bzw. mit 1, 2, 3, 4 oder 5 C-Atomen bzw. 1, 2 oder 3 C-Atomen, d.h. $C_{1-8}$-Alkanyle, $C_{2-8}$-Alkenyle und $C_{2-8}$-Alkinyle bzw. $C_{1-5}$-Alkanyle, $C_{2-5}$-Alkenyle und $C_{2-5}$-Alkinyle bzw. $C_{1-3}$-Alkanyle, $C_{2-3}$-Alkenyle und $C_{2-3}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl ($-CH_2CH=CH_2$, $-CH=CH-CH_3$, $-C(=CH_2)-CH_3$), Propinyl ($-CH-C\equiv CH$, $-C\equiv C-CH_3$), 1,1-Dimethylethyl, 1,1-Dimethylpropyl, Butenyl, Butinyl, Pentenyl, Pentinyl, Hexyl, Hexenyl, Hexinyl, Heptyl, Heptenyl, Heptinyl, Octyl, Octenyl oder Octinyl umfaßt. Besonders bevorzugt im Sinne dieser Erfindung sind Methyl, Ethyl, n-Propyl und n-Butyl.

Der Ausdruck "Cycloalkyl" oder "$C_{3-8}$-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. Vorteilhaft ist $C_{3-8}$-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält. Besonders bevorzugt im Sinne dieser Erfindung sind Cyclobutyl, Cyclopentyl und Cyclohexyl.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und

$CH_2$-$CH_2$-$CH_2$-$CH_2$-$CH_2$-$CH_2$- zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit Definitionen von Substituenten bedeutet "Alkyl" "$C_{1-5}$-Alkyl", soweit "Alkyl" nicht näher spezifiziert ist.

Im Zusammenhang mit "Alkyl" und "Cycloalkyl" versteht man unter dem Begriff "substituiert" im Sinne dieser Erfindung die Substitution eines oder mehrerer Wasserstoffreste durch F, Cl, Br, I, -CN, $NH_2$, $N(CH_3)_2$ $NO_2$, SH, OH, $OCH_3$, $OC_2H_5$, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von $CF_3$ oder an verschiedenen Stellen wie im Falle von -CH(OH)-CH=CH-$CHCl_2$. Die Mehrfachsubstitution kann mit dem gleichen oder mit verschiedenen Substituenten erfolgen. Ggf. kann ein Substituent auch seinerseits substituiert sein; so umfaßt - $C_2H_5$ u.a. auch -O-$CH_2$-$CH_2$-O-$CH_2$-$CH_2$-OH. Bevorzugt im Sinne dieser Erfindung ist es, wenn Alkyl oder Cycloalkyl substituiert sind mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$, vorzugsweise F, Cl, Br, I, CN, $CH_3$, $C_2H_5$, $NH_2$, $NO_2$, SH, $CF_3$, OH, $OCH_3$, $OC_2H_5$ oder $N(CH_3)_2$. Ganz besonders bevorzugt ist es, wenn Alkyl oder Cycloalkyl substituiert sind mit OH, $OCH_3$ oder $OC_2H_5$.

In Bezug auf "Aryl" oder "Heteroaryl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, $NH_2$, $N(CH_3)_{2,}$ $NO_2$, $OCH_3$, $OC_2H_{5,}$ CF3; $CH_3$, $C_2H_{5;}$ an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Besonders bevorzugt im Sinne dieser Erfindung ist es, wenn Aryl oder Heteroaryl substituiert sind mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch -

insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Bevorzugt sind Verbindungen der allgemeinen Formel I,
worin

$R^1$ und $R^2$, unabhängig voneinander für H; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl, unsubstituiert oder einfach oder mehrfach substituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;

oder die Reste $R^1$ und $R^2$ zusammen für $CH_2CH_2OCH_2CH_2$, $CH_2CH_2NR^{11}CH_2CH_2$ oder $(CH_2)_{3-6}$ stehen,

wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;

$R^3$ für $C_{1-8}$-Alkyl, jeweils gesättigt oder ungesättigt, verzweigt oder unverzweigt, einfach oder mehrfach substituiert oder unsubstituiert;

$R^5$ für =O; H; $C_{1-5}$-Alkyl, gesättigt oder ungesättigt, verzweigt oder unverzweigt, unsubstituiert oder einfach oder mehrfach substituiert; $COOR^{13}$, $CONR^{13}$, $OR^{13}$; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;

oder $R^5$ und $R^6$ gemeinsam $(CH_2)_n$ mit n = 2, 3, 4, 5 oder 6 bedeuten, wobei einzelne Wasserstoffatome auch durch F, Cl, Br, I, $NO_2$, $CF_3$, $OR^{13}$, CN oder $C_{1-5}$-Alkyl ersetzt sein können;

$R^7$, $R^8$, $R^9$ und $R^{10}$ unabhängig voneinander für

H, F, Cl, Br, I, $NO_2$, $CF_3$, $OR^{13}$, $SR^{13}$, $SO_2R^{13}$, $SO_2OR^{13}$, $NHC(=O)NR^{14}R^{15}$, $SO_2NR^{14}R^{15}$, CN, $COOR^{13}$,$NR^{14}R^{15}$; $C_{1-5}$-Alkyl, $C_{3-8}$-Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, stehen;

wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeutet;

$R^{14}$ und $R^{15}$ unabhängig voneinander H; $C_{1-5}$-Alkyl , jeweils gesättigt oder ungesättigt, verzweigt oder unverzweigt, unsubstituiert oder einfach oder mehrfach substituiert; oder $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeuten;

oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$-Alkyl gesättigt oder ungesättigt, verzweigt oder unverzweigt, unsubstituiert oder einfach oder mehrfach substituiert, bedeutet;

X für O, S, SO, $SO_2$ oder $NR^{17}$ steht;

$R^{17}$ für H; $C_{1-5}$-Alkyl, gesättigt oder ungesättigt, verzweigt oder unverzweigt; $COR^{12}$ oder $SO_2R^{12}$,

wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-8}$-Cycloalkyl oder Heteroaryl, jeweils einfach oder mehrfach substituiert oder unsubstituiert; $OR^{13}$; $NR^{14}R^{15}$ bedeutet;

worin "Alkyl substituiert" oder "Cycloalkyl substituiert" Alkyl oder Cycloalkyl substituiert mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ bedeutet und

"Aryl substituiert oder "Heteroaryl substituiert" Aryl oder Heteroaryl substituiert mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ bedeutet,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen,

mit Ausnahme der Verbindung

2',3',4',9'-Tetrahydro-N,N-dimethyl-4-butyl-spiro[cyclohexan-1,1'(1'H)-pyrido[3,4-b]indol]-4-amin.

Für eine bevorzugte Ausführungsform der erfindungsgemäßen spirocyclischen Cyclohexan-Derivate gilt, dass $R^1$ und $R^2$ unabhängig voneinander für H, $C_{1-8}$-Alkyl, verzweigt oder unverzweigt, gesättigt oder ungesättigt, unsubstituiert oder einfach oder mehrfach substituiert, oder Phenyl oder Benzyl, unsubstituiert oder einfach oder mehrfach substituiert, stehen,
oder gemeinsam für einen Ring stehen und $(CH_2)_{3-6}$ bedeuten,
insbesondere
$R^1$ und $R^2$ unabhängig voneinander für H, Methyl, Ethyl, n-Propyl, oder gemeinsam für $-CH_2CH_2CH_2-$ oder $-CH_2CH_2CH_2CH_2-$ stehen, wobei vorzugsweise nur einer der Reste $R^1$ und $R^2$ H bedeutet.

Besonders bevorzugt stehen $R^1$ und $R^2$ unabhängig voneinander für H, $CH_3$ oder $C_2H_5$, wobei nicht beide Reste $R^1$ und $R^2$ für H stehen,
oder $R^1$ und $R^2$ bilden einen Ring und stehen für $-CH_2CH_2CH_2-$ oder $- CH_2CH_2CH_2CH_2-$.

Ganz besonders bevorzugt stehen $R^1$ und $R^2$ für H oder $CH_3$, wobei $R^1$ und $R^2$ nicht gleichzeitig $CH_3$ bedeuten, insbesondere für $CH_3$.

Bevorzugt sind auch spirocyclische Cyclohexanderivate der allgemeinen Formel I, worin
$R^3$ für Ethyl, n-Propyl, 2-Propyl, Allyl, n-Butyl, iso-Butyl, sec.-Butyl, tert.-Butyl, n-Pentyl, iso-Pentyl, neo-Pentyl, n-Hexyl, steht, jeweils unsubstituiert oder einfach oder mehrfach substituiert mit OH, $OCH_3$ oder $OC_2H_5$.
insbesondere
$R^3$ für Ethyl, n-Propyl, 2-Propyl, Allyl, n-Butyl, iso-Butyl, sec.-Butyl, tert.-Butyl, n-Pentyl, iso-Pentyl, neo-Pentyl, n-Hexyl

steht, jeweils unsubstituiert oder einfach oder mehrfach substituiert mit OH, $OCH_3$ oder $OC_2H_5$.

Besonders bevorzugt sind substituierte Cyclohexanderivate der allgemeinen Formel I, worin $R^3$ Ethyl, n-Propyl oder n-Butyl bedeutet, unsubstituiert oder einfach oder mehrfach substituiert mit $OCH_3$, OH oder $OC_2H_5$, insbesondere mit $OCH_3$.

Für eine bevorzugte Ausführungsform der erfindungsgemäßen spirocyclischen Cyclohexan-Derivate gilt, dass der Rest $R^5$ für H, $CH_3$, COOH, $COOCH_3$, oder $CH_2OH$ steht.

Besonders bevorzugt sind substituierte Cyclohexan-Derivate, worin $R^5$ für H steht..

Bevorzugt sind auch substituierte Cyclohexanderivate der allgemeinen Formel I, worin $R^6$ H; Methyl, Ethyl, $CF_3$, Benzyl oder Phenyl bedeuten kann, wobei der Benzyl- oder Phenylrest mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ substituiert sein kann .

Besonders bevorzugt sind spirocyclische Cyclohexanderivate, worin $R^6$ H bedeutet.

Bevorzugt sind weiterhin spirocyclische Cyclohexan-Derivate, worin $R^7$ $R^8$, $R^9$ und $R^{10}$ unabhängig voneinander H; $C_{1-8}$-Alkyl, verzweigt oder unverzweigt, unsubstituiert oder ein- oder mehrfach substituiert; F, Cl, Br, I, $CF_3$, OH, $OCH_3$, $NH_2$, COOH, $COOCH_3$, $NHCH_3$ Thienyl, Pyrimidinyl, Pyridyl, $N(CH_3)_2$ oder $NO_2$ bedeuten
vorzugsweise
einer der Reste $R^7$ $R^8$, $R^9$ und $R^{10}$ für H; $C_{1-8}$-Alkyl, verzweigt oder unverzweigt, unsubstituiert oder ein- oder mehrfach substituiert; F, Cl, Br, I, OH, $OCH_3$, COOH, $COOCH_3$, $NH_2$, $NHCH_3$ oder $N(CH_3)_2$ oder $NO_2$ steht, während die übrigen Reste H sind,
oder
zwei der Reste $R^7$ $R^8$, $R^9$ und $R^{10}$ unabhängig voneinander für H; $C_{1-5}$-Alkyl, verzweigt oder unverzweigt, unsubstituiert oder ein- oder mehrfach substituiert; F, Cl, Br, I, OH, $OCH_3$, COOH, $COOCH_3$, $NH_2$, $NHCH_3$ oder $N(CH_3)_2$ oder $NO_2$ stehen, während die übrigen Reste H sind.

Besonders bevorzugt sind spirocyclische Cyclohexanderivate, worin $R^7$ $R^8$, $R^9$ und $R^{10}$ für H, F, OH, Cl oder $OCH_3$ stehen.

Verbindungen, bei denen X für O steht, sind ganz besonders bevorzugt. Weiterhin ganz besonders bevorzugt sind Verbindungen der allgemeinen Formel I, X für $NR^{17}$ steht.

Bevorzugt sind dabei spirocyclische Cyclohexan-Derivate, worin $R^{17}$ $COR^{12}$ und $R^{12}$ H; $C_{1-5}$-Alkyl; $C_{3-8}$-Cycloalkyl; oder über $C_{1-3}$-Alkyl gebundenes Aryl, $C_{3-8}$-Cycloalkyl oder Heteroaryl, jeweils einfach oder mehrfach substituiert oder unsubstituiert; $NR^{14}R^{15}$ bedeutet;
insbesondere $R^{12}$ H; Benzyl, Phenethyl, Phenethenyl; jeweils unsubstituiert oder substituiert mit $OCH_3$; $CH_3$, 2,2-Dimethylpropyl oder Cyclopentyl bedeutet.

Ganz besonders bevorzugt sind Verbindungen aus der Gruppe:

4',9'-Dihydro-N,N-dimethyl-4-ethyl-spiro[cyclohexan-1,1'(3'H)-pyrano[3,4-b]indol]-4-amin, 2-hydroxy-1,2,3-propantricarboxylat

6'-Fluor-4',9'-dihydro-N,N-dimethyl-4-ethyl-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-ethyl-spiro[cyclohexan-1,1'(1'H)-pyrido[3,4-b]indol]-4-amin, 2-hydroxy-1,2,3-propantricarboxylat

4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat

6'-Fluor-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat

6'-Fluor-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat

6'-Hydroxy-4',9'-dihydro-N,N-dimethyl-4-butyl-spiro[cyclohexan-1,1'(3'H)-pyrano[3,4-b]indol]-4-amin, 2,2,2-trifluoracetat

6'-Hydroxy-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-butyl-spiro[cyclohexan-1,1'(1'H)-pyrido[3,4-b]indol]-4-amin, 2-hydroxy-1,2,3-propantricarboxylat

2',3',4',9'-Tetrahydro-N,N-dimethyl-4-butyl-2'-methylcarbonyl-spiro[cyclohexan-1,1'(1'H)-pyrido[3,4-b]indol]-4-amin, 2-hydroxy-1,2,3-propantricarboxylat

2',3',4',9'-Tetrahydro-N,N-dimethyl-4-butyl-2'-cyclopentylcarbonyl-spiro[cyclohexan-1,1'(1'H)-pyrido[3,4-b]indol]-4-amin, 2-hydroxy-1,2,3-propantricarboxylat

2',3',4',9'-Tetrahydro-N,N-dimethyl-4-butyl-2'-(2,2)-dimethylpropancarbonyl-spiro[cyclohexan-1,1'(1'H)-pyrido[3,4-b]indol]-4-amin, 2-hydroxy-1,2,3-propantricarboxylat

2',3',4',9'-Tetrahydro-N,N-dimethyl-4-butyl-2'-(3,4-dimethoxybenzylcarbonyl)-spiro[cyclohexan-1,1'(1'H)-pyrido [3,4-b]indol]-4-amin, 2-hydroxy-1,2,3-propantricarboxylat

2',3',4',9'-Tetrahydro-N,N-dimethyl-4-butyl-2'-ethylaminocarbonyl-spiro[cyclohexan-1,1'(1'H)-pyrido[3,4-b]indol]-4-amin, 2-hydroxy-1,2,3-propantricarboxylat

2',3',4',9'-Tetrahydro-N,N-dimethyl-4-butyl-2'-4-methoxybenzylaminocarbonyl-spiro[cyclohexan-1,1'(1'H)-pyrido [3,4-b]indol]-4-amin

2',3',4',9'-Tetrahydro-N,N-dimethyl-4-butyl-2'-methyl-spiro[cyclohexan-1,1'(1'H)-pyrido[3,4-b]indol]-4-amin, 2-hydroxy-1,2,3-propantricarboxylat

6'-Fluor-4',9'-dihydro-N-ethyl-N-methyl-4-butyl-spiro[cyclohexan-1, 1'(3'H)-pyrano[3,4-b]indol]-4-amin, 2-hydroxy-1,2,3-propantricarboxylat

6'-Fluor-4',9'-dihydro-N-benzyl-N-methyl-4-butyl-spiro[cyclohexan-1,1'(3'H)-pyrano[3,4-b]indol]-4-amin

6'-Fluor-4',9'-dihydro-N-phenyl-4-butyl-spiro[cyclohexan-1,1'(3'H)-pyrano[3,4-b]indol]-4-amin

4-Butyl-6'-fluor-4-(*N*-morpholino)-1',3',4',9'-tetrahydrospiro[cyclohexan-1,1'-pyrano[3,4-b]indol]

4-Butyl-6'-fluor-4-(*N*-morpholino)-1',3',4',9'-tetrahydrospiro[cyclohexan-1,1'-pyrano[3,4-b]indol]

4',9'-Dihydro-N,N-dimethyl-4-methoxypropyl-spiro[cyclohexan-1,1'(3'H)-pyrano[3,4-b]indol]-4-amin, 2-hydroxy-1,2,3-propantricarboxylat

6'-Fluor-4',9'-dihydro-N,N-dimethyl-4-methoxypropyl-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-(3-methoxypropyl)--spiro[cyclohexan-1,1'(1'H)-pyrido[3,4-b]indol]-4-amin, 2-hydroxy-1,2,3-propantricarboxylat

4',9'-Dihydro-N,N-dimethyl-4-(4-methoxybutyl)-spiro[cyclohexan-1,1'(3'H)-pyrano[3,4-b]indol]-4-amin, 2-hydroxy-1,2,3-propantricarboxylat

6'-Fluor-4',9'-dihydro-N,N-dimethyl-4-(4-methoxybutyl)-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-(4-methoxybutyl)-spiro[cyclohexan-1,1'(1'H)-pyrido[3,4-b]indol]-4-amin, 2-hydroxy-1,2,3-propantricarboxylat

2',3',4',9'-Tetrahydro-N,N-dimethyl-4-butyl-spiro[cyclohexan-1,1'(1'H)-pyrido[3,4-b]indol]-4-amin, 2-hydroxy-1,2,3-propantricarboxylat

6'-Fluor-4',9'-dihydro-N,N-dimethyl-4-ethyl-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-(3-methoxypropyl)-spiro[cyclohexan-1,1'(1'H)-pyrido[3,4-b]indol]-4-amin, 2-hydroxy-1,2,3-propantricarboxylat 6'-Fluor-4',9'-dihydro-N,N-dimethyl-4-methoxypropyl-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-butyl-2'-ethylaminocarbonyl-spiro[cyclohexan-1,1'(1'H)-pyrido[3,4-b]indol]-4-amin

4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-(4-methoxybutyl)-spiro[cyclohexan-1,1'(1'H)-pyrido[3,4-b]indol]-4-amin, 2-hydroxy-1,2,3-propantricarboxylat

6'-Fluor-4',9'-dihydro-N-benzyl-4-allyl-spiro[cyclohexan-1,1'(3'H)-pyrano[3,4-b]indol]-4-amin, 2-hydroxy-1,2,3-propantricarboxylat

6'-Fluor-4',9'-dihydro-N-phenyl-4-allyl-spiro[cyclohexan-1,1'(3'H)-pyrano[3,4-b]indol]-4-amin

6'-Fluor-4',9'-dihydro-N-(4-methoxybenzyl)-4-allyl-spiro[cyclohexan-1,1'(3'H)-pyrano[3,4-b]indol]-4-amin

N-{6'-Fluor-4',9'-dihydro-4-butyl-spiro[cyclohexane-1,1'(3'H)-pyrano[3,4-b]indol]-4-yl}-pyrrolidin, 2-hydroxy-1,2,3-propantricarboxylat

N-{6'-Fluor-4',9'-dihydro-4-butyl-spiro[cyclohexan-1,1'(3'H)-pyrano[3,4-b]indol]-4-yl}-piperidin

N-{6'-Fluor-4',9'-dihydro-4-butyl-spiro[cyclohexan-1,1'(3'H)-pyrano[3,4-b]indol]-4-yl}-piperidin, 2-hydroxy-1,2,3-propantricarboxylat

N-{6'-Fluor-4',9'-dihydro-4-butyl-spiro[cyclohexan-1,1'(3'H)-pyrano[3,4-b]indol]-4-yl}-n-methylpiperazin, 2-hydroxy-1,2,3-propantricarboxylat

4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat

6'-Hydroxy-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat

2',3',4',9'-Tetrahydro-N,N-dimethyl-4-butyl-2'-(2-phenylethencarbonyl)-spiro[cyclohexan-1,1'(1'H)-pyrido[3,4-b]indol]-4-amin, 2-hydroxy-1,2,3-propantricarboxylat

gegebenenfalls auch als Gemisch.

Die erfindungsgemäßen Substanzen wirken beispielsweise auf den im Zusammenhang mit verschiedenen Erkrankungen relevanten ORL1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes spirocyclisches Cyclohexan-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen spirocyclischen Cyclohexan-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare

Zubereitungsformen können die erfindungsgemäßen spirocyclischen Cyclohexan-Derivate verzögert freisetzen. Die erfindungsgemäßen spirocyclischen Cyclohexan-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens eines erfindungsgemäßen spirocyclischen Cyclohexan-Derivats appliziert.

In einer bevorzugten Form des Arzneimittel liegt ein enthaltenes erfindungsgemäßes spirocyclisches Cyclohexan-Derivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße spirocyclische Cyclohexan-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen spirocyclischen Cyclohexan-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen spirocyclischen Cyclohexan-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes spirocyclisches Cyclohexan-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen spirocyclischen Cyclohexan-Derivats, oder eines erfindungsgemäßen Arzneimittels.

Ein weiterer Gegenstand der Erfindung ist ein Verfahren zur Herstellung der erfindungsgemäßen spirocyclischen Cyclohexan-Derivate wie in der folgenden Beschreibung und Beispielen ausgeführt. Insbesondere geeignet ist dabei ein, Verfahren zur Herstellung eines erfindungsgemäßen spirocyclischen Cyclohexan-Derivats, wobei ein Cyclohexanonderivat der allgemeinen Formel E mit einem Indolderivat der allgemeinen Formel F oder H umgesetzt wird.

Y = OH, SH

Z = O, S

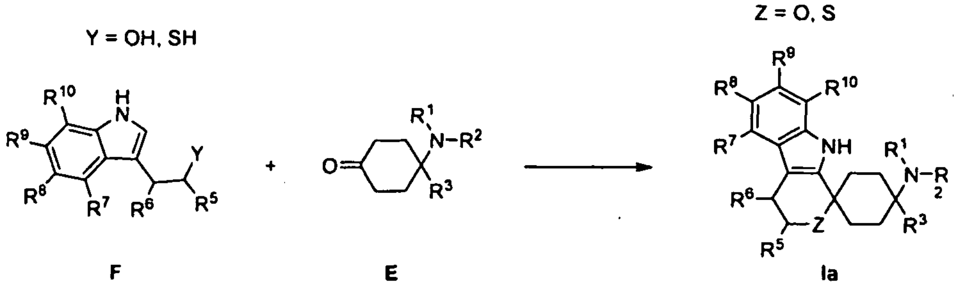

F E Ia

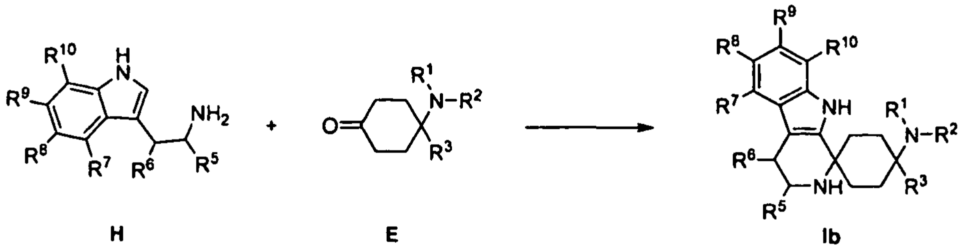

H E Ib

Tryptophole des Typs F (Y = O) können in Reaktionen von Typ der Oxa-Pictet-Spengler-, und Tryptamine des Typs H in Reaktionen von Typ der Pictet-Spengler-Reaktion, mit Ketonen unter Zugabe von mindestens einer geeigneten Reagens aus der Gruppe Säuren, Säureanhydride, Ester oder schwach sauer reagierende Salze oder Lewissäuren unter Bildung von Produkten der Formel I zur Reaktion gebracht werden. Für X = SH erfolgt die Reaktion analog.
Bevorzugt kommt dabei mindestens ein Reagens aus der Gruppe Carbonsäuren, Phosphorsäuren oder Sulfonsäuren oder deren jeweiligen Anhydride, Carbonsäuretrialkylsilylester, sauer reagierende Salze, Mineralsäuren oder Lewissäuren ausgewählt aus der Gruppe bestehend aus Bortrifluorid, Indium(III)chlorid, Titantetrachlorid, Aluminium(III)chlorid, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltrifluormethansulfonats ausgewählt aus der Gruppe bestehend aus Scandium(III)trifluormethansulfonat, Ytterbium (III)trifluormethansulfonat und Indium(III) trifluormethansulfonat, ggf. unter Zusatz von Celite, mit festphasengebundenen Reaktanden oder Reagenzien, bei erhöhter oder erniedrigter Temperatur, mit oder ohne Mikrowelleneinstrahlung, ggf. in einem geeigneten Lösungsmittel oder Lösungsmittelgemisch wie beispielsweise, chlorierten oder unchlorierten, vorzugsweise aromatischen, Kohlenwasserstoffen, Acetonitril; in etherischen Lösungsmitteln, vorzugsweise in Diethylether oder THF;oder in Nitromethan, in geeigneten Fällen auch in Alkoholen oder Wasser, zum Einsatz.
Besonders bevorzugt werden dabei Pyridinium-para-Toluolsulfonat, Phosphorpentoxid in Gegenwart von Celite, Bortrifluorid-etherat, Trifluoressigsäure, Orthotitansäure-tetraisopropylester zusammen mit Trifluoressigsäure, Trifluormethansulfonsäuretrimethylsilylester, Trifluormethansulfonsäure, Methansulfonsäure, Trifluoressigsäure, Essigsäure, Phosphorsäure, Polyphosphorsäure, Polyphosphatester, p-Toluolsulfonsäure, Salzsäure HCl-Gas, Schwefelsäure zusammen mit Acetatpuffer, Zinntetrachlorid, eingesetzt.

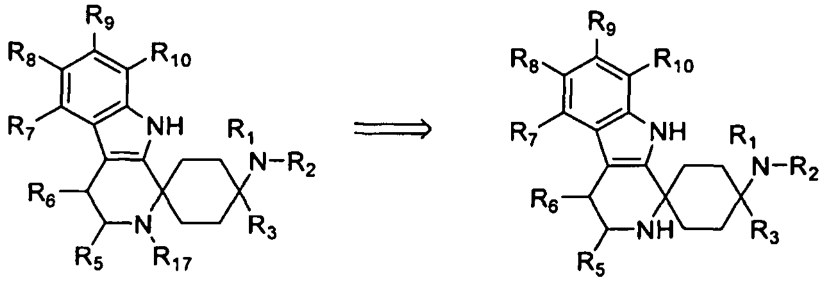

L/M/N Ib

Sekundäre Amine des Typs I können nach dem Fachmann bekannten Verfahren zu Verbindungen des Typs **L/M/N** acyliert, sulfonyliert oder carbamoyliert werden. Bevorzugt werden diese Reaktionen bei erhöhter Temperatur, besonders bevorzugt unter Mikrowelleneinstrahlung, durchgeführt.
Eine solche, dem Fachmann bekannte Methode kann die Umsetzung mit einem Anhydrid oder einem Säurechlorid unter Zugabe einer Base, beispielsweise Triethylamin, darstellen.

**Synthese der Ketonbausteine**

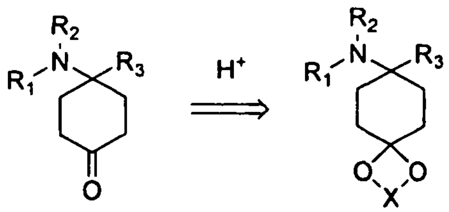


E

C oder deren Salze mit Säuren D

Verbindungen der Formel E können aus entsprechenden Acetalen C, oder aus deren Salzen D, nach dem Fachmann bekannten Methoden durch Entschützen mittels Säuren freigesetzt werden. Dabei ist X aus der Gruppe Alkyl, Alkyl/ Alkyliden/ mit Aryl oder Alkyl (gesättigt/ungesättigt) substituiertem Alkyliden ausgewählt

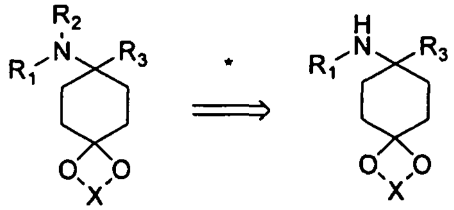

Ca Cb

Aminoacetale Cb mit maximal einem Substituenten am Stickstoffatom können nach dem Fachmann bekannten Verfahren, z.B. durch reduktive Aminierung, in entsprechende Amino-acetale Ca mit einem oder zwei weiteren Substituenten am Stickstoff überführt werden.

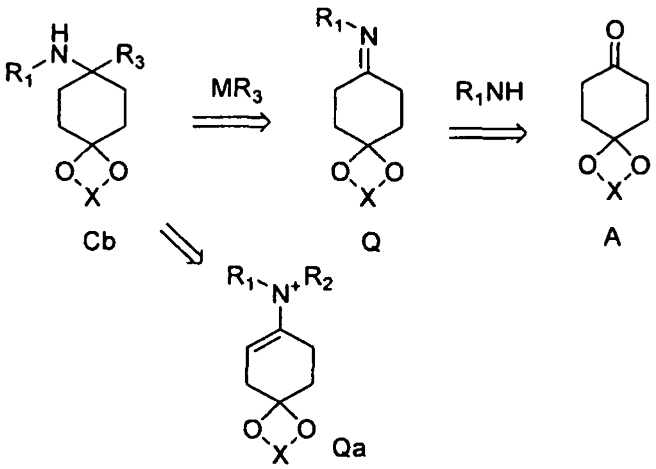


Cb Q A

Qa

Aminoacetale Cb mit maximal einem Substituenten am Stickstoffatom können nach dem Fachmann bekannten Verfahren durch Addition von Kohlenstoff-Nucleophilen an Imine **Q** erhalten werden, vorzugsweise Organometallverbindungen in inerten Lösungsmitteln, besonders bevorzugt mit Grignardreagenzien oder Organolithiumverbindungen, bevorzugt in Ethern, bevorzugt bei Temperaturen von -100 bis Raumtemperatur.

Aminoacetale **C** mit zwei Substituenten am Stickstoffatom können auch nach dem Fachmann bekannten Verfahren durch Addition von Kohlenstoff-Nucleophilen an Salze von Enaminen **Qa** erhalten werden, vorzugsweise mit Organometallverbindungen in inerten Lösungsmitteln.

Die Herstellung von Iminen ist aus der Literatur bekannt.

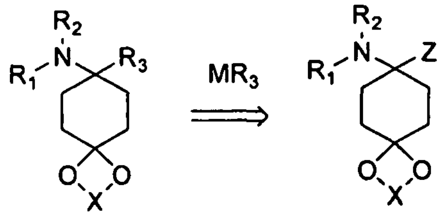


Ca/b B

Acetale **C** können auch durch Substitution von geeigneten Abgangsgruppen **Z** in Strukturen der Formel **B** erhalten werden. Geeignete Abgangsgruppen sind bevorzugt Cyanogruppen; 1,2,3-Triazol-1-yl-Gruppen. Weitere geeignete Abgangsgruppen sind 1*H*-Benzo[d][1,2,3]triazol-1-yl-Gruppen und Pyrazol-1-yl-Gruppen (Katritzky et al., Synthesis 1989, 66-69).
Ein besonders bevorzugter Weg zu Verbindungen der Struktur **C** ist die Umsetzung von Aminonitrilen **B** mit entsprechenden Organometallverbindungen, vorzugsweise Grignardverbindungen, vorzugsweise in Ethern, vorzugsweise bei Raumtemperatur. Die Organometallverbindungen sind entweder käuflich erhältlich oder können nach bekannten Verfahren hergestellt werden.
Ein weiterer besonders bevorzugter Weg zu Verbindungen der Struktur **C** ist die Umsetzung von Aminotriazolen **B** mit entsprechenden Organometallverbindungen, vorzugsweise Grignardverbindungen, vorzugsweise in Ethern, vorzugsweise bei Raumtemperatur.
Die Organometallverbindungen sind entweder käuflich erhältlich oder können nach literaturbekannten Methoden hergestellt werden.

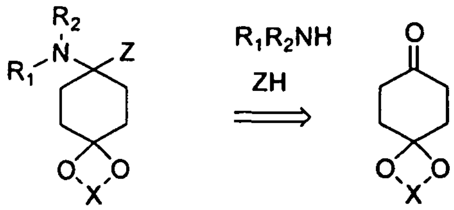


B A

Strukturen der Formel **B** lassen sich durch Reaktion von Ketonen **A** mit Aminen und aciden Reaktanden Z-H herstellen. Geeignete Reaktanden Z-H sind z. B. Cyanwasserstoff, 1,2,3-Triazol, Benzotriazol oder Pyrazol.
Ein besonders bevorzugter Weg zu Verbindungen der Struktur **B** ist die Umsetzung von Ketonen mit Metallcyaniden und dem entsprechenden Amin in Gegenwart von Säure, vorzugsweise in einem Alkohol, bei Temperaturen von - 40 bis 60 °C, vorzugsweise bei Raumtemperatur mit Alkalimetallcyaniden in Methanol.
Ein weiterer besonders bevorzugter Weg zu Verbindungen der Struktur **B** ist die Umsetzung von Ketonen mit 1,2,3-Triazol und dem entsprechenden Amin unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich **B** analoge Strukturen mit Benzotriazol- oder Pyrazol- statt Triazolgruppen einführen.
Verbindungen der allgemeinen Formeln F und H sind entweder kommerziell erhältlich, oder ihre Herstellung ist aus dem Stand der Technik bekannt oder in für den Fachmann offensichtlicher Weise aus dem Stand der Technik ableitbar. Insbesondere relevant sind hierfür die folgenden Zitate: Jirkovsky et al., J. Heterocycl. Chem., 12, 1975, 937-940; Beck et al., J. Chem. Soc. Perkin 1, 1992, 813-822; Shinada et al., Tetrahedron Lett., 39, 1996, 7099-7102; Garden et al., Tetrahedron, 58, 2002, 8399-8412; Lednicer et al., J. Med. Chem., 23, 1980, 424-430; Bandini et al. J. Org. Chem. 67, 15; 2002, 5386 - 5389; Davis et al., J.Med.Chem. 35, 1, 1992, 177-184; Yamagishi et al., J.Med.Chem. 35, 11, 1992, 2085-2094; Gleave et al.; Bioorg.Med.Chem.Lett. 8, 10, 1998, 1231-1236; Sandmeyer, Helv.Chim.Acta; 2; 1919; 239; Katz et al.; J. Med. Chem. 31, 6, 1988; 1244-1250; Bac et al. Tetrahedron Lett. 1988, 29, 2819; Ma et al. J. Org. Chem. 2001, 66, 4525; Kato et al. J. Fluorine Chem. 99, 1, 1999, 5 - 8.

**Untersuchungen zur Löslichkeit**

Die Untersuchungen zur Löslichkeit wurden anhand von fünf erfindungsgemäßen Verbindungen und fünf Beispielverbindungen durchgeführt. Die Daten wurden anhand einer Serie von Verbindungen erhoben, die, abgesehen von

dem Rest an $R^3$, große Gemeinsamkeiten aufweisen, damit Vergleichbarkeit gewährleistet ist.
Es zeigte sich, dass Verbindungen, die an $R^3$ einen Alkylrest tragen, deutlich besser löslich sind als die Verbindungen, die an $R^3$ einen Phenyl- oder Thienylrest tragen. Überraschenderweise bewirkt gerade diese Strukturvariation eine Erhöhung der Löslichkeit. Die Einführung einer OH-Gruppe an $R^8$, eine typische Derivatisierung (Metabolisierung), die vom lebenden Organismus zur Erhöhung der Löslichkeit vorgenommen wird, um eine Verbindung über die Niere auszuscheiden, führte nicht zu einer vergleichbaren Erhöhung der Löslichkeit (Verbindungen V-4 und V-5).

**Beispiele**

Die folgenden Beispiele dienen zur näheren Erläuterung der Erfindung, schränken aber den allgemeinen Erfindungsgedanken nicht ein.
Die Ausbeuten der hergestellten Verbindungen sind nicht optimiert.
Alle Temperaturen sind unkorrigiert.
Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.
Als stationäre Phase für die Säulenchromatographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt.
Die Mischungsverhältnisse von Laufmitteln für chromatographische Untersuchungen sind stets in Volumen/ Volumen angegeben.

**Ketone**

**Baustein B-1:**

**8-Dimethylamino-1,4-dioxa-spiro[4.5]decan-8-carbonitril (B-1)**

Zu einem Gemisch aus 4N Salzsäure (50 ml) und Methanol (30 ml) wurde unter Eiskühlung 40-proz. wässrige Dimethylamin-Lösung (116 ml, 0.92 mol), Cyclohexan-1,4-dion-monoethylenketal (30.0 g, 0.192 mol) und Kaliumcyanid (30.0 g, 0.46 mol) hinzugefügt. Die Mischung wurde 72 h bei Raumtemperatur gerührt und anschließend nach der Zugabe von Wasser (80 ml) mit Ether (4 x 100 ml) extrahiert. Nach dem Einengen der Lösung wurde der Rückstand in Dichlormethan (200 ml) aufgenommen und mit Magnesiumsulfat über Nacht getrocknet. Die organische Phase wurde eingeengt und das Ketal **B-1** als weißer Feststoff erhalten.
*Ausbeute:* 38.9 g (96 %)
*Schmelzpunkt:* 86-88°C
*$^1$H-NMR (DMSO-$d_6$):* 1.57 (2 H, m); 1.72 (2 H; m); 1.85 (2 H, m); 1.99 (2 H, m); 2.25 (6 H, s); 3.87 (4 H, m).
*$^{13}$C-NMR (DMSO-$d_6$):* 30.02; 31.32; 60.66; 63.77; 106.31; 118.40.

**Baustein B-2:**

**8-(Ethylmethylamino)-1,4-dioxaspiro[4.5]decan-8-carbonitril (B-2)**

Zu einem Gemisch aus 4 *N* Salzsäure (15 ml, 60 mmol) und Methanol (10 ml) wurden unter Eiskühlung zuerst Ethylmethylamin (16.0 g, 23 ml, 262 mmol) und Wasser (10 ml), danach 1,4-Dioxaspiro[4.5]deca-8-on (9.40 g, 60 mmol) sowie Kaliumcyanid (9.20 g, 141 mmol) gegeben. Die Reaktionsmischung wurde 5 d bei Raumtemperatur gerührt. Anschließend wurde Wasser (100 ml) zugegeben und die Lösung mit Diethylether (5 $\times$ 50 ml) extrahiert. Die vereinigten organischen Phasen wurden mit Natriumsulfat getrocknet und i. Vak. eingeengt.
Ausbeute: 10.8 g (80 %), gelbes Öl
$^1$H-NMR (DMSO-$d_6$): 1.04 (t, 3H, J = 7.1 Hz); 1.50-1.59 (m, 2H); 1.68-1.77 (m, 2H); 1.89-1.95 (m, 2H); 1.98-2.06 (m, 2H); 2.23 (s, 3H); 2.42-2.48 (m, 2H, überlagert vom DMSO-Signal); 3.87 (s, 4H).

**Baustein E-1:**

*Dieser Baustein wurde anstatt des gewünschten Zielproduktes erhalten. Es ist offensichtlich, dass **D-1** auch gezielt aus Erthylmagnesiumbromid und **B-1** hergestellt werden kann.*

**(8-Ethyl-1,4-dioxa-spiro[4.5]dec-8-yl)-dimethyl-amin (D-1)**

Eine Mischung von Ethylbromid (30.0 g, 0.3 mol) und 3-Brompyridin (16.0 g, 0.1 mol) wurde tropfenweise zu Magnesiumpulver (10.0 g) in Diethylether (50 ml) gegeben. Nach dem die Grignardbildung abgeschlossen war, wurde die graue Lösung bei 0 °C innerhalb von 15 min mit Aminonitril **B-1** (10.5 g, 47.6 mmol) in THF (80 ml) versetzt und die Reaktionslösung über Nacht bei Raumtemperatur gerührt. Anschließend wurde die Reaktionslösung unter Eiskühlung mit 20 %iger Ammoniumchlorid-Lösung (50 ml) und Wasser (50 ml) versetzt. Die Reaktionslösung wurde mit Diethylether (100 ml) verdünnt, die org. Phase abgetrennt und die wässr. Phase 2 x mit $Et_2O$ (100 ml) extrahiert. Die vereinten org. Phasen wurden mit Wasser (50 ml) und NaCl-Lösung (50 ml) gewaschen, über $Na_2SO_4$ getrocknet, filtriert und das Solvens i. Vak. entfernt. Der Rückstand wurde in 2-Butanon (200 ml) aufgenommen und bei 0 °C mit $Me_3SiCl$ (10 ml) versetzt. Die Reaktionslösung wurde unter Luftfeuchtigkeit 5 h gerührt und der ausgefallene Feststoff abgesaugt.
*Ausbeute:* 6.8 g (64 %), hellbrauner Feststoff
*$^1$H-NMR (DMSO-$d_6$):* 0.94 (3 H, t); 1.51-1.60 (2 H, m); 1.77-1.86 (8 H, m); 2.64 (6 H, 2 s); 3.83-3.89 (4 H, m).

**4-Dimethylamino-4-ethyl-cyclohexanon (E-1)**

Das Hydrochlorid **D-1** (6.67 g, 0.026 mmol) wurde in 6N HCl (40 ml) gelöst und bei Raumtemperatur über Nacht gerührt. Die Reaktionsmischung wurde zweimal mit Diethylether (100 ml) extrahiert. Anschließend wurde unter Eiskühlung mit 5N NaOH alkalisch gestellt und erneut dreimal mit $Et_2O$ (100 ml) extrahiert. Die vereinten org. Phasen wurden über $NaSO_4$ getrocknet, filtriert und das Solvens i. Vak. entfernt.
*Ausbeute:* 4.16 g (92 %), braunes Öl
*$^1$H-NMR (DMSO-$d_6$):* 0.81 (3 H, t); 1.43-1.50 (2 H, q); 1.67-1.89 (2 H, m); 1.83-1.89 (2 H, m); 1.99-2.06 (2 H, m); 2.22 (6 H, 2 s); 2.39-2.43 (4 H, m).
*$^{13}$C-NMR (DMSO-$d_6$):* 8.71; 21.99; 30.41; 36.17; 37.07; 38.66; 55.53; 210.57.

<u>**Baustein E-2:**</u>

*Variante 1:*

**(8-Butyl-1,4-dioxa-spiro[4.5]dec-8-yl)-dimethyl-amin Hydrochlorid (D-2)**

8-Dimethylamino-1,4-dioxa-spiro[4.5]decan-8-carbonitril **B-1** (10.5 g, 50 mmol) wurde in THF (150 ml) unter Eiskühlung und Argon vorgelegt. Innerhalb von 15 min wurde 2M Butyl-magnesiumchlorid in THF (62.5 ml, 125 mmol) zugetropft und 16 h bei Raumtemperatur gerührt.
Der Ansatz wurde unter Eiskühlung mit 20 %-iger Ammoniumchlorid-Lösung (37 ml) und Wasser (50 ml) versetzt und mit Ether (3 x 50 ml) extrahiert. Die org. Phase wurde mit Wasser (1 x 50 ml) und gesättigte Natriumchlorid-Lösung (1 x 50 ml) gewaschen, die organische Phase mit $Na_2SO_4$ getrocknet und i. Vak. eingeengt. Das Rohprodukt (2.05 g) wurde in Ethylmethylketon (75 ml) gelöst, unter Eiskühlung mit $ClSiMe_3$ (9.5 ml, 75 mmol) versetzt und 6 h bei Raumtemperatur gerührt. Der ausgefallene weiße Niederschlag wurde abgesaugt und i. Vak. getrocknet.
*Ausbeute:* 3.1 g (22 %)
*$^1$H-NMR (DMSO-$d_6$):* 0.91 (3 H, t); 1.31 (4 H, m); 1.56 (2 H, m); 1.75 (8 H, m); 2.64 (6 H, s); 3.87 (4 H, s); 9.87 (1 H, s).

*Variante 1:*

**4-Butyl-4-dimethylamino-cyclohexanon (E-2)**

8-Butyl-1,4-dioxa-spiro[4.5]dec-8-yl)-dimethyl-amin Hydrochlorid **D-2** (3.10 g, 11.1 mmol) wurde in $H_2O$ (4.7 ml) und konz. HCl (7 ml) vorgelegt und 24 h bei Raumtemperatur gerührt. Der Ansatz wurde mit Ether (1 x 15 ml) extrahiert, die wässrige Phase unter Eiskühlung mit 5N NaOH alkalisch eingestellt und mit Dichlormethan (3 x 20 ml) extrahiert. Die org. Phase wurde über $Na_2SO_4$ getrocknet und i. Vak. eingeengt.
*Ausbeute:* 1.96 g (89 %), Öl
*$^1$H-NMR (DMSO-$d_6$):* 0.88 (3 H, t); 1.23 (4 H, m); 1.40 (2 H, m); 1.68 (2 H, m); 1.91 (2 H, m); 2.31 (2 H, m); 2.22 (6 H, s); 2.42 (2 H, m).
*$^{13}$C-NMR (DMSO-$d_6$):* 13.91; 23.21; 26.06; 29.53; 31.07; 37.04; 38.88; 55.36; 210.37.

*Variante 2:*

**(8-Butyl-1,4-dioxa-spiro[4.5]dec-8-yl)-dimethyl-amin Hydrochlorid (D-2)**

Zu einer Lösung von Aminonitril **B-1** (38,3 g, 0,182 mol) in abs. Tetrahydrofuran (420 ml) wurde unter Kühlung mit einer Eis-Kochsalzmischung langsam 2M n-Butylmagnesiumchlorid-Lösung in THF (228 ml, 0,456 mol) unter Argon hinzugefügt. Dabei sollte die Reaktionstemperatur nicht über 10 °C steigen. Anschließend wurde 16 h bei Raumtemperatur gerührt. Es entstand eine braune klare Lösung. Zur Aufarbeitung des Reaktionsgemisches wurde unter Eiskühlung (0 bis 10 °C) gesättigte Ammoniumchloridlösung (150 ml) zugetropft. Dabei entstand ein weißer Feststoff, der durch Zugabe von Wasser (ungefähr 250 ml) gelöst wurde. Die Reaktionsmischung wurde mit Diethylether (4 × 100 ml) extrahiert. Die organische Phase wurde mit Wasser (100 ml) und gesättigter NaCl-Lösung (100 ml) gewaschen, getrocknet und eingeengt. Es blieb ein gelbes Öl (44,5 g) zurück, das außer der gewünschten Butylverbindung noch das Edukt Nitril enthielt. Das Rohprodukt wurde in Ethylmethylketon (275 ml) gelöst, unter Eiskühlung mit $ClSiMe_3$ (32 ml, 0,245 mol) versetzt und im offenen Kolben bei Raumtemperatur gerührt. Das Hydrochlorid D-2 wurde durch mehrmalige Filtration im Abstand von 2 h abgetrennt. Nach einer Reaktionszeit von 6-8 h konnte das Hydrochlorid **D-2** in einer Ausbeute von 82 % (41,8 g) als weißer Feststoff isoliert werden.

*Variante 2:*

**4-Butyl-4-dimethylamino-cyclohexanon (E-2)**

Das Hydrochlorid **D-2** (41,8 g, 0,15 mmol) wurde in Wasser (78 ml) gelöst und unter Rühren und Eiskühlung mit 37-proz. Salzsäure (100 ml, 1,2 mol) versetzt. Die klare Reaktionsmischung wurde 7 Tage bei Raumtemperatur gerührt. Nach beendeter Hydrolyse wurde die Reaktionsmischung mit Diethylether extrahiert (2 × 70 ml). Die organischen Extrakte wurden verworfen. Die wäßrige Phase wurde unter Eiskühlung mit 5N Natronlauge alkalisch gemacht (ungefähr 250 ml) und kräftig gerührt. Die Lösung wurde mit Diethylether extrahiert (3 × 100 ml). Die vereinigten organischen Extrakte wurden mit Wasser (2 × 70 ml) gewaschen, getrocknet und eingeengt. Das Keton **E-2** wurde als hellbraunes Öl in einer Ausbeute von 96 % (28,4 g) gewonnen. Die Ausbeute an Keton **E-2** -bezogen auf das in der ersten Stufe eingesetzte Ketal - betrug 75 %.

**<u>Baustein E-3:</u>**

**1-Chlor-4-methoxy-butan**

Natriumhydrid (24.0 g, 1.0 mol) und Iodmethan (142 g, 1.0 mol) wurden in abs. THF (350 ml) vorgelegt. Unter Argon und Eiskühlung wurde innerhalb von 1.5 h eine Lösung von 4-Chlorbutan-1-ol (54 g, 0.5 mol) in abs. THF (50 ml) zugetropft, dabei trat leichte Gasbildung auf. Der Ansatz wurde 24 h bei Raumtemperatur gerührt.
Zu der Reaktionslösung wurde 20%-ige $NH_4Cl$-Lösung (130 ml) getropft. Die organische Phase wurde abgetrennt, über $Na_2SO_4$ getrocknet und das Trockenmittel abfil-triert.
Die organische Phase wurde unter Normaldruck destilliert.
Siedepunkt: 150-162 °C
Ausbeute: 10.4 g (17 %)
*$^1$H-NMR* (DMSO-$d_6$): 1.93 (2 H, m); 3.23 (3 H; s); 3.44 (2 H, t); 3.66 (2 H, t).

**[8-(4-Methoxy-butyl)-1,4-dioxa-spiro[4.5]dec-8-yl]-dimethyl-amin (C-3)**

Magnesium (1.62 g, 66.8 mmol) und $I_2$ in abs. Diethylether (25 ml) wurden unter Argonatmosphäre und zeitweiliger Erwärmung mit einer Lösung von 1-Chlor-4-methoxy-butan (8.19 g, 66.8 mmol) in abs. Ether (12 ml) versetzt. Die Mischung wurde 1 h unter Rückfluss gerührt, bis sich das Magnesium weitgehend aufgelöst hatte.
Unter Eiskühlung wurde eine Lösung von 8-Dimethylamino-1,4-dioxa-spiro[4.5]decan-8-carbonitril **B-1** (10.5 g, 50.1 mmol) in abs. THF (40 ml) zugetropft. Dabei fiel ein zäher Niederschlag aus, zur besseren Durchmischung wurde weiteres abs. THF (20 ml) addiert. Die Mischung wurde bei Raumtemperatur 24 h gerührt. Der Ansatz wurde unter Eiskühlung mit $NH_4Cl$-Lösung (20%-ig, 80 ml) und Wasser (100 ml) versetzt, die organische Phase abgetrennt und die wässrige Phase mit Ether (3 x 100 ml) extrahiert.
Die vereinigten organischen Phasen wurden mit gesättigter NaCl-Lösung (80 ml) und Wasser (80 ml) gewaschen, über $Na_2SO_4$ getrocknet und i. Vak. eingeengt.
Das Rohprodukt wurde durch Flash-Chromatographie mit Chloroform/Methanol (50:1 ⇒ 20:1 ⇒ 9:1) gereinigt.
*Ausbeute:* 6.44 g (59 %), gelbes Öl

*$^{13}C$-NMR (DMSO-$d_6$):* 19.81; 27.10; 29.26; 30.34; 35.79; 37.48; 57.76: 63.72; 64.07; 71.35; 106.46.

**4-Dimethylamino-4-(4-methoxy-butyl)-cyclohexanon (E-3)**

Das [8-(4-Methoxy-butyl)-1,4-dioxa-spiro[4.5]dec-8-yl]-dimethyl-amin **(C-3)** (6.44 g, 23.7 mmol) wurde in Wasser (9.3 ml) gelöst, mit konz. HCl (14.6 ml) versetzt und bei Raumtemperatur 4 d gerührt.
Die Reaktionsmischung wurde mit Ether (2 x 50 ml) gewaschen. Anschließend wurde die Lösung mit 5N NaOH alkalisch gestellt und mit Dichlormethan (3 x 50 ml) extrahiert. Die vereinigten organischen Phasen wurden mit Wasser (50 ml) gewaschen, über $Na_2SO_4$ getrocknet, filtriert und das Solvens i. Vak. entfernt.
Ausbeute: 4.91 g (91 %), gelbes Öl
$^{13}C$-NMR (DMSO-$d_6$): 20.56; 29.75; 29.83; 30.98; 36.92; 37.06; 55.40: 57.73; 71.76; 210.39.

**Baustein E-4:**

**1-Chlor-3-methoxy-propan**

3-Methoxypropan-1-ol (47.1 g, 50 ml, 0.523 mol) wurde in Pyridin (41.3 g, 42.6 ml, 0.523 mol) gelöst, auf 10 °C abgekühlt und bei 10-30 °C unter kräftigem Rühren tropfenweise mit Thionylchlorid (93.3 g, 56.9 ml, 0.784 mol) versetzt. Dabei fiel ein fester Niederschlag aus, die Mischung wurde dann 3 h bei 65 ° C nachgerührt.
Der Ansatz wurde auf ein Gemisch aus Eis (130 g) und konz. HCl (26 ml) gegossen. Die wässrige Lösung wurde mit Ether (2 x 20 ml) extrahiert und die vereinigten organischen Phasen mit $K_2CO_3$-Lösung gewaschen. Bei Zugabe des Trocknungsmittels $K_2CO_3$ war starke Gasbildung zu beobachten, daher wurde die Lösung über Nacht stehen gelassen. Das Trockenmittel wurde abfiltriert und die organische Phase mit $K_2CO_3$-Lösung bis zur alkalischen Reaktion gewaschen. Die organische Phase wurde abgetrennt, mit Wasser gewaschen und über $K_2CO_3$ getrocknet, filtriert und bei Normaldruck destilliert.
*Siedepunkt:* 113 °C
Ausbeute: 41.2 g (72 %), farblose Flüssigkeit
*$^1H$-NMR (DMSO-$d_6$):* 1.93 (2 H, m); 3.23 (3 H; s); 3.44 (2 H, t); 3.66 (2 H, t).

**[8-(3-Methoxy-propyl)-1,4-dioxa-spiro[4.5]dec-8-yl]-dimethyl-amin (C-4)**

Magnesium (10.0 g, 92 mmol) und $I_2$ in abs. Diethylether (30 ml) wurden unter Argonatmosphäre und zeitweiliger Erwärmung tropfenweise mit einer Lösung von 1-Chlor-3-methoxy-propan (10.0 g, 92 mmol) in abs. Ether (15 ml) versetzt. Anschließend wurde der Ansatz unter Rückfluss 60 min nachgerührt, das Magnesium war danach nicht vollständig aufgelöst.
Unter Eiskühlung wurde eine Lösung von 8-Dimethylamino-1,4-dioxa-spiro[4.5]decan-8-carbonitril **B-1** (9.68 g, 46 mmol) in abs. THF (30 ml) zugetropft. Dabei fiel ein zäher Niederschlag aus, zur besseren Durchmischung wurden 100 ml abs. THF nachgegeben. Die Mischung wurde bei Raumtemperatur 24 h gerührt. Der Ansatz wurde unter Eiskühlung mit 20%-iger $NH_4Cl$-Lösung (100 ml) und Wasser (120 ml) versetzt, die organische Phase wurde abgetrennt und die wässrige Phase mit Ether (3 x 120 ml) extrahiert.
Die vereinigten organischen Phasen wurden mit gesättigter NaCl-Lösung (120 ml) und Wasser (120 ml) gewaschen, über $Na_2SO_4$ getrocknet und i. Vak. eingeengt. Die Rohausbeute betrug 10.8 g braunes Öl.
9.8 g Rohprodukt wurden durch Flash-Chromatographie mit $CHCl_3$/MeOH (50:1 $\Rightarrow$ 20:1 $\Rightarrow$ 9:1) gereinigt.
*Ausbeute:* 8.11 g (75 %), gelbes Öl
*$^1H$-NMR (DMSO-$d_6$):* 1.44 (8 H, m); 1.62 (4 H; m); 2.25 (6 H, s); 3.21 (3 H, s); 3.31 (2 H, m); 3.82 (4 H, s).
*$^{13}C$-NMR (DMSO-$d_6$):* 23.99; 26.52; 28.87; 29.88; 36.97; 55.24: 57.67; 63.40; 72.62; 108.07.

**4-Dimethylamino-4-(3-methoxy-propyl)-cyclohexanon (E-4)**

Das Amin **C-4** (8.11 g, 31.5 mmol) wurde in Wasser (12 ml) gelöst, unter Eiskühlung mit konz. HCl (19.5 ml) versetzt und 3 d bei Raumtemperatur gerührt. Die Reaktionsmischung wurde mit Ether (2 x 75 ml) gewaschen. Anschließend wurde die Lösung mit 5N NaOH alkalisch gestellt und mit Dichlormethan (3 x 75 ml) extrahiert. Die vereinigten organischen Phasen wurden mit Wasser (75 ml) gewaschen, über $Na_2SO_4$ getrocknet, filtriert und das Solvens i. Vak. entfernt.
*Ausbeute:* 6.03 g (90 %), gelbes Öl
*$^1H$-NMR (DMSO-$d_6$):* 1.44 (4 H, m); 1.68 (2 H; m); 1.88 (2 H, m); 2.00 (1 H, m); 2.05 (1 H, m); 2.20 (6 H, s); 2.41 (2 H, m); 3.22 (3 H, s); 3.28 (2 H, m).
*$^{13}C$-NMR (DMSO-$d_6$):* 24.01; 26.34; 30.88; 36.15; 37.06; 55.26: 57.70; 72.55; 210.39.

**Baustein E-5:**

**(8-Cyclohexyl-1,4-dioxa-spiro[4.5]dec-8-yl)-dimethyl-amin (C-5)**

Zu einer Lösung des Aminonitrils **B-1** (10.5 g, 50 mmol) in abs. THF (150 ml) wurde unter Argon und Eiskühlung innerhalb von 15 min 2M Cyclohexyl-magnesiumchlorid-Lösung in Ether (62.5 ml, 125 mmol) bei 5-10 °C getropft und anschließend bei Raumtemperatur über Nacht gerührt. Zur Aufarbeitung des Reaktionsgemisches wurde unter Eiskühlung 20 %ige Ammoniumchlorid-Lösung (50 ml) und Wasser (50 ml) zugegeben und mit Ether (3 x 100 ml) extrahiert. Die organische Phase wurde mit Wasser und gesättigter Natriumchlorid-Lösung gewaschen, über Natriumsulfat getrocknet und i. Vak. eingeengt. Der verbliebene Rückstand wurde durch Flash-Chromatographie mit $CHCl_3$/MeOH (20: 1) gereinigt.
*Ausbeute:* 1.18 g (9 %), farbloses Öl
*$^1$H-NMR (DMSO-$d_6$):* 1.05 (6 H, m); 1.43 (5 H; m); 1.61 (8 H, m), 2.35 (6 H, s); 3.86 (4 H, s).

**4-Cyclohexyl-4-dimethylamino-cyclohexanon (E-5)**

(8-Cyclohexyl-1,4-dioxa-spiro[4.5]dec-8-yl)-dimethyl-amin (C-5) (1.18 g, 4.41 mmol) wurde mit 6N Salzsäure (7 ml) versetzt und bei Raumtemperatur über Nacht gerührt. Nach beendeter Hydrolyse wurde die Reaktionsmischung mit Ether (2 x 10 ml) extrahiert, die wässrige Lösung unter Eiskühlung mit 5N Natronlauge alkalisch gestellt, die Reaktionsmischung mit Dichlormethan (3 x 20 ml) extrahiert, die organische Phase über Natriumsulfat getrocknet und i. Vak. eingeengt. Das Rohprodukt (637 mg) wurde durch Flash-Chromatographie mit $CHCl_3$/MeOH (9:1) gereinigt.
*Ausbeute:* 366 mg (37 %), farbloses Öl
*$^1$H-NMR (DMSO-$d_6$):* 1.08 (5 H, m); 1.68 (8 H; m); 1.99 (4 H, m); 2.29 (2 H, s), 2.41 (6 H, s).

**Baustein E-6:**

**(8-Cyclopentyl-1,4-dioxa-spiro[4.5]dec-8-yl)-dimethyl-amin (C-6)**

Zu einer Lösung des Aminonitrils **B-1** (10.5 g, 50 mmol) in abs. THF (150 ml) wurde unter Argon und Eiskühlung innerhalb von 15 min 2M Cyclopentylmagnesiumbromid-Lösung in Ether (62.5 ml, 125 mmol) bei 5-10 °C getropft und anschließend bei Raumtemperatur 72 h gerührt. Zur Aufarbeitung des Reaktionsgemisches wurde unter Eiskühlung 20 %-ige Ammoniumchlorid-Lösung (50 ml) und Wasser (50 ml) zugegeben und mit Ether (3 x 100 ml) extrahiert. Die organische Phase wurde mit Wasser und gesättigter Natriumchlorid-Lösung gewaschen, über Natriumsulfat getrocknet und i. Vak. eingeengt. Der verbliebene Rückstand wurde durch Flash-Chromatographie mit $CHCl_3$/MeOH (40: $\Rightarrow$ 20:1) getrennt. Da das gewünschte Produkt noch nicht sauber war, wurde eine weitere Säulen-Chromatographie mit $CHCl_3$/ MeOH (40:1) durchgeführt.
*Ausbeute:* 692 mg (5 %), farbloses Öl
*$^1$H-NMR (DMSO-$d_6$):* 1.23 (2 H, m); 1.46 (9 H; m); 1.69 (4 H, m); 2.04 (1 H, m); 2.23 (6 H. s); 3.86 (4 H, s).

**4-Cyclopentyl-4-dimethylamino-cyclohexanon (E-6)**

Das Ketal **C-6** (0.68 g, 2.68 mmol) wurde mit 6N Salzsäure (5 ml) versetzt und bei Raumtemperatur über Nacht gerührt. Nach beendeter Hydrolyse wurde die Reaktionsmischung mit Ether (2 x 20 ml) extrahiert, die wässrige Lösung unter Eiskühlung mit 5N Natronlauge alkalisch gestellt, mit Dichlormethan (3 x 10 ml) extrahiert, die organische Phase über Natriumsulfat getrocknet und i. Vak. eingeengt.
*Ausbeute:* 424 mg (76 %), farbloses Öl
*$^1$H-NMR (DMSO-$d_6$):* 1.28 (2 H, m); 1.54 (8 H; m); 1.99 (4 H, m); 2.14 (1 H, m); 2.29 (6 H, s).
*$^{13}$C-NMR (DMSO-$d_6$):* 24.58; 28.13; 29.24; 36.07; 37.79 ; 42.97; 57.07; 210.67.

**Baustein E-7:**

**(8-Butyl-1,4-dioxaspiro[4.5]dec-8-yl)ethylmethylamin (C-7)**

Zu einer 2 *M* Lösung von Butylmagnesiumchlorid in Tetrahydrofuran (20 ml, 40 mmol) wurde bei 0 °C unter Argon eine Lösung von **B-2** (3.50 g, 15.6 mmol) in Tetrahydrofuran (50 ml) getropft und die Mischung über Nacht bei Raumtemperatur gerührt. Anschließend wurde die Reaktionsmischung unter Eiskühlung vorsichtig mit gesättigter Ammoniumchloridlösung (60 ml) versetzt, der pH-Wert mit Natronlauge auf 10 korrigiert und mit Diethylether (3 $\times$ 50 ml) extrahiert. Die vereinigten organischen Phasen wurden mit Natriumsulfat getrocknet und i. Vak. eingeengt.

Das Rohprodukt wurde ohne Reinigung weiter umgesetzt.

**4-Butyl-4-(ethylmethylamino)cyclohexanon (E-7)**

Eine Lösung von C-7 (4.43 g, 17.3 mmol) in Aceton (15 ml) wurde zuerst mit Wasser (2.5 ml) und dann mit konzentrierter Salzsäure (2.5 ml) versetzt und über das Wochenende bei Raumtemperatur gerührt. Anschließend wurde das Reaktionsgemisch mit 2 M Kaliumcarbonatlösung alkalisch gestellt (pH 10), mit Diethylether (3 × 40 ml) extrahiert, die vereinigten organischen Phasen mit Natriumsulfat getrocknet und i. Vak. eingeengt. Das Rohprodukt wurde mittels Flashchromatographie (200 g, 20 × 5.7 cm) mit Cyclohexan / Ethylacetat (2:1) gereinigt.
Ausbeute: 2.08 g (57 %), gelbes Öl
$^1$H-NMR (DMSO-$d_6$): 0.87 (t, 3H, J = 7.0 Hz); 1.00 (t, 3H, J = 7.0 Hz); 1.20-1.29 (m, 4H); 1.38-1.42 (m, 2H); 1.63-1.71 (m, 2H); 1.92-2.00 (m, 4H); 2.20 (s, 3H); 2.36-2.47 (m, 4H).

**<u>Baustein E-8</u>**

**Benzylmethyl-[8-(4*H*-[1,2,3]triazin-1-yl)-1,4-dioxaspiro[4.5]dec-8-yl]amin**

Eine Lösung von 1,4-Dioxaspiro[4,5]decan-8-on (3.9 g, 25 mmol), N-Benzylmethylamin (3.32 g, 3.54 ml, 27.5 mmol) und 1,2,3 Triazol (2.07 g, 30 mmol) in Toluol (40 ml) wurde 8 h am Wasserabscheider (Dean-Stark) unter Rückfluss erhitzt. Die Reaktionslösung wurde nach Abkühlen auf Raumtemperatur direkt in weiter eingesetzt.

**Benzyl-(8-butyl-1,4-dioxaspiro[4.5]dec-8-yl)methylamin (D-8)**

Zu einer 2 M Lösung von n-Butylmagnesiumchlorid in Tetrahydrofuran (50 ml, 100 mmol) wurde bei 0° C unter einem Argonstrom die Reaktionslösung von Benzylmethyl-[8-(4*H*-[1,2,3]triazin-1-yl)-1,4-dioxaspiro[4.5]dec-8-yl]amin (20 ml, ca. 25 mmol) getropft. Das Gemisch wurde auf Raumtemperatur erwärmt und 2 h gerührt und anschließend in gesättigte Ammoniumchloridlösung (60 ml) gegossen. Die Phasen wurden getrennt, die wässrige mit Diethylether (3 × 30 ml) extrahiert, die vereinigten organischen Phasen mit Natriumsulfat getrocknet und i. Vak. eingeengt. Das Rohprodukt wurde in Dichlormethan aufgenommen, die unlöslichen Bestandteile wurden abfiltriert, das Filtrat erneut i. Vak. eingeengt und der Rückstand (6.31 g) durch Flashchromatographie (400 g, 20 × 7.6 cm) mit Cyclohexan / Ethylacetat (9:1) gereinigt.
Ausbeute: 3.4 g (43 % über zwei Stufen), farbloses Öl
$^1$H-NMR (DMSO-$d_6$): 0.90 (t, 3H, J = 6.8 Hz); 1.18-1.33 (m, 4H); 1.36-1.47 (m, 4H); 1.51-1.59 (m, 2H); 1.70-1.93 (m, 4H); 2.03 (3 H, s); 3.57 (s, 2H); 3.85 (s, 4H); 7.15-7.25 (m, 1 H); 7.27-7.36 (m, 4 H).

**4-(Benzylmethylamino)-4-butylcyclohexanon (E-8)**

Eine Lösung von D-8 (3.40 g, 10.7 mmol) in Aceton (70 ml) wurde mit Wasser (10 ml) und 37 % Salzsäure (14.1 ml) versetzt und 5.5 h bei Raumtemperatur gerührt. Zu dem Gemisch wurde anschließend langsam gesättigte Kaliumcarbonatlösung getropft, bis pH 10 erreicht war. Das Gemisch wurde mit Diethylether (4 × 40 ml) extrahiert, die vereinigten organischen Phasen wurden mit Natriumsulfat getrocknet und i. Vak. eingeengt.
Ausbeute: 2.3 g (74 %), gelbliches Öl
$^1$H-NMR (DMSO-$d_6$): 0.91 (t, 3H, J = 6.74 Hz); 1.20-1.37 (m, 5H); 1.48-1.59 (m, 2H); 1.78 (dt, 2H, J = 13.7 und 5.5 Hz); 2.00-2.17 (m, 4H); 2.09 (s, 3H); 2.50-2.60 (m, 1H); 3.66 (s, 2H); 7.12-7.26 (m, 1 H); 7.26-7.38 (m, 4H).

**<u>Baustein E-9:</u>**

**1,4-Dioxaspiro[4.5]dec-8-yliden)-(4-methoxybenzyl)amin**

Zu einer Lösung von 1,4-Dioxaspiro[4.5]decan-8-on (10.0 g, 64 mmol) in Dichlormethan (100 ml) wurden Molsieb 4 Å (20 g) und 4-Methoxybenzylamin (11.8 g, 83 mmol) gegeben. Die Suspension wurde 16 h bei Raumtemperatur gerührt, dann filtriert und das Filtrat ohne weitere Aufarbeitung in die nächste Stufe eingesetzt eingesetzt.
$^1$H-NMR (300 MHz, *$CDCl_3$):* 1.81 (t, *J* = 6.3 Hz, 2H); 1.89 (t, *J* = 6.3 Hz, 2H); 2.50 (t, *J* = 6.3 Hz, 4H); 3.74 (s, 3H); 3.95 (s, 4H); 4.45 (s, 2H); 6.83 (d, *J* = 8.6 Hz, 2H); 7.18 (d, *J* = 8.6 Hz, 2H).
$^{13}$C-NMR (100 MHz, *$CDCl_3$*): 25.0; 34.0; 34.8; 36.2; 53.8; 55.1; 64.3; 108.3; 113.7; 128.0; 128.6; 158.2; 171.2.

**(8-Allyl-1,4-dioxaspiro[4.5]dec-8-yl)-(4-methoxybenzyl)amin (C-9)**

Zur Lösung von 1,4-Dioxaspiro[4.5]dec-8-yliden)-(4-methoxybenzyl)amin (17.6 g, 64 mmol) in Dichlormethan

(120 ml) wurde eine 1 *M* Lösung von Allylmagnesiumbromid (100 ml, 100 mmol) in Diethylether getropft und das Reaktionsgemisch 4 h bei Raumtemperatur gerührt. Anschließend wurde die Mischung unter Eiskühlung auf gesättigte Ammoniumchlorid-Lösung (100 ml) gegossen und mit Dichlormethan (3 × 40 ml) extrahiert. Die vereinigten organischen Phasen wurden mit Natriumsulfat getrocknet und i. Vak. eingeengt. Der Rückstand wurde durch Flashchromatographie (400 g, 20 × 7.6 cm) mit Chloroform / Methanol (10:0.1) gereinigt.
Ausbeute: 10.8 g (53 %), braunes Öl
$^1$H-NMR (300 MHz, *$d_6$-DMSO*)*:* 1.30 (br s, 1H); 1.42 (t, *J* = 11.5 Hz, 4H); 1.51-1.64 (m, 2H); 1.72-1.86 (m, 2H); 2.18 (d, *J* = 7.3 Hz, 2H); 3.51 (s, 2H); 3.72 (s, 3H), 3.83 (s, 4H); 4.99-5.16 (m, 2H); 5.76-5.93 (m, 1 H); 6.82-6.89 (m, 2H); 7.24 (m, 2H). $^{13}$C-NMR (100 MHz, *$d_6$-DMSO*)*:* 29.9; 32.1; 41.8; 44.3; 52.6; 54.9; 63.4; 108.3; 113.4; 117.2; 128.9; 133.6; 134.8; 157.9.

**4-Allyl-4-(4-methoxybenzylamino)cyclohexanon (E-9)**

Zu einer Lösung von (8-Allyl-1,4-dioxaspiro[4.5]dec-8-yl)-(4-methoxybenzyl)amin **(C-9)** (1.0 g, 3.15 mmol) in Aceton (10 ml) und Wasser (0.5 L) wurde konzentrierte Salzsäure (0.5 ml) gegeben und das Gemisch 16 h bei Raumtemperatur gerührt. Anschließend wurde die Lösung mit gesättigter Natriumhydrogencarbonat-Lösung (40 ml) versetzt und mit Dichlormethan (3 × 40 ml) extrahiert. Die vereinigten organischen Phasen wurden mit Natriumsulfat getrocknet und i. Vak. eingeengt.
Ausbeute: 864 mg (100 %), braunes Öl
$^1$H-NMR (400 MHz, $d_6$-*DMSO*): 1.64 (dt, *J* = 13.2, 4.6 Hz, 2H); 1.89 (d, *J* = 13.0 Hz, 2H); 2.04 (d, *J* = 14.9 Hz, 2H); 2.30 (d, *J* = 7.2 Hz, 2H); 2.45-2.63 (m, 2H); 3.61 (s, 2H); 3.72 (s, 3H); 5.12 (dd, *J* = 13.1, 11.2 Hz, 2H); 5.90 (dt, *J* = 17.1, 7.3 Hz, 1 H); 6.86 (d, *J* = 8.3 Hz, 2H); 7.28 (d, *J* = 8.2 Hz, 2H). Das NH-Signal konnte nicht identifiziert werden.
$^{13}$C-NMR (100 MHz, *$d_6$-DMSO*)*:* -3.1; -0.9; 4.4; 7.4; 15.7; 17.9; 76.4; 80.5; 92.1; 96.4; 97.5; 120.9; 174.1.

<u>**Baustein E-10:**</u>

**Phenyl-(1,4-dioxaspiro[4.5]dec-8-yliden)amin**

In Analogie zur Synthese des Ketons **E-13** wurde das entsprechende N-Phenylsubstituierte Keton **E-10** synthetisiert. In Analogie zur Synthese von Benzyl-(1,4-dioxaspiro[4.5]dec-8-yliden)amin (vgl. Baustein **E-13)** wurde 1,4-Dioxaspiro[4.5]decan-8-on quantitativ unter Wasserabspaltung mit Anilin zum Imin Phenyl-(1,4-dioxaspiro[4.5]dec-8-yliden)amin umgesetzt.

**(8-Allyl-1,4-dioxaspiro[4.5]dec-8-yl)-penyl-amin (C-10)**

Bei der anschließenden Reaktion von Phenyl-(1,4-dioxaspiro[4.5]dec-8-yliden)amin mit Allylmagnesiumbromid (in Analogie zu **C-13)** konnte das gewünschte (8-Allyl-1,4-dioxaspiro[4.5]dec-8-yl)-penyl-amin **(C-10)** in guter Ausbeute isoliert werden.

**4-Allyl-4-phenylaminocyclohexanon (E-10)**

Zu einer Lösung von **C-10** (333 mg, 1.22 mmol) in Aceton (10 ml) und Wasser (0.5 ml) wurde konzentrierte Salzsäure (0.5 ml) gegeben und das Gemisch 2 d bei Raumtemperatur gerührt. Das Reaktionsgemisch wurde anschließend mit gesättigter Natriumhydrogencarbonat-Lösung (40 ml) versetzt und mit Dichlormethan (3 × 40 ml) extrahiert. Die vereinigten organischen Phasen wurden mit Natriumsulfat getrocknet und i. Vak. eingeengt.
Ausbeute: 285 mg (100 %), farblose Kristalle
Schmelzpunkt: 76-78 °C
$^1$H-NMR (400 MHz, *$d_6$-DMSO*)*:* 1.78 (dt, *J* = 13.0, 4.6 Hz, 2H); 2.06-2.29 (m, 4H); 2.49 (m, 4H); 5.00 (dd, *J* = 10.1, 1.9 Hz, 2H); 5.30 (s, 1 H); 5.65-5.87 (m, 1H); 6.58 (t, *J* = 7.2 Hz, 1 H); 6.82 (dd, *J* = 8.5 Hz, 2H); 7.07 (m, 2H).
$^{13}$C-NMR (100 MHz, *$d_6$-DMSO*)*:* 34.5; 36.3; 41.0; 53.8; 115.3; 116.4; 117.5; 128.7; 134.3; 147.2; 210.4.

<u>**Baustein E-11:**</u>

*Variante 1:*

**(1,4-Dioxaspiro[4.5]dec-8-yliden)phenylimin**

Eine Lösung von 1,4-Dioxaspiro[4.5]deca-8-on (5.46 g, 35 mmol) und Anilin (3.35 g, 3.28 ml, 36 mmol) in Toluol

(100 ml) wurde 15 h am Wasserabscheider, der zusätzlich mit wasserfreiem Natriumsulfat (2 g) beschickt war, erhitzt. Zur Umsatzkontrolle wurde eine Probe entnommen, i. Vak. eingeengt und sofort ein $^1$H-NMR-Spektrum in DMSO gemessen.
Nach vollständiger Umsetzung wurde die Reaktionslösung i. Vak. eingeengt und der Rückstand in wasserfreiem Tetrahydrofuran gelöst.
$^1$H-NMR (DMSO-$d_6$): 1.70 (t, 2H, J = 6.7 Hz); 1.86-1.94 (m, 2H); 2.21 (t, 2H, J = 6.8 Hz); 2.35 (t, 2H, J = 7.0 Hz); 3.91-3.94 (m, 4H); 6.67-6.71 (m, 2H); 6.96-7.04 (m, 1 H); 7.24-7.31 (m, 2H).

*Variante 2:*

**(1,4-Dioxaspiro[4.5]dec-8-yliden)phenylimin**

Eine Lösung von 1,4-Dioxaspiro[4.5]decan-8-on (6.20 g, 39.6 mmol) in Dichlormethan (65 ml) wurde mit Molsieb 4 Å (12.5 g) und Anilin (3.80 g, 3.73 ml, 40.8 mmol) versetzt und über das Wochenende bei Raumtemperatur gerührt. Zur Umsatzkontrolle wurde eine Probe entnommen, i. Vak. eingeengt und sofort ein $^1$H-NMR-Spektrum in $CDCl_3$ aufgenommen. Nach vollständiger Umsetzung wurde das Reaktionsgemisch filtriert und das Filtrat i. Vak. eingeengt.
$^1$H-NMR ($CDCl_3$): 1.76 (t, 2H, J = 6.6 Hz); 1.93-2.05 (m, 2H); 2.35 (t, 2H, J = 6.7 Hz); 2.64 (t, 2H, J = 6.6 Hz); 3.96-4.02 (m, 4H); 6.71 (d, 2H, J = 7.8 Hz); 7.05 (t, 1H, J = 7.2 Hz); 7.29 (t, 2H, J = 7.9 Hz).

*Variante 1:*

**(8-Butyl-1,4-dioxaspiro[4.5]dec-8-yl)phenylamin (C-11) und 4-Butyl-4-phenyl-aminocyclohexanon (E-11)**

Zu einer 1.6 M Lösung von n-Butyllithium in n-Hexan (27 ml, 42 mmol) wurde bei 0 °C unter Argon eine Lösung von (1,4-Dioxaspiro[4.5]dec-8-yliden)phenylimin (17 mmol) in wasserfreiem Tetrahydrofuran getropft. Anschließend wurde das Reaktionsgemisch langsam auf Raumtemperatur erwärmt und über Nacht gerührt. Danach wurde die Reaktionsmischung unter Eiskühlung mit Wasser (40 ml) versetzt und mit Diethylether (3 × 50 ml) extrahiert. Die vereinigten organischen Phasen wurden i. Vak. eingeengt und der Rückstand mittels Flashchromatographie (100 g, 20 × 4.0 cm) mit Cyclohexan / Ethylacetat (9:1) und 1 % Triethylamin gereinigt.

**C-11:**
Ausbeute: 645 mg (13 %), braunes Öl
$^1$H-NMR (DMSO-$d_6$): 0.78 (t, 3H, J = 6.8 Hz); 1.17-1.22 (m, 4H); 1.42-1.71 (m, 8H); 1.94-2.03 (m, 2H); 3.83 (s, 4H); 4.93 (s, 1 H); 6.49 (t, 1 H, J = 7.3 Hz); 6.71 (d, 2H, J = 8.0 Hz); 7.01 (t, 2H, J = 7.8 Hz).
**E-11:**
Ausbeute: 1.01 g (24 %), braunes Öl
$^1$H-NMR (DMSO-$d_6$): 0.78 (t, 3H, J = 7.0 Hz); 1.12-1.30 (m, 4H); 1.57-1.87 (m, 4H); 2.04-2.15 (m, 2H); 2.19-2.31 (m, 2H); 2.40-2.60 (m, 2H, überlagert vom DMSO-Signal); 5.25 (s, 1 H); 6.55 (t, 1H, J = 7.2 Hz); 6.77 (d, 2H, J = 8.6 Hz); 7.00-7.09 (m, 2H).

Darüber hinaus wurde noch ein Gemisch von **C-11** und **E-11** (816 mg, ca. 20 %) erhalten.

*Variante 2:*

**(8-Butyl-1,4-dioxaspiro[4.5]dec-8-yl)phenylamin (C-11)**

Zu einer 1.6 *M* Lösung von n-Butyllithium in *n*-Hexan (63 ml, 98 mmol) wurde bei 0 °C unter Argon eine Lösung von (1,4-Dioxaspiro[4.5]dec-8-yliden)phenylimin (39.6 mmol) in wasserfreiem Tetrahydrofuran getropft. Anschließend wurde das Reaktionsgemisch langsam auf Raumtemperatur erwärmt und über Nacht gerührt. Danach wurde die Reaktionsmischung unter Eiskühlung mit Wasser (40 ml) versetzt und mit Diethylether (3 × 50 ml) extrahiert. Die vereinigten organischen Phasen wurden mit Natriumsulfat getrocknet, i. Vak. eingeengt und der Rückstand mittels Flashchromatographie (100 g, 20 × 4.0 cm) mit Cyclohexan / Ethylacetat (9:1) und 1 % Triethylamin gereinigt.
Ausbeute: 2.83 g (25 %), braunes Öl

**4-Butyl-4-phenylaminocyclohexanon (E-11)**

Eine Lösung von **C-11** (645 mg, 2.23 mmol) in Aceton (15 ml) wurde mit Wasser (2.5 ml) und konzentrierter Salzsäure (2.5 ml) versetzt und über das Wochenende bei Raumtemperatur gerührt. Anschließend wurde das Reaktionsgemisch mit Kaliumcarbonatlösung alkalisch (pH 10) gestellt, mit Diethylether (3 × 30 ml) extrahiert, die vereinigten organischen Phasen wurden mit Natriumsulfat getrocknet und i. Vak. eingeengt.
Ausbeute: 547 mg (100 %); braunes Öl
$^1$H-NMR (DMSO-$d_6$): 0.78 (t, 3H, J = 7.0 Hz); 1.16-1.26 (m, 4H); 1.65-1.78 (m, 4H); 2.04-2.13 (m, 2H); 2.21-2.29 (m, 2H); 2.42-2.58 (2H, überlagert vom DMSO-Signal); 5.25 (s, 1 H); 6.55 (t, 1H, J = 7.2 Hz); 6.77 (d, 2H, J = 7.7 Hz); 7.03 (d, 2H, J = 7.3 Hz); 7.07 (d, 2H, J = 7.3 Hz).

**Baustein E-12:**

**4-(8-Butyl-1,4-dioxaspiro[4.5]dec-8-yl)morpholin (C-12)**

In einem ausgeheizten Kolben wurde eine Lösung von Morpholin (4.79 g, 4.8 ml, 55 mmol), 1,4-Dioxaspiro [4.5]dec-8-on (7.8 g, 50 mmol) und 1,2,3-Triazol (4.14 g, 60 mmol) in Toluol (50 ml) 7 h unter Rückfluss am Wasserabscheider erhitzt. Anschließend wurde unter Argon zur auf 0 °C abgekühlten Lösung eine 2 M Lösung von n-Butylmagnesiumchlorid in Tetrahydrofuran (100 ml, 200 mmol) so getropft, dass die Innentemperatur unter 30 °C blieb. Das Reaktionsgemisch wurde 2 h bei Raumtemperatur gerührt und danach unter Eis-Wasser-Kühlung zu 20 % Ammoniumchlorid-Lösung (120 ml) getropft. Die organische Phase wurde abgetrennt und die wässrige Phase mit Ethylacetat (3 × 100 ml) extrahiert. Die vereinigten organischen Phasen wurden mit 2 N Natronlauge (100 ml) und Wasser (100 ml) gewaschen, mit Natriumsulfat getrocknet und i. Vak. eingeengt. Das Rohprodukt (7.67 g) wurde durch Flashchromatographie (400 g, 20 × 7.5 cm) mit Ethylacetat / Cyclohexan (1:2) gereinigt.
Ausbeute: 3.86 g (27 %), farbloses Öl
$^1$H-NMR ($CDCl_3$): 0.88 (t, *J* = 6.9 Hz, 3H); 1.14-1.73 (m, 12H); 1.88 (dt, *J* = 12.6, 3.4 Hz, 2H); 2.44-2.61 (m, 4H); 3.56-3.73 (m, 4H); 3.93 (m, 4H).

**4-Butyl-4-morpholin-4-ylcyclohexanon (E-12)**

Zu einer Lösung von **C-12** (3.40 g, 12 mmol) in Aceton (20 ml) wurde 6 *M* Salzsäure (5 ml) gegeben. Nach 24 h wurde die Reaktionslösung mit weiterer 6 *M* Salzsäure (2.5 ml) versetzt, weitere 20 h bei Raumtemperatur gerührt, anschließend mit 25 % Kaliumcarbonat-Lösung alkalisch (pH~10) gestellt und mit Diethylether (3 × 25 ml) extrahiert. Die vereinigten organischen Phasen wurden mit Natriumsulfat getrocknet und i. Vak. eingeengt. Das Rohprodukt (2.7 g) wurde durch Flashchromatographie (200 g, 20 × 5.6 cm) mit Ethylacetat / Cyclohexan (1:4) gereinigt.
Ausbeute: 2.18 g (76 %), farbloses Öl
$^1$H-NMR ($CDCl_3$): 0.90 (t, 3 H, *J* = 7.0 Hz); 1.09- 2.23 (m, 12 H); 2.55 (dd, 2 H, *J* = 14.3, 5.8 Hz); 2.59-2.65 (m, 4 H); 3.67-3.73 (m, 4 H).

**Baustein E-13:**

**Benzyl-(1,4-dioxaspiro[4.5]dec-8-yliden)amin**

Zu einer Lösung von 1,4-Dioxaspiro[4.5]decan-8-on (10.0 g, 64 mmol) in Dichlormethan (100 ml) wurden Molsieb 4 Å (20 g) und Benzylamin (8.90 g, 83 mmol) gegeben und das Reaktionsgemisch 16 h bei Raumtemperatur gerührt. Die Suspension wurde anschließend filtriert und das Filtrat i. Vak. eingeengt.
Ausbeute: 15.6 g (99 %), gelbliches Öl
$^1$H-NMR (300 MHz, *$CDCl_3$*): 1.83 (t, *J* = 6.3 Hz, 2H); 1.92 (t, *J* = 6.6 Hz, 2H); 2.53 (s, 4H); 3.98 (s, 4H); 4.54 (s, 2H); 7.25 (m, 5H).
$^{13}$C-NMR (100 MHz, *$CDCl_3$*): 25.6; 34.2; 34.9; 36.3; 54.6; 64.4; 108.0; 126.6; 127.9; 128.4; 140.2; 171.7.

**(8-Allyl-1,4-dioxaspiro[4.5]dec-8-yl)-benzyl-amin (C-13)**

Zu einer Lösung von Benzyl-(1,4-dioxaspiro[4.5]dec-8-yliden)amin (15.6 g, 63.7 mmol) in Dichlormethan (120 ml) wurde eine 1 *M* Lösung von Allylmagnesiumbromid (127 ml, 127 mmol) getropft und das Reaktionsgemisch 72 h bei Raumtemperatur gerührt. Die Mischung wurde anschließend unter Eiskühlung vorsichtig auf gesättigte Ammoniumchlorid-Lösung (100 ml) gegossen und mit Dichlormethan (3 × 40 ml) extrahiert. Die vereinigten organischen Phasen wurden mit Natriumsulfat getrocknet, i. Vak. eingeengt und der Rückstand durch Flashchromatographie (400 g, 20 × 7.6 cm) mit Chloroform / Methanol (10 : 0.2) gereinigt.
Ausbeute: 5.92 g (32 %), braunes Öl
$^1$H-NMR (300 MHz, *$d_6$-DMSO*): 1.25-1.52 (m, 4H); 1.53-1.66 (m, 2H); 1.74-1.87 (m, 2H); 2.20 (d, *J* = 7.4 Hz, 2H); 3.59 (s, 2H); 3.83 (s, 4H); 4.89-5.19 (m, 2H); 5.86 (tdd, *J* = 14.9, 10.4, 7.3 Hz, 1H); 7.20 (t, *J* = 7.0 Hz, 1H); 7.20-7.35 (m, 4H).

$^{13}$C-NMR (100 MHz, *$d_6$-DMSO*): 29.9; 32.0; 41.9; 44.9; 52.7; 63.4; 63.4; 108.3; 117.2; 126.3; 127.8; 127.9; 134.8; 141.8.

**4-Allyl-4-benzylaminocyclohexanon (E-13)**

Zu einer Lösung von C-13 (500 mg, 1.74 mmol) in Aceton (20 ml) und Wasser (2 ml) wurde konzentrierte Salzsäure (2 ml) gegeben und das Gemisch 16 h bei Raumtemperatur gerührt. Anschließend wurde das Reaktionsgemisch mit Natriumhydrogencarbonat-Lösung versetzt (40 ml) und mit Ethylacetat (3 × 40 ml) extrahiert. Die vereinigten organischen Phasen wurden mit Natriumsulfat getrocknet und i. Vak. eingeengt.
Ausbeute: 423 mg (100 %), braunes Öl
$^{1}$H-NMR (300 MHz, *$d_6$-DMSO*): 1.64 (dt, *J* = 13.2, 4.9 Hz, 2H); 1.75-1.97 (m, 2H); 2.04 (dd, *J* = 14.8, 3.4 Hz, 2H); 2.31 (d, *J* = 7.3 Hz, 2H); 2.46-2.65 (m, 2H); 3.68 (s, 2H); 5.03-5.20 (m, 2H); 5.81-6.00 (m, 1 H); 7.14-7.26 (m, 1H); 7.26-7.35 (m, 2H); 7.39 (m, 2H). Das NH-Signal konnte nicht identifiziert werden.
$^{13}$C-NMR (100.4 MHz, *$d_6$-DMSO*): 33.8; 36.4; 41.4; 45.0; 52.8; 117.2; 117.5; 126.4; 127.9; 134.5; 141.5; 211.1.

**<u>Baustein E-14:</u>**

**1-(8-Pyrrolidin-1-yl-1,4-dioxaspiro[4.5]dec-8-yl)-1*H*-[1,2,3]triazol**

Zu einer Lösung von 1,4 Dioxaspiro[4,5]decan-8 on (3.9 g, 25 mmol) in Toluol (40 ml) wurden Pyrrolidin (1.95 g, 2.29 ml, 27.5 mmol), 1,2,3-Triazol (2.07 g, 30 mmol) und Molsieb 4 Å (7.14 g) gegeben. Das Gemisch wurde 7 h bei 90 °C gerührt. Anschließend wurde die Lösung dekantiert und sofort weiter umgesetzt.

**1-(8-Butyl-1,4-dioxaspiro[4.5]dec-8-yl)pyrrolidin (C-14)**

Zu einer 2 *M* Lösung von n-Butylmagnesiumchlorid (25 ml, 50 mmol) in Tetrahydrofuran wurde unter Eiskühlung und Argon die Reaktionslösung soeben hergestellten Triazol Derivates (ca. 6.9 g, 25 mmol) in Toluol (38 ml) getropft. Das Reaktionsgemisch wurde über Nacht bei Raumtemperatur gerührt und anschließend in gesättigte Ammoniumchloridlösung (60 ml) gegossen. Die Phasen wurden getrennt und die wässrige mit Diethylether (3 × 70 ml) extrahiert. Die vereinigten organischen Phasen wurden mit Natriumsulfat getrocknet, i. Vak. eingeengt und der Rückstand (12 g) durch Flashchromatographie (400 g, 20 x 7.6 cm) mit Ethylacetat / Methanol (9:1) gereinigt.
Ausbeute: 2.70 g (40 % über zwei Stufen), braunes Öl (**C-14**)
$^{1}$H-NMR (DMSO-$d_6$): 0.87 (t, 3H, J = 7.1 Hz); 1.12-1-29 (m, 4H); 1.30-1.45 (m, 4H); 1.46-1.60 (m, 4H); 1.61-1.75 (m, 6H); 1.93 (t, 1H, J = 7.1 Hz); 2.36 (t, 1H, J = 7.0 Hz), 2.58 (br s, 2H), 3.83 (s, 4H).

**4-Butyl-4-pyrrolidin-1-yl-cyclohexanon (E-14)**

Eine Lösung von **C-14** (2.70 g, 10.1 mmol) in Aceton (100 ml) wurde mit Wasser (10.0 ml) und 37 % Salzsäure (14.0 ml) versetzt und über Nacht bei Raumtemperatur gerührt. Zum Gemisch wurde anschließend langsam 4 *M* Natronlauge getropft, bis pH 10 erreicht war. Das Gemisch wurde mit Diethylether (4 × 40 ml) extrahiert, die vereinigten organischen Phasen wurden mit Natriumsulfat getrocknet und i. Vak. eingeengt. Das Rohprodukt (2.6 g) wurde durch Flashchromatographie (260 g, 30 × 5.6 cm) mit Ethylacetat / Methanol (9:1) gereinigt.
Ausbeute: 1.06 g (47 %), braunes Öl **(E-14)**
$^{1}$H-NMR (DMSO-$d_6$): 0.88 (t, 3H, J = 6.7 Hz); 1.14-1.34 (m, 4H); 1.40-1.50 (m, 2H); 1.62-1.88 (m, 8H); 2.04 (dt, 2H, J = 15.0, 3.9 Hz); 2.42 (ddd, 2H, J = 6.3, 11.8, 15.5 Hz); 2.63 (t, 4H, J = 6.0 Hz).

**<u>Baustein E-15</u>**:

**4-(8-[1,2,3]Triazol-1-yl-1,4-dioxaspiro[4.5]dec-8-yl)piperidin**

In einem ausgeheizten Kolben wurde eine Lösung von Piperidin (1.87 g, 2.17 ml, 22 mmol), 1,4-Dioxaspiro [4.5]dec-8-on (3.12 g, 20 mmol) und 1,2,3-Triazol (1.66 g, 24 mmol) in Toluol (20 ml) mit Molsieb 4 Å versetzt und 7 h bei 104 °C unter Rückfluss gerührt. Anschließend wurde diese Lösung vom Molsieb abdekantiert. Das Molsieb wurde mit Toluol gewaschen und abfiltriert. Die vereinigten flüssigen Phasen wurden als 0.6 M Lösung weiter umgesetzt.

**4-(8-Butyl-1,4-dioxaspiro[4.5]dec-8-yl)piperidin (C-15)**

In einem ausgeheizten Kolben wurde eine 2 M Lösung von n-Butylmagnesiumchlorid in Tetrahydrofuran (22 ml, 44 mmol) unter Argon bei 0 °C mit einer 0.6 M Lösung des soeben hergestellten Triazol Derivats in Toluol (18 ml,

11 mmol) innerhalb von 1 h tropfenweise versetzt. Das Gemisch wurde 2 h bei Raumtemperatur gerührt und danach unter Eis-Wasser-Kühlung zu 20 % Ammoniumchlorid-Lösung (24 ml) getropft. Die organische Phase wurde abgetrennt und die wässrige Phase mit Diethylether (4 × 20 ml) extrahiert. Die vereinigten organischen Phasen wurden mit 2 N Natronlauge (30 ml) und Wasser (20 ml) gewaschen, mit Natriumsulfat getrocknet und i. Vak. eingeengt. Das Rohprodukt (1.9 g) wurde durch Flashchromatographie (100 g, 22 × 4 cm) mit Ethylacetat / Cyclohexan (1:2) gereinigt.
Ausbeute: 1.03 g (33 %), farbloses Öl **(C-15)**
$^1$H-NMR (DMSO-$d_6$): 0.86 (t, 3H, J = 6.9 Hz); 1.09-1.52 (m, 16H); 1.60-1.79 (m, 4H); 2.44 (br s, 4H), 3.82 (s, 4H).

**4-Butyl-4-piperidin-4-ylcyclohexanon (E-15)**

Zu einer Lösung von **C-15** (1.0 g, 3.6 mmol) in Aceton (15 ml) wurde 6 M Salzsäure (5 ml) gegeben. Die Reaktionslösung wurde 6 d bei Raumtemperatur gerührt, anschließend mit 25 % Kaliumcarbonat-Lösung alkalisch (pH~9) gestellt und mit Diethylether (3 × 20 ml) extrahiert. Die vereinigten organischen Phasen wurden mit Natriumsulfat getrocknet und i. Vak. eingeengt.
Ausbeute: 860 mg (100 %), farbloses Öl **(E-15)**
$^1$H-NMR (DMSO-$d_6$): 0.87 (t, 3H, J = 6.9 Hz); 1.06-1.54 (m, 14H); 1.54-1.74 (m, 3H); 1.88-2.07 (m, 4H); 2.21-2.46 (m, 3H).

**Baustein E-16:**

**1-Methyl-4-(8-[1,2,3]triazol-1-yl-1,4-dioxaspiro[4.5]dec-8-yl)piperazin**

In einem ausgeheizten Kolben wurde eine Lösung von *N*-Methylpiperazin (2.60 g, 2.88 ml, 26 mmol), 1,4-Dioxaspiro[4.5]decan-8-on (3.90 g, 25 mmol) und 1,2,3-Triazol (1.87 g, 27 mmol) in Toluol (25 ml) 6 h am Wasserabscheider unter Rückfluss erhitzt.
Anschließend wurde die Reaktionslösung in einen verschließbaren Messzylinder überführt und das Rohprodukt weiter in eingesetzt.

**1-(8-Butyl-1,4-dioxaspiro[4.5]dec-8-yl)-4-methylpiperazin (C-16)**

Zu einer Lösung des soeben hergestellten Triazolderivats (12.5 mmol) in Toluol (12 ml) wurde unter Argon eine 2 *M* n-Butylmagnesiumchlorid-Lösung in Tetrahydrofuran (15 ml, 30 mmol) so getropft, dass die Innentemperatur unter 24 °C blieb. Nach beendeter Zugabe wurde das Reaktionsgemisch 2 h bei Raumtemperatur gerührt und dann auf 0 °C gekühlt und zu einer 20 % Ammoniumchloridlösung (50 ml) getropft, die wässrige Phase mit Diethylether (3 × 40 ml) extrahiert, die vereinigten organischen Phasen wurden mit 2 *N* Natronlauge (70 ml) und Wasser (70 ml) gewaschen, mit Natriumsulfat getrocknet und i. Vak. eingeengt.
Das Rohprodukt **C-16** (3.57 g) wurde weiter umgesetzt.

**4-Butyl-4-(4-methylpiperazin-1-yl)cyclohexanon (E-16)**

Eine Lösung von **C-16** (3.57 g, 12.0 mmol) in Aceton (15 ml) wurde zuerst mit Wasser (2.5 ml) und dann mit konzentrierter Salzsäure (2.5 ml) versetzt und über das Wochenende bei Raumtemperatur gerührt. Anschließend wurde das Reaktionsgemisch mit 2 *M* Kaliumcarbonatlösung alkalisch (pH 10) gestellt, mit Diethylether (3 × 40 ml) extrahiert, die vereinigten organischen Phasen wurden mit Natriumsulfat getrocknet und i. Vak. eingeengt. Das Rohprodukt wurde mittels Flashchromatographie (200 g, 20 × 5.7 cm) mit Methanol gereinigt.
Ausbeute: 2.04 g (67 %), gelbes Öl **(E-16)**
$^1$H-NMR(DMSO-$d_6$): 0.87 (t, 3H, J = 7.0 Hz); 1.16-1.28 (m, 4H); 1.37-1.43 (m, 2H); 1.66 (dt, 2H, J = 13.5, 4.5 Hz); 1.90-2.02 (m, 4H); 2.15 (s, 3H); 2.28-2.43 (m, 6H); 2.53-2.57 (m, 4H).
$^{13}$C-NMR: 13.9; 23.2; 26.3; 31.0 (2C); 31.9; 36.1 (2C); 44.0 (2C); 45.7; 55.5; 55.8; 210.4.

**Baustein E-17:**

**8-(Cyclopentylmethyl)-N,N-dimethyl-1,4-dioxaspiro[4.5]decan-8-amin**

Zu einer Mischung von Magnesium (3.64 g, 150 mmol) in abs. Ether (30 mL) wurde unter Argon eine Lösung von Iodmethylcyclopentan (31.5 g, 150 mmol) in abs. Ether (150 mL) so zugetropft, dass der Ether leicht siedete. Anschließend wurde die Reaktionslösung 30 min unter Rückfluss gekocht, auf RT abgekühlt und mit einer Lösung von 8-Dimethylamino-1,4-dioxa-spiro[4.5]decan-8-carbonitril **B-1** (10.5 g, 50 mmol) in abs. THF (100 mL) tropfenweise versetzt. Die Reaktionslösung begann zu Sieden und ein weißer Feststoff fiel aus. Es wurde 6 h unter Rückfluss gekocht

und anschließend bei RT über Nacht gerührt. Zur Aufarbeitung des Reaktionsgemisches wurde unter Eiskühlung 20 %-ige $NH_4Cl$-Lösung (200 mL) zugegeben und mit Ether (3 x 100 mL) extrahiert. Die organische Phase wurde über $Na_2SO_4$ getrocknet und i. Vak. eingeengt. Der verbliebene Rückstand wurde durch Flash-Chromatographie mit EE/EtOH (20:1) gereinigt.
Ausbeute: 13.4 g (100 %)
$^1$H-NMR (DMSO-$d_6$): 1.04 (2 H, m); 1.37 (4 H, m); 1.45-1.78 (17 H, m); 2.13 (6 H, s); 3.62 (4 H, s).

**4-Cyclopentylmethyl-4-dimethylamino-cyclohexanon (E-17)**

(8-Cyclopentylmethyl-1,4-dioxa-spiro[4.5]dec-8-yl)-dimethyl-amin (13.4 g, 50 mmol) wurde bei Raumtemperatur mit 5%-iger Schwefelsäure (600 mL) versetzt und 3 d bei RT gerührt. Der Reaktionsansatz wurde mit Ether (2 x 50 mL) extrahiert. Anschließend wurde die wässrige Phase unter Eisbadkühlung mit 5N NaOH alkalisch gestellt und mit Dichlormethan (3 x 50 mL) extrahiert. Die organische Phase wurde über $Na_2SO_4$ getrocknet und i. Vak. zur Trockene eingeengt.
Ausbeute: 8.46 g (76 %), farblose Kristalle.
$^1$H-NMR (DMSO-$d_6$): 1.04 (2 H, m); 1.48 (6 H, m); 1.83 (5 H, m); 1.93 (4 H, m); 2.20 (6 H, s); 2.44 (2 H, m).
$^{13}$C-NMR (DMSO-$d_6$): 24.7; 31.7; 34.6; 35.4; 36.1; 36.2; 36.9; 55.9; 210.4.

***Indolbausteine* - *F* & *H***

<u>**Baustein F-1**</u>:

Tryptophol (F-1) *(CAS.: 526*-55-6), *kommerziell erhältlich*

<u>**Baustein F-2:**</u>

(5-Fluor-3-hydroxy-2-oxo-2,3-dihydro-1H-indol-3-yl)essigsäure-ethylester[1]

5-Fluorisatin (10 mmol) wurde in einem Gemisch aus Ethanol/Pyridin/Essigsäure (50 ml, 15 : 5 : 2) gelöst, mit Ethyl-kaliummalonat (1,87 g, 11 mmol) versetzt und für 14h zum Rückfluß erhitzt. Der Verlauf der Reaktion wurde mittels DC (Eluent: Ethylacetat/Hexan 1:1) kontrolliert. Zur Aufarbeitung wurde das Lösungsmittelgemisch im Vakuum abdestilliert. Der Rückstand wurde in Ethylacetat (50 ml) aufgenommen und mit Wasser (50 ml) ausgeschüttelt. Nach Phasentrennung wurde die wäßrige Phase zweimal mit Ethylacetat (je 30 ml) extrahiert. Die kombinierten organischen Phasen wurden mit 2N HCl (50 ml) gewaschen, über $Na_2SO_4$ getrocknet und im Vakuum auf 20 ml eingeengt. Zu der Lösung wurde solange Hexan zugegeben, bis Kristallisation des gewünschten Produktes einsetzte. Zur Vervollständigung der Kristallisation wurde der Ansatz 12 h auf 10 °C gekühlt. Der Feststoff wurde abgesaugt und im Vakuum getrocknet.
Ausbeute: 89%
[1] S. J. Garden, R. B. da Silva, A. C. Pinto, Tetrahedron 2002, 58, 8399-8412 (speziell Seite 8406).

2-(5-Fluoro-1 H-indol-3-yl)ethanol (F-2)[2]

Das soeben erhaltene Aldolprodukt (10 mmol) wurde unter Ar-Atmosphäre in absolutem THF (20 ml) gelöst. Der Ansatz wurde anschließend unter Wasserbadkühlung mit $BH_3 \times THF$ (40 ml, 1 M Lösung, 40 mmol) versetzt und bei Raumtemperatur für 14h gerührt. Der Reaktionsverlauf wurde mittels DC überwacht. Nach Beendigung der Reaktion wurde die Reaktionslösung zu einer Mischung aus Ethylacetat (50 ml) und H2O (50 ml) gegeben. Nach Phasentrennung wurde die wäßrige Phase zweimal mit Ethylacetat (je 30 ml) extrahiert. Die kombinierten organischen Phasen wurden über $Na_2SO_4$ getrocknet und im Vakuum eingeengt. Der Rückstand wurde über Kieselgel mit Ethylacetat filtriert. Das nach Entfernen des Lösungsmittels erhaltene Produkt **(F-2)** lag in den meisten Fällen als ausreichend reines Öl vor und kristallisierte spontan. Gegebenenfalls wurde eine säulenchromatische Reinigung an Kieselgel durchgeführt. Ausbeute 95%
[2] S. J. Garden, R. B. da Silva, A. C. Pinto, Tetrahedron 2002, 58, 8399-8412

<u>**Baustein F-3:**</u>

**3-(2-Hydroxy-ethyl)-1H-indol (F-3)**

$LiAlH_4$ (1.99 g, 52.3 mmol) wurde in abs. THF (60 mL) unter Argon vorgelegt und (5-Hydroxy-1*H*-indol-3-yl)-essigsäure (5.00 g, 26.2 mmol) in abs. THF (100 mL) innerhalb von 30 min zugegeben. Der Ansatz wurde 3 h unter

Rückfluss gekocht. Zur Aufarbeitung wurde unter Eiskühlung zu dem Ansatz THF (10 mL) und $H_2O$ (4 mL) gegeben und 30 min nachgerührt. Der Ansatz wurde über Celite filtriert, mit Dichlormethan (150 mL) nachgespült und das Filtrat i. Vak. eingeengt.
Ausbeute: 2.17 g (47 %)
$^1$H-NMR (DMSO-$d_6$): 2.74 (2 H, m); 3.60 (3 H, m); 6.58 (1 H, m); 6.78 (1 H, s); 6.99 (1 H, s); 7.08 (1 H, d); 10.5 (1 H, bs).

__Beispiel AA-1:__

**4',9'-Dihydro-N,N-dimethyl-4-ethyl-spiro[cyclohexan-1,1'(3'H)-pyrano[3,4-b]indol]-4-amin, 2-hydroxy-1,2,3-propantricarboxylat (2:1) (Eines von 2 möglichen Diastereomeren)**

Tryptophol **F-1** (484 mg, 3.00 mmol) und Keton **E-1** (507 mg, 3.00 mmol) wurden in Dichlormethan (25 ml) gelöst und mit Methansulfonsäure (316 mg, 3.30 mmol) versetzt. Die Reaktionslösung wurde über Nacht bei Raumtemperatur gerührt. Es wurden erneut Methansulfonsäure (316 mg, 3.30 mmol) hinzugegeben und weitere 3 h gerührt. Die Reaktionslösung wurde mit 1 N NaOH alkalisch gestellt, die org. Phase abgetrennt und die wässrige Phase dreimal mit Dichlormethan (15 ml) extrahiert. Die vereinten org. Phasen wurden über $Na_2SO_4$ getrocknet, filtriert und das Solvens unter vermindertem Druck entfernt. Das Rohprodukt wurde säulenchromatographisch mit $CHCl_3$/EtOH (10:1) gereinigt.
*Ausbeute:* 672 mg (72 %); weißer Feststoff
*$^1$H-NMR (DMSO-$d_6$):* 0.85 (3 H, t); 1.23-1.75 (8 H, br. m); 2.14 (2 H, br. m); 2.28 (6 H, br. s); 2.01 (6 H, s); 2.66 (2 H, t); 3.89 (2 H, t); 6.93 (1 H, t); 7.01 (1 H, t); 7.29-7.37 (2 H, 2 d); 10.80 (br, 1 H).

Die Bildung des entsprechenden Citrats erfolgte mit dem soeben hergestellten Spiroether (0.66 g, 2.11 mmol) in heißem EtOH (10 ml) und Citronensäure (405 mg, 2.11 mmol) gelöst in heißem EtOH (2 ml). Es wurde 2 h bei Raumtemperatur gerührt. Der ausgefallene Feststoff **AA-1** wurde abgesaugt und getrocknet.
*Ausbeute:* 889 mg (82 %), weißer Feststoff **(AA-1)**
*Schmelzpunkt:* 240-242 °C
*$^1$H-NMR (DMSO-$d_6$):* 0.89 (3 H, t); 1.53 (2 H, m); 1.62 (4 H, br. t); 1.67 (2 H, br. t); 2.12 (2 H, br. t); 2.55 (6 H, s); 2.57-2.70 (4 H, m); 3.90 (2 H, t); 6.97 (1 H, t); 7.05 (1 H, t); 7.35-7.39 (2 H, 2 d); 10.73 (1 H, br).
*$^{13}$C-NMR (DMSO-$d_6$):* 8.86; 22.15; 23.47; 25.22; 37.17; 44.19; 59.08; 71.23; 72.07; 99.65; 105.28; 111.35; 117.62; 118.39; 120.65; 126.38; 135.61; 139.04; 171.84.

__Beispiel AA-2:__

**6'-Fluor-4',9'-dihydro-N,N-dimethyl-4-ethyl-spiro[cyclohexan-1,1'(3'H)-pyrano[3,4-b]indol]-4-amin, 2-hydroxy-1,2,3-propantricarboxylat (1:1) (Unpolareres Diastereomer)**

4-Dimethylamino-4-ethyl-cyclohexanon **E-1** (600 mg, 3.55 mmol) und 5-Fluor-tryptophol F-2 (852 mg, 3.55 mmol) wurden unter Argon in abs. $CH_2Cl_2$ (15 ml) vorgelegt und anschließend mit Methansulfonsäure (250 $\mu$l, 3.89 mmol) versetzt. Der Ansatz wurde 72 h bei Raumtemperatur gerührt, bis zur alkalischen Reaktion mit 1 N NaOH versetzt und mit $CH_2Cl_2$ (3 x 20 ml) extrahiert. Die org. Phase wurde über $Na_2SO_4$ getrocknet und i. Vak. eingeengt. Der Rückstand wurde durch FlasH-Chromatographie mit $CHCl_3$/MeOH (20:1, 4:1, 1:1+1% TEA) gereinigt.

Das dabei erhaltene, unpolarere Cyclisierungsprodukt (164 mg, 0.496 mmol) wurde in heißem Ethanol (5 ml) gelöst und die in heißem Ethanol gelöste Citronensäure (90 mg, 0.496 mmol) addiert. Der Ansatz wurde auf Raumtemperatur abgekühlt und der ausgefallene Niederschlag **AA-2** abgesaugt und i. Vak. getrocknet.
*Ausbeute:* 124 mg (7 %) **(AA-2)**
*Schmelzpunkt:* 233-236 °C
*$^1$H-NMR (DMSO-$d_6$):* 0.88 (3 H, t); 1.47 (2 H, m); 1.53-1.87 (8 H, m); 2.05 (2 H, t); 2.48 (6 H, m); 2.60 (4 H, m); 3.91 ((2 H, t); 6.83 (1 H, m); 7.12 (1 H, m); 7.35 (1 H, m); 10.74 (1 H, s).

__Beispiel AA-3:__

**2',3',4',9'-Tetrahydro-N,N-dimethyl-4-ethyl-spiro[cyclohexan-1,1'(1'H)-pyrido[3,4-b]indol]-4-amin, 2-hydroxy-1,2,3-propantricarboxylat (1:1) (Eines von 2 möglichen Diastereomeren)**

Tryptamin **H-1** (528 mg, 3.30 mmol) und Keton **E-1** (507 mg, 3.30 mmol) wurden in Methanol (15 ml) gelöst und über Nacht gerührt. Das Methanol wurde unter vermindertem Druck entfernt, der Rückstand in Dichlorethan (15 ml) aufgenommen und mit Trifluoressigsäure (494 mg, 3.30 mmol) versetzt. Die Reaktionslösung wurde 72 h bei Raumtemperatur gerührt, mit 1 N NaOH alkalisch gestellt, die org. Phase abgetrennt und die wässrige Phase dreimal mit Dichlormethan (15 ml) extrahiert. Die vereinten org. Phasen wurden über $NaSO_4$ getrocknet, filtriert und das Solvens unter vermindertem Druck entfernt. Das Rohprodukt wurde säulenchromatographisch mit $CHCl_3$/MeOH (1:4) gereinigt.

Ausbeute: 265 mg (26 %), weißer Feststoff
*$^1H$-NMR* (DMSO-$d_6$): 0.85 (3 H, t); 1.32-1.48 (6 H, br. m); 1.82 (2 H, br. t); 2.11 (2 H, br. t); 2.25 (6 H, s); 2.53 (2 H, t); 2.96 (2 H, t); 6.78 (2 H, dt); 6.95 (1 H, dt); 7.29 (2 H, d); 10.49 (br, 1 H,).
$^{13}C$-NMR (DMSO-$d_6$): 9.25; 22.87; 23.47; 26.21; 30.66; 38.66; 51.31; 55.49; 106.23; 110.85; 116.94; 117.64; 119.76; 126.77; 135.35; 144.85.

Die Bildung des entsprechenden Citrats erfolgte mit dem soeben hergestellten Spiroamin (0.25 g, 0.80 mmol) in heißem EtOH (10 ml) und Citronensäure (0.15 g, 0.80 mmol), gelöst in heißem EtOH (1 ml). Es wurde 2 h bei Raumtemperatur gerührt. Der ausgefallene Feststoff **AA-3** wurde abgesaugt und getrocknet.
*Ausbeute:* 347 mg (86 %), weißer Feststoff
*Schmelzpunkt:* 228-230 °C
*$^1H$-NMR (DMSO-$d_6$):* saubere, aber sehr breite Signale, daher keine Zuordnung. *$^{13}C$-NMR (DMSO-$d_6$):* 9.01; 23.77; 25.62; 28.83; 37.09; 44.23; 55.47; 71.23; 105.42; 111.22; 117.64; 118.52; 121.22; 125.82; 135.76; 171.01.

**<u>Beispiel AA-4:</u>**

**4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat (1:1) (Eines von 2 möglichen Diastereomeren)**

Das Cyclohexanon **E-2** (394 mg, 2 mmol) und Tryptophol F-1 (322 mg, 2 mmol) wurden unter Argon in abs. $CH_2Cl_2$ (15 ml) vorgelegt. Anschließend wurde Methansulfonsäure (142 μl, 2.2 mmol) zugegeben und 24 h bei Raumtemperatur gerührt.
Zur Aufarbeitung wurde der Ansatz mit 1 N NaOH versetzt und mit $CH_2Cl_2$ (3 x 15 ml) extrahiert. Die org. Phase wurde über $Na_2SO_4$ getrocknet und i. Vak. eingeengt. Der Rückstand wurde durch FlasH-Chromatographie mit $CHCl_3$/MeOH (9:1) gereinigt und anschließend aus Ethanol umkristallisiert.
*Ausbeute:* 330 mg (49 %)
Die NMR-Spektren der freien Base wurden ausgewertet, da die Spektren des Citrates schlecht aufgelöst waren.
*$^1H$-NMR (DMSO-$d_6$):* 0.91 (3 H, t); 1.25 (6 H, m); 1.55 (4 H, m); 1.73 (2 H, m); 2.11 (2 H, m); 2.26 (6 H, s); 2.66 (2 H, t); 3.91 (2 H, t); 6.98 (2 H, m); 7.32 (2 H, m); 10.72 (1 H, s).
*$^{13}C$-NMR (DMSO-$d_6$):* 14.04; 18.50; 22.18; 23.37; 26.61; 26.99; 29.93; 30.79; 37.24; 55.14; 55.97; 58.75; 71.99; 104.90; 111.21; 117.41; 118.19; 120.33; 126.42; 135.81; 139.74.

Die Bildung des entsprechenden Citrats erfolgte mit dem soeben hergestellten Spiroether (150 mg, 0.44 mmol) welcher in heißem Ethanol (5 ml) gelöst und zu welchem die in heißem Ethanol (1 ml) gelöste Citronensäure (84 mg, 0.44 mmol) addiert wurde. Anschließend wurde die Lösung auf Raumtemperatur abgekühlt und 2 h nachgerührt. Der ausgefallene weiße Niederschlag **AA-4** wurde abgesaugt und i. Vak. getrocknet.
*Ausbeute:* 180 mg (77 %) **(AA-4)**
*Schmelzpunkt:* 210-214 °C

**<u>Beispiel AA-5:</u>**

**6'-Fluor-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat (2:1) (Eines von 2 möglichen Diastereomeren)**

4-Butyl-4-dimethylamino-cyclohexanon **E-2** (394 mg, 2 mmol) und 5-Fluor-tryptophol **F-2** (482 mg, 2 mmol) wurden unter Argon in abs. $CH_2Cl_2$ (15 ml) vorgelegt und anschließend mit Trifluormethansulfonsäure (194 μl, 2.2 mmol) versetzt.
Der Ansatz wurde 72 h bei Raumtemperatur gerührt. Zur Aufarbeitung wurde die Lösung mit 1 N NaOH versetzt und mit $CH_2Cl_2$ (3 x 15 ml) extrahiert. Die org. Phase wurde über $Na_2SO_4$ getrocknet und i. Vak. eingeengt. Der Rückstand wurde durch FlasH-Chromatographie mit $CHCl_3$/MeOH (9:1 bis 1:1) gereinigt. Zur weiteren Reinigung wurde das Produkt aus Ethanol umkristallisiert.
*Ausbeute:* 119 mg (16 %)
Die NMR-Spektren der freien Base wurden ausgewertet, da die Spektren des Citrates schlecht aufgelöst waren.
*$^1H$-NMR (DMSO-$d_6$):* 0.90 (3 H, t); 1.19 (6 H, m); 1.54 (4 H, m); 1.67 (2 H, m); 2.12 (2 H, m); 2.24 (6 H, s); 2.59 (2 H, t); 3.88 (2 H, t); 6.83 (1 H, m); 7.12 (1 H, m); 7.28 (1 H, m); 10.85 (1 H, s).
*$^{13}C$-NMR (DMSO-$d_6$):* 14.03; 18.49; 22.10; 23.36; 26.59; 26.93; 29.87; 30.74; 37.22; 55.12; 55.97; 58.69; 72.01; 102.16; 102.39; 105.39; 108.00; 108.26; 111.90; 111.99; 126.55; 126.65; 132.43; 141.93; 155.56; 157.85.

Die Bildung des entsprechenden Citrats erfolgte mit dem soeben hergestellten Spiroether. Diese Spiroverbindung (119 mg, 0.33 mmol) wurde in heißem Ethanol (5 ml) gelöst und die in heißem Ethanol gelöste Citronensäure (63 mg, 0.33 mmol) addiert. Der Ansatz wurde auf Raumtemperatur abgekühlt, der ausgefallene weiße Niederschlag **(AA-**

**5)** abgesaugt und i. Vak. getrocknet.
Ausbeute: 106 mg (58 %) **(AA-5)**
Schmelzpunkt: 217-220 °C

**Beispiel AA-6:**

**6'-Fluor-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat (1:1) (Eines von 2 möglichen Diastereomeren)**

Beispiel **AA-6** wurde analog zu Beispiel **AA-5** hergestellt. Bei der Citratfällung wurde allerdings anstatt des Hemicitrats das Citrat isoliert.

**E-2** (4.0 g/ 20.3 mmol) und Fluortryptophol **F-2** (4.89 g/ 20.3 mmol) wurden unter Argon in abs. $CH_2Cl_2$ (50 ml) vorgelegt. Anschließend wurde Methansulfonsäure (1.44 ml / 22.33 mmol) zugegeben und 48 h bei Raumtemperatur gerührt. Zur Aufarbeitung wurde der Ansatz mit 1 N NaOH versetzt und 10 min kräftig gerührt. Die Phasen wurden getrennt, die wässrige Phase mit $CH_2Cl_2$ (1 x 30 ml) extrahiert, dabei fiel ein Feststoff aus , der abgesaugt und aus Ethanol umkristallisiert wurde. Die organische Phase wurde über $Na_2SO_4$ getrocknet und i. Vak. eingeengt. Der Rückstand wurde ebenfalls aus Ethanol umkristallisiert. Beide Feststoffe waren Zielprodukt.
Ausbeute: 1.9 g (26 %)

Das soeben erhaltene Cyclisierungsprodukt (1.0 g, 2.77 mmol) wurde in heißem Ethanol (5 ml) gelöst. Die in heißem Ethanol gelöste Citronensäure (0.528 g, 2.77 mmol) wurde zugegeben. Der Ansatz wurde auf Raumtemperatur abgekühlt, dabei fiel ein weißer Niederschlag aus. Der Niederschlag **(AA-6)** wurde abgesaugt und i. Vak. getrocknet.
Ausbeute: 1.5 g (98 %) **(AA-6)**

**Beispiel AA-7:**

**6'-Hydroxy-4',9'-dihydro-N,N-dimethyl-4-butyl-spiro[cyclohexan-1,1'(3'H)-pyrano[3,4-b]indol]-4-amin, 2,2,2-trifluoracetat (1:1) (Eines von 2 möglichen Diastereomeren)**

3-(2-Hydroxy-ethyl)-1H-indol **F-3** (620 mg, 3.49 mmol) und Keton E-2 (680 mg, 3.49 mmol) wurden in abs.$CH_2Cl_2$ (100 ml) unter Argon vorgelegt, unter Eiskühlung mit TMS-triflat (686 µl, 3.55 mmol) in $CH_2Cl_2$ (2 ml) versetzt und 30 min bei Raumtemperatur gerührt. Der Ansatz wurde weitere 16 h bei Raumtemperatur gerührt. Zur Aufarbeitung wurde $H_2O$ (22 ml) und $K_2CO_3$ (490 mg, 3.55 mmol) addiert und bei Raumtemperatur 20 min nachgerührt. Die Phasen wurden getrennt. Die wässrige Phase wurde mit Dichlormethan (2 x 20 ml) extrahiert. Die organische Phase wurde über $Na_2SO_4$ getrocknet und i. Vak. eingeengt.

Das soeben erhaltene Cyclisierungsprodukt (100 mg, 0.273 mmol) wurde in heißem Ethanol (5 ml) gelöst. Die in heißem Ethanol gelöste Citronensäure (52 mg, 0.273 mmol) wurde zugegeben. Der Ansatz wurde auf Raumtemperatur abgekühlt, dabei fiel ein weißer Niederschlag aus. Der Niederschlag **AA-7** wurde i. Vak. getrocknet.
Ausbeute: 48 mg (31 %), laut NMR sind keine Citratsignale vorhanden

*Anmerkung:* Wahrscheinlich wurde durch ein Versehen Trifuoroessigsäure mit eingeschleppt, so dass nicht das gewünschte Citrat sondern ein Trifluoroessigsäuresalz erhalten wurde.
Ausbeute: 109 mg (9 %) **(AA-7)**
Schmelzpunkt: 265-269 °C
1H-NMR (DMSO-d6): 0.94 (3 H, t); 1.29 (4 H, m); 1.60 (2 H, m); 1.81 (4 H, t); 1.96 (2 H, t); 2.40 (4 H, m); 2.59 (6 H, m); 3.87 (2 H; t); 6.55 (1 H, d); 6.70 (1 H, s); 7.04 (1 H, d); 8.54 (1 H, s); 9.45 (1 H, bs): 10.98 (1 H, bs).
13C-NMR (DMSO-d6): 13.77; 22.14; 22.70; 24.86; 26.07; 29.10; 30.94; 37.12; 59.36; 66.16; 70.51; 101.97; 104.52; 110.81; 111.05; 126.74; 129.56; 138.88; 150.28 (freie Base).

**Beispiel AA-8:**

**6'-Hydroxy-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat (2:3) (Eines von 2 möglichen Diastereomeren**

3-(2-Hydroxy-ethyl)-1*H*-indol **F-3** (2.68 g, 15.09 mmol) und Keton E-2 (2.94 g, 15.09 mmol) wurden in abs.$CH_2Cl_2$ (100 ml) unter Argon vorgelegt und unter Eiskühlung mit dem Triflat (2.96 ml, 15.34 mmol) in $CH_2Cl_2$ (5 ml) versetzt und 30 min bei Raumtemperatur gerührt. Der Ansatz wurde weitere 16 h bei Raumtemperatur gerührt. Zur Aufarbeitung wurde $H_2O$ (110 ml) und $K_2CO_3$ (2.45 g) zugegeben und bei Raumtemperatur 20 min gerührt. Die Phasen wurden getrennt. Die wässrige Phase wurde mit Dichlormethan (2 x 20 ml) extrahiert. Die organische Phase wurde über $Na_2SO_4$ getrocknet und i. Vak. eingeengt. Der Rückstand wurde durch FlasH-Chromatographie mit $CHCl_3$/MeOH (9:1) gereinigt

und in Essigester umkristallisiert.
Ausbeute: 476 mg (9 %)

Der soeben erhaltene Spiroether (471 mg, 1.32 mmol) wurde in heißem Ethanol (5 ml) gelöst. Die in heißem Ethanol gelöste Citronensäure (245 mg, 1.32 mmol) wurde zugegeben. Der Ansatz wurde auf Raumtemperatur abgekühlt, dabei fiel kein weißer Niederschlag aus. Der Ansatz wurde i. Vak. zur Trockene eingeengt.
Ausbeute: 524 mg (72 %) **(AA-8)**

**<u>Beispiel AA-9:</u>**

**2',3',4',9'-Tetrahydro-N,N-dimethyl-4-butyl-spiro[cyclohexan-1,1'(1'H)-pyrido[3,4-b]indol]-4-amin, 2-hydroxy-1,2,3-propantricarboxylat (1:1) (Unpolareres Diastereomer)**

Tryptamin H-1 (2,43 g, 15,2 mmol) und das Keton **E-2** (3,0 g, 15,2 mmol) wurden in abs. Methanol (90 ml) gelöst und 25 h bei Raumtemperatur unter Argon gerührt. Die Reaktionsmischung wurde anschließend eingeengt. Der Rückstand wurde in abs. 1,2-Dichlorethan (150 ml) gelöst, schnell mit Trifluoressigsäure (10,4 ml, 15,5 g, 136 mmol) versetzt und 3 d bei Raumtemperatur gerührt. Die braune Lösung wurde unter Eiskühlung mit 1 N Natronlauge (130 ml) versetzt und 20 min bei Raumtemperatur gerührt. Die Phasen der Lösung wurden getrennt. Die wäßrige Phase wurde mit 1,2-Dichlorethan (2 × 70 ml) extrahiert. Die organischen Phasen wurden vereinigt, mit Wasser (50 ml) gewaschen, getrocknet und eingeengt. Der braune ölige Rückstand wurde mit Methanol (60 ml) versetzt, wodurch es zur Kristallisation kam. Die Suspension wurde noch 10 min gerührt. Die farblosen Kristalle wurden abgesaugt und mit Methanol (60 ml) gewaschen (1,28 g). Es handelte sich hierbei um das reine unpolarere Spiroamin. Das Filtrat wurde eingeengt und der erhaltene braune Feststoff wurde erneut mit Methanol (50 ml) versetzt und 1 h im Eisbad gerührt. Nach Absaugen und Waschen mit kaltem Methanol (20 ml) konnten 673 mg des unpolareren Spiroamins gewonnen werden.

Das Filtrat wurde eingeengt und der Rückstand (2,4 g) chromatographisch aufgetrennt [Kieselgel 60 (130 g); Methanol (500 ml), Methanol/Triethylamin (100 : 1, 1,5 l)]. Das unpolarere Spiroamin wurde zusammen mit Verunreinigungen erhalten (1,02 g). Diese Fraktion wurde mit kaltem Methanol (10 ml) versetzt und abgesaugt. Der erhaltene Feststoff (332 mg) war reines unpolares Produkt. Das unpolarere Spiroamin wurde in einer Gesamtausbeute von 44 % (2,28 g) mit einem Schmelzpunkt von 180-182 °C erhalten. Das polarere Spiroamin wurde in einer weiteren Fraktion in einer Ausbeute von 12 % (622 mg) mit einem Schmelzpunkt von 93-96 °C gewonnen.

Das soeben hergestellte unpolarere Spiroamin (92 mg, 0.27 mmol) wurde in heißem Ethanol (5 ml) gelöst. Die in heißem Ethanol gelöste Citronensäure (51 mg, 0.27 mmol) wurde zugegeben. Der Ansatz wurde auf Raumtemperatur abgekühlt, dabei fiel ein weißer Niederschlag aus. Der Niederschlag **AA-9** wurde abfiltriert und i. Vak. getrocknet.
Ausbeute: 58 mg (40 %) **(AA-9)**

**<u>Beispiel AA-10:</u>**

**2',3',4',9'-Tetrahydro-N,N-dimethyl-4-butyl-2'-methylcarbonyl-spiro[cyclohexan-1,1'(1'H)-pyrido[3,4-b]indol]-4-amin, 2-hydroxy-1,2,3-propantricarboxylat (1:1) (Unpolareres Diastereomer)**

Acetylchlorid (0,126 ml, 139 mg, 1,77 mmol) wurde unter Argon in abs. Dichlormethan (5 ml) gelöst und bei Raumtemperatur mit der freien Base des unpolareren Spiroamins **AA-9** (200 mg, 0,59 mmol), gelöst in Dichlormethan (15 ml), innerhalb von 30 min versetzt. Nach 15 min war eine Fällung sichtbar, die sich zum Ende der Zugabe wieder aufgelöst hatte. Nach einer Reaktionszeit von 30 min entstand erneut eine Fällung. Es wurde noch weitere 21 h bei Raumtemperatur gerührt. Zur Aufarbeitung wurde der farblose Ansatz mit Wasser (10 ml) und 1N Natronlauge (5 ml) versetzt und 1 h gerührt. Die Phasen wurden getrennt. Die wäßrige Phase wurde mit Dichlormethan (20 ml) extrahiert. Die vereinigten organischen Phasen wurden mit Wasser (20 ml) gewaschen, getrocknet und eingeengt. Dabei wurde ein beigefarbenes Öl (277 mg) erhalten, das chromatographisch aufgetrennt wurde [Kieselgel 60 (35 g); Ethylacetat/Methanol (20 : 1, 300 ml)].
*Ausbeute:* 56 % (125 mg)
*Schmelzpunkt:* 163-166 °C

Das soeben hergestellte unpolarere Amid (125 mg, 0,327 mmol) wurde bei 50 °C in Ethanol (5 ml) gelöst und mit einer ethanolischen Lösung (3 ml) von Citronensäure (70 mg, 0,36 mmol) versetzt. Nach einer Reaktionszeit von 3 h bei Raumtemperatur wurde das farblose Citrat AA-10 durch Filtration abgetrennt und mit Ethanol (2 × 3 ml) gewaschen. Das unpolarere Amid wurde als Citrat in einer Ausbeute von 63 % (118 mg) mit einem Schmelzpunkt von 220-222 °C erhalten.

**Beispiel AA-11:**

**2',3',4',9'-Tetrahydro-N,N-dimethyl-4-butyl-2'-cyclopentylcarbonyl-spiro[cyclohexan-1,1'(1'H)-pyrido[3,4-b]indol]-4-amin, 2-hydroxy-1,2,3-propantricarboxylat (2:1) Unpolareres Diastereomer**

Cyclopentancarbonsäurechlorid (0,215 ml, 234 mg, 1,77 mmol) wurde unter Argon in abs. Dichlormethan (5 ml) gelöst und bei Raumtemperatur mit dem unpolareren Spiroamin (unpolarere freie Base aus **AA-9,** 200 mg, 0,59 mmol), gelöst in Dichlormethan (15 ml), innerhalb von 45 min versetzt. Es wurde noch weitere 1,5 h bei Raumtemperatur gerührt. Zur Aufarbeitung wurde der farblose Ansatz mit Wasser (10 ml) und 1 N Natronlauge (5 ml) versetzt und 1 h gerührt. Die Phasen wurden getrennt. Die wäßrige Phase wurde mit Dichlormethan (20 ml) extrahiert. Die vereinigten organischen Phasen wurden mit Wasser (20 ml) gewaschen, getrocknet und eingeengt. Dabei wurde ein beigefarbenes Öl (325 mg) erhalten, das chromatographisch aufgetrennt wurde [Kieselgel 60 (40 g); Ethylacetat, (350 ml)]. Das Amid wurde als farbloser hygroskopischer Feststoff in einer Ausbeute von 87 % (222 mg) isoliert.

Das soeben erhaltene Amid (186 mg, 0,427 mmol) wurde bei 60 °C in Ethanol (8 ml) gelöst und mit einer ethanolischen Lösung (3 ml) von Citronensäure (90 mg, 0,47 mmol) versetzt. Eine Fällung begann sofort. Nach einer Reaktionszeit von 2 h bei Raumtemperatur wurde das farblose Citrat **AA-11** durch Filtration abgetrennt und mit Ethanol (2 × 3 ml) gewaschen. Das unpolarere Amid wurde als Citrat in einer Ausbeute von 69 % (183 mg) mit einem Schmelzpunkt von 228-230 °C erhalten.

**Beispiel AA-12:**

**2',3',4',9'-Tetrahydro-N,N-dimethyl-4-butyl-2'-(2,2)-dimethylpropancarbonyl-spiro[cyclohexan-1,1'(1'H)-pyrido [3,4-b]indol]-4-amin, 2-hydroxy-1,2,3-propantricarboxylat (1:1) (Unpolareres Diastereomer)**

3,3-Dimethylbuttersäurechlorid (0,246 ml, 238 mg, 1,77 mmol) wurde unter Argon in abs. Dichlormethan (5 ml) gelöst und bei Raumtemperatur mit der freien Base des unpolareren Spiroamins **AA-9** (200 mg, 0,59 mmol), gelöst in Dichlormethan (15 ml), innerhalb von 30 min versetzt. Nach einer Reaktionszeit von 24 h wurde die gelbe Reaktionslösung mit Wasser (10 ml) und 1 N Natronlauge (5 ml) versetzt und 1 h gerührt. Die Phasen wurden getrennt. Die wäßrige Phase wurde mit Dichlormethan (20 ml) extrahiert. Die vereinigten organischen Phasen wurden mit Wasser (20 ml) gewaschen, getrocknet und eingeengt. Dabei wurde ein beigefarbenes Öl (322 mg) erhalten, das chromatographisch aufgetrennt wurde [Kieselgel 60 (40 g); Ethylacetat (250 ml), Ethylacetat/Methanol (4:1, 400 ml), Methanol (300 ml)]. Das Amid wurde als farbloses Öl nur in einer Ausbeute von 7 % (40 mg) gewonnen.

Die Acylierung wurde wie zuvor beschrieben wiederholt. Die Reaktionslösung blieb farblos. Der Abbruch der Reaktion erfolgte aber bereits nach 1,5 h. Nach chromatographischer Auftrennung des Reaktionsgemisches [Kieselgel 60 (40 g); Ethylacetat (250 ml)] wurde das Amid nun in einer Ausbeute von 78 % (200 mg) als farbloser Feststoff mit einem Schmelzpunkt von 220-222 °C gewonnen.

Das gewonnene unpolarere Amid (230 mg, 0,525 mmol) wurde bei 50 °C in Ethanol (8 ml) gelöst und mit einer ethanolischen Lösung (4 ml) von Citronensäure (111 mg, 0,578 mmol) versetzt. Nach einer Reaktionszeit von 16 h bei Raumtemperatur wurde das farblose Citrat durch Filtration abgetrennt und mit Ethanol (2 × 3 ml) gewaschen.

Das unpolarere Spiroamin **AA-12** wurde als Citrat in einer Ausbeute von 66 % (219 mg) mit einem Schmelzpunkt von 216-218 °C erhalten.

**Beispiel AA-13:**

**2',3',4',9'-Tetrahydro-N,N-dimethyl-4-butyl-2'-(3,4-dimethoxybenzylcarbonyl)-spiro[cyclohexan-1,1'(1'H)-pyrido[3,4-b]indol]-4-amin, 2-hydroxy-1,2,3-propantricarboxylat (1:1) (Unpolareres Diastereomer)**

3,4-Dimethoxyphenylacetylchlorid (380 mg, 1,77 mmol) wurde unter Argon in abs. Dichlormethan (5 ml) gelöst und bei Raumtemperatur mit der freien Base des unpolareren Spiroamins **AA-9** (200 mg, 0,59 mmol), gelöst in Dichlormethan (15 ml), innerhalb von 50 min versetzt. Es entstand sofort eine Fällung. Es wurde noch weitere 1,5 h bei Raumtemperatur gerührt. Zur Aufarbeitung wurde der Ansatz mit Wasser (10 ml) und 1 N Natronlauge (5 ml) versetzt und 1 h gerührt. Die Phasen wurden getrennt. Die wäßrige Phase wurde mit Dichlormethan (20 ml) extrahiert. Die vereinigten organischen Phasen wurden mit Wasser (20 ml) gewaschen, getrocknet und eingeengt. Dabei wurde ein beigefarbenes Öl (357 mg) erhalten, das chromatographisch aufgetrennt wurde [Kieselgel 60 (40 g); Ethylacetat, (250 ml), Ethylacetat/Methanol (8:1, 200 ml)]. Das Amid wurde als farbloser Feststoff in einer Ausbeute von 75 % (230 mg) mit einem Schmelzpunkt von 135-140 °C isoliert.

Das soeben gewonnene unpolarere Amid (216 mg, 0,417 mmol) wurde bei 60 °Cin Ethanol (11 ml) gelöst und mit einer ethanolischen Lösung (3 ml) von Citronensäure (89 mg, 0,46 mmol) versetzt. Nach einer Reaktionszeit von 5

h bei Raumtemperatur wurde das farblose Citrat durch Filtration abgetrennt und mit Ethanol (2 × 3 ml) gewaschen. Das unpolarere Amid wurde als Citrat **AA-13** in einer Ausbeute von 92 % (270 mg) mit einem Schmelzpunkt von 188-190 °C erhalten.

**Beispiel AA-14:**

**2',3',4',9'-Tetrahydro-N,N-dimethyl-4-butyl-2'-ethylaminocarbonyl-spiro[cyclohexan-1,1'(1'H)-pyrido[3,4-b]indol]-4-amin, 2-hydroxy-1,2,3-propantricarboxylat (1:1) (Unpolareres Diastereomer, Summe Rotamere ca. 95%)**

Die freie Base des unpolareren Spiroamins **AA-9** (204 mg, 0,6 mmol) wurde in abs. Acetonitril (30 ml) suspendiert und mit Ethylisocyanat (0,052 ml, 47 mg, 0,66 mmol) versetzt. Die Reaktionsmischung wurde 6 h zum Rückfluß erhitzt. Die klare Lösung wurde eingeengt. Der ölige Rückstand wurde in Diethylether (20 ml) aufgenommen und mit Wasser (5 ml) gewaschen. Nach Trocknen und Einengen wurde der unpolarere Harnstoff als farbloser Feststoff in einer Ausbeute von 57 % (139 mg) mit einem Schmelzpunkt von 154-158°C erhalten.
Der soeben gewonnene unpolarere Harnstoff (139 mg, 0,4 mmol) wurde in Ethanol (10 ml) gelöst und mit einer ethanolischen Lösung (5 ml) von Citronensäure (85 mg, 0,44 mmol) versetzt. Nach einer Reaktionszeit von 20 h bei Raumtemperatur wurde das farblose Citrat durch Filtration abgetrennt. Da das Produkt eine schmierige Konsistenz hatte, wurde mit Diethylether (2 × 3 ml) gewaschen. Weiteres Produkt konnte aus dem Filtrat nicht gewonnen werden. Der unpolarere Harnstoff wurde als Citrat **AA-14** in einer Ausbeute von 38 % (90 mg) mit einem Schmelzpunkt von 215-231 °C erhalten.

**Beispiel AA-15:**

**2',3',4',9'-Tetrahydro-N,N-dimethyl-4-butyl-2'-4-methoxybenzylaminocarbonyl-spiro[cyclohexan-1,1'(1'H)-pyrido[3,4-b]indol]-4-amin (Polareres Diastereoisomer)**

4-Methoxybenzylisocyanat (0,75 mmol) wurde in abs. Acetonitril (30 ml) gelöst, mit Triethylamin (0,07 ml, 511 mg, 5 mmol) und der freien Base des unpolareren Spiroamins **AA-9** (170 mg, 0,5 mmol) versetzt. Die Reaktionsmischung wurde 6 h zum Sieden erhitzt, wobei die Reaktionslösung klar wurde. Da im DC keine Umsetzung erkennbar war, wurde weitere 7 h unter Rückfluß erhitzt. Der Ansatz wurde eingeengt. Der feste farblose Rückstand wurde mit Diethylether versetzt, die Suspension 15 min gerührt und dann abgesaugt. Der unpolarere Harnstoff **AA-15** wurde in einer Ausbeute von 92 % (200 mg) erhalten.

**Beispiel AA-16:**

**2',3',4',9'-Tetrahydro-N,N-dimethyl-4-butyl-2'-methyl-spiro[cyclohexan-1,1'(1'H)-pyrido[3,4-b]indol]-4-amin, 2-hydroxy-1,2,3-propantricarboxylat (1:1) (Unpolareres Diastereomer)**

Die freie Base des unpolareren Spiroamins **AA-9** (200 mg, 0,59 mmol) wurde mit Wasser (0,04 ml) versetzt und bei 0°C in 95-proz. Ameisensäure (0,6 ml, 732 mg, 15,9 mmol) gelöst. Bei dieser Temperatur wurde 37-proz. wäßrige Formaldehydlösung (0,46 ml, 178 mg, 5,9 mmol) hinzugefügt, 10 min im Eisbad gerührt und der Ansatz 1 h auf 100 °C erwärmt. Unter Eiskühlung wurde die beigefarbene Lösung mit Wasser (5 ml) und 1 N Natronlauge (15 ml) versetzt. Die trübe Mischung wurde 30 min bei Raumtemperatur gerührt, mit Dichlormethan (20 ml) versetzt und nochmals 30 min gerührt. Die Phasen wurden getrennt. Die wäßrige Phase wurde mit Dichlormethan (15 ml) extrahiert. Die vereinigten organischen Phasen wurden mit Wasser (15 ml) gewaschen, getrocknet und eingeengt. Der Rückstand (225 mg) war ein beigefarbenes Öl, das chromatographisch aufgetrennt wurde [Kieselgel 60 (40 g); Ethylacetat (250 ml)]. Das Spiroamin wurde als farbloser Feststoff in einer Ausbeute von 25 % (51 mg) gewonnen.

Das soeben gewonnene unpolarere Spiroamin (51 mg, 0,144 mmol) wurde bei 60 °C in Ethanol (2 ml) gelöst und mit einer ethanolischen Lösung (2 ml) von Citronensäure (64 mg, 0,316 mmol) versetzt. Nach einer Reaktionszeit von 6 h wurde das Citrat 5/6 als farbloser Feststoff abgesaugt, mit Ethanol (2 × 2 ml) und Diethylether (2 × 5 ml) gewaschen. Das unpolarere Spiroamin wurde als hygroskopisches Citrat **AA-16** in einer Ausbeute von 47 % (37 mg) erhalten

**Beispiel AA-17:**

**6'-Fluor-4',9'-dihydro-N-ethyl-N-methyl-4-butyl-spiro[cyclohexan-1,1'(3'H)-pyrano[3,4-b]indol]-4-amin, 2-hydroxy-1,2,3-propantricarboxylat (1:1) (Eines von 2 möglichen Diastereomeren)**

Eine Lösung von **E-7** (500 mg, 2 mmol) und 5-Fluortryptophol **F-2** (430 mg, 2.4 mmol) in wasserfreiem Dichlormethan (25 ml) wurde unter Eiskühlung mit Trifluormethansulfonsäure (450 mg, 265 μl, 3 mmol) versetzt und über Nacht bei Raumtemperatur gerührt. Anschließend wurde das Reaktionsgemisch mit 0.5 N Natronlauge (10 ml) versetzt, 2 h bei Raumtemperatur gerührt, die organische Phase abgetrennt und die wässrige Phase mit Dichlormethan (2 × 20 ml) extrahiert. Die vereinigten organischen Phasen wurden mit Natriumsulfat getrocknet und i. Vak. eingeengt. Das Rohprodukt wurde mittels Flashchromatographie (100 g, 20 × 4.0 cm) mit Cyclohexan / Ethylacetat (9:1) und 1 % Triethylamin gereinigt.
Ausbeute: 469 mg (53 %), weißer Feststoff
Schmelzpunkt: 112-121 °C
$^1$H-NMR (DMSO-$d_6$): 0.89 (t, 3H, J = 6.8 Hz); 1.13 (t, 3H, J = 6.9 Hz); 1.18-1.33 (m, 4H); 1.51-1.58 (m, 4H); 1.65-1.73 (m, 2H); 1.65-1.73 (m, 2H); 2.04-2.13 (m, 2H); 2.22(s, 3H); 2.40-2.48 (m, 2H); 2.62 (t, 2H, J = 5.3 Hz); 3.88 (t, 2H, J = 5.3 Hz); 6.80-6.88 (m, 1 H); 7.11 (dd, 1H, J = 9.8, 2.3 Hz); 7.31 (dd, 1H, J = 8.8, 4.6 Hz); 10.67 (s, 1H).
$^{13}$C-NMR:14.0; 14.9; 20.0; 22.1, 23.4; 26.6; 27.3 (2C); 29.9 (2C); 32.7; 42.5; 56.1; 58.6; 72.1; 102.3 (d, J = 23 Hz); 105.4 (d, J = 4 Hz); 108.2 (d, J = 26 Hz); 111.9 (d, J = 10 Hz); 126.1 (d, J = 10 Hz); 132.4; 141.9; 156.7 (d, J= 231).

Der hergestellte Spiroether (366 mg, 0.98 mmol) wurde in heißem Isopropanol (60 ml) mit Citronensäure (232 mg, 1.21 mmol) in Isopropanol (5 ml) versetzt. Der ausgefallene Niederschlag **AA-17** wurde abfiltriert und getrocknet.
Ausbeute: 203 mg (37 %), weißer Feststoff **AA-17**
Schmelzpunkt: 206-209 °C
$^1$H-NMR (DMSO-$d_6$): 0.86 (t, 3H, J = 6.9 Hz); 1.12-1.29 (m, 7H); 1.31-1.81 (m, 6H); 1.98-2.09 (m, 2H); 2.36 (s, 3H); 2.46-2.69 (m, 10H), 3.85 (t, 2H, J = 5.4 Hz); 6.82 (dt, 1 H, J = 9.3, 2.6 Hz); 7.09 (dd, 1 H, J = 9.8, 2.4 Hz); 7.30 (dd, 1 H, J = 8.7, 4.6 Hz); 10.55 (s, 1 H).

**Beispiel AA-18:**

**6'-Fluor-4',9'-dihydro-N-benzyl-N-methyl-4-butyl-spiro[cyclohexan-1,1'(3'H)-pyrano[3,4-b]indol]-4-amin (Eines von 2 möglichen Diastereomeren)**

Eine Lösung von **E-8** (500 mg, 1.73 mmol) und 2-(5-Fluor-1*H*-indol-3-yl)ethanol **F-2** (311 mg, 1.73 mmol) in wasserfreiem Dichlormethan (30 ml) wurde unter Eiskühlung mit Trifluormethansulfonsäure (346 mg, 204 μL, 2.30 mmol) versetzt und über Nacht bei Raumtemperatur gerührt. Das Reaktionsgemisch wurde anschließend mit 0.5 M Natronlauge (17 ml) versetzt und 1 h bei Raumtemperatur gerührt. Die Phasen wurden getrennt, die wässrige Phase mit Dichlormethan (3 × 20 ml) extrahiert, die vereinigten organischen Phasen mit Natriumsulfat getrocknet und i. Vak. eingeengt. Das Rohprodukt (954 mg) wurde durch Flashchromatographie (100 g, 20 × 3.6 cm) mit Cyclohexan / Ethylacetat (9:1) gereinigt.
Ausbeute: 424 mg (56 %), amorpher, weißer Feststoff **AA-18**
Schmelzpunkt: 58-62 °C
$^1$H-NMR (DMSO-$d_6$): 0.97 (t, 3H, J = 6.79 Hz); 1.38-1.49 (m, 6H); 1.77-1.87 (m, 4H); 1.88-1.96 (m, 4H); 2.10 (s, 3H); 2.63 (t, 2H, J = 5.2 Hz); 3.62 (s, 2H); 3.89 (t, 2H, J = 5.2 Hz); 6.87 (dt, 1H, J = 9.1 und 2.5 Hz); 7.13 (dd, 2H, J = 9.8 und 2.4 Hz); 7.24-7.35 (m, 5H); 11.03 (s, 1 H).
$^{13}$C-NMR (DMSO-$d_6$): 14.3; 22.1; 23.1; 25.1; 25.4; 26.3; 30.4; 31.5; 34.1; 53.4; 56.5; 58.8; 71.7; 102.4 (d, J = 23 Hz); 105.6 (d, J = 5 Hz); 108.3 (d, J = 26 Hz); 111.6 (d, J 11 Hz); 126.2; 126.6 (d, J = 10 Hz); 127.8; 128.=; 132.2; 141.7; 141.9; 156.7 (d, J = 231 Hz).

**Beispiel AA-19:**

**6'-Fluor-4',9'-dihydro-N-phenyl-4-butyl-spiro[cyclohexan-1,1'(3'H)-pyrano[3,4-b]indol]-4-amin (Eines von 2 möglichen Diastereomeren)**

Eine Lösung von **E-11** (368 mg, 1.5 mmol) und 2-(5-Fluor-1*H*-indol-3-yl)ethanol F-2 (269 mg, 1.5 mmol) in wasserfreiem Dichlormethan wurde bei 10 °C möglichst schnell mit Trifluormethansulfonsäure (300 mg, 177 μl, 2.0 mmol) versetzt und über Nacht bei Raumtemperatur gerührt. Zur Umsatzkontrolle wurde eine Probe (0.5 ml) entnommen, diese mit 0.5 N Natronlauge gewaschen und die organische Phase mit Natriumsulfat getrocknet. Nach vollständiger Umsetzung wurde das Reaktionsgemisch mit 0.5 N Natronlauge (10 ml) versetzt, 2 h bei Raumtemperatur gerührt, die

organische Phase abgetrennt, die wässrige Phase mit Dichlormethan (2 × 20 ml) extrahiert, die vereinigten organischen Phasen mit Natriumsulfat getrocknet und i. Vak. eingeengt. Anschließend wurde das Rohprodukt mittels Flashchromatographie (18 g, 20 × 2.0 cm) mit Cyclohexan / Ethylacetat (9:1) und 1 % Triethylamin gereinigt.
Ausbeute: 327 mg (54 %), weißer Feststoff **AA-19**
Schmelzpunkt: 150-162 °C
$^1$H-NMR (DMSO-$d_6$): 0.87 (t, 3H, J = 6.9 Hz); 1.25-1.35 (m, 4H); 1.77-1.97 (m, 10H); 2.64 (t, 2H, J = 5.2 Hz); 3.90 (t, 2H, J = 5.3 Hz); 4.92 (s, 1H): 6.50 (t, 1 H, J = 7.1 Hz); 6.75 (d, 2H, J = 7.9 Hz); 6.83-6.90 (m, 1H); 7.02 (t, 2H, J = 7.8 Hz); 7.14 (dd, 1H, J = 9.8, 2.5 Hz); 7.30 (dd, 1 H, J = 8.7, 4.6 Hz); 11.03 (s, 1 H).
$^{13}$C-NMR:14.2; 22.0; 22.7; 25.1; 30.7; 30.9; 31.1; 54.0; 58.8; 71.6; 102.5 (d, J = 23 Hz); 105.6 (d, J = 5 Hz); 108.3 (d, J = 26 Hz); 111.6 (d, J = 11 Hz); 115.2; 126.6 (d, J = 10 Hz); 128.5; 132.2; 141.6; 147.5; 156.7 (d, J = 237 Hz).

<u>**Beispiel AA-20:**</u>

**4-Butyl-6'-fluor-4-(*N*-morpholino)-1',3',4',9'-tetrahydrospiro[cyclohexan-1,1'-pyrano[3,4-b]indol] (Unpolareres Diastereomer)**

Zu einer Lösung von **E-12** (479 mg, 2 mmol) und 2-(5-Fluor-1 H-indol-3yl)ethanol **F-2** (358 mg, 2 mmol) in Dichlormethan (50 ml) wurde unter Eis-Wasser-Kühlung Trifluormethansulfonsäure (400 mg, 236 μl, 2.66 mmol) getropft. Das Reaktionsgemisch wurde 20 h bei Raumtemperatur gerührt, anschließend wurde mit 0.5 M Natronlauge (20 ml) versetzt und danach 3 h bei Raumtemperatur gerührt. Die organische Phase wurde abgetrennt, die wässrige mit Dichlormethan (3 × 20 ml) extrahiert, die vereinigten organischen Phasen wurden mit Natriumchlorid-Lösung (50 ml) gewaschen, mit Natriumsulfat getrocknet und i. Vak. eingeengt. Das Isomerengemisch (815 mg) wurde durch Flashchromatographie (100 g, 22 × 4 cm) mit Ethylacetat / Cyclohexan (1:3) getrennt.

*Fraktion* 1: Unpolares Diastereoisomer, **AA-20**
Ausbeute: 259 mg (32 %), weißer Feststoff
Schmelzpunkt: >250 °C
$^1$H-NMR ($CDCl_3$): 0.92 (t, 3H, *J* = 6.5 Hz); 1.19-2.10 (m, 14H); 2.58-2.65 (m, 4H); 2.75 (t, 2H, *J* = 5.3 Hz); 3.75-3.81 (m, 4H); 3.99 (t, 2H, *J* = 5.4 Hz); 6.91 (dt, 1H, *J* = 8.8, 1.8 Hz); 7.12 (dd, 1 H, *J* = 9.5, 2.5 Hz); 7.30-7.26 (m, 1 H), 7.55 (s, 1 H).
$^{13}$C-NMR ($CDCl_3$):14.1; 22.5; 23.8; 26.7 (2 C); 26.9; 30.3 (2 C); 33.4; 45.1(2 C); 56.1; 59.6; 68.5 (2 C); 72.3; 103.3 (d, *J* = 23 Hz); 107.5 (d, J = 4 Hz); 109.7 (d, *J* = 26 Hz); 111.3(d, *J*=10 Hz); 127.6 (d, *J* = 10 Hz); 132.1; 141.2; 157.9 (d, J = 235 Hz).

*Fraktion* 2: Polareres Diastereoisomer, vgl. Beispiel **AA-21**
Ausbeute: 335 mg (42 %), weißer Feststoff
Schmelzpunkt: 238-241 °C
$^1$H-NMR ($CDCl_3$): 0.98 (t, 3H, *J* = 6.4 Hz); 1.30-2.05 (m, 14H); 2.63-2.68 (m, 4H); 2.75 (t, 2H, *J* = 5.3 Hz); 3.68-3.72 (m, 4H); 3.99 (t, 2H, *J* = 5.4 Hz); 6.90 (dt, 1 H, *J* = 9.3, 2.4 Hz); 7.12 (dd, 1H, *J* = 9.4, 2.0 Hz); 7.24 (dd, 1H, *J* = 8.8, 4.3 Hz); 7.63 (s, 1 H).
$^{13}$C-NMR ($CDCl_3$):14.4; 22.4; 23.6; 25.3; 25.6 (2 C); 30.7; 32.4 (2 C); 45.7 (2 C); 56.4; 59.6; 68.2 (2 C); 71.9; 103.4 (d, *J* = 24 Hz); 107.8; 109.8 (d, *J* = 27 Hz); 111.3 (d, *J* = 9 Hz); 127.5; 132.1; 140.7; 158.0 (d, *J* = 234 Hz).

<u>**Beispiel AA-21:**</u>

**4-Butyl-6'-fluor-4-(*N*-morpholino)-1',3',4',9'-tetrahydrospiro[cyclohexan-1,1'-pyrano[3,4-b]indol] (Polareres Diastereomer)**

Das unter Beispiel AA-20 angefallene polarere Diastereomer wird als Beispiel **AA-21** weitergeführt.

**AA-21** (polareres Diastereoisomer)
Ausbeute: 335 mg (42 %), weißer Feststoff
Schmelzpunkt: 238-241 °C
$^1$H-NMR ($CDCl_3$): 0.98 (t, 3H, *J* = 6.4 Hz); 1.30-2.05 (m, 14H); 2.63-2.68 (m, 4H); 2.75 (t, 2H, *J* = 5.3 Hz); 3.68-3.72 (m, 4H); 3.99 (t, 2H, *J* = 5.4 Hz); 6.90 (dt, 1 H, *J* = 9.3, 2.4 Hz); 7.12 (dd, 1H, *J* = 9.4, 2.0 Hz); 7.24 (dd, 1H, *J* = 8.8, 4.3 Hz); 7.63 (s, 1 H).
$^{13}$C-NMR ($CDCl_3$):14.4; 22.4; 23.6; 25.3; 25.6 (2 C); 30.7; 32.4 (2 C); 45.7 (2 C); 56.4; 59.6; 68.2 (2 C); 71.9; 103.4 (d, *J* = 24 Hz); 107.8; 109.8 (d, *J* = 27 Hz); 111.3 (d, *J* = 9 Hz); 127.5; 132.1; 140.7; 158.0 (d, *J* = 234 Hz).

**Beispiel AA-22:**

**4',9'-Dihydro-N,N-dimethyl-4-methoxypropyl-spiro[cyclohexan-1,1'(3'H)-pyrano[3,4-b]indol]-4-amin, 2-hydroxy-1,2,3-propantricarboxylat (1:1) (Eines von 2 möglichen Diastereomeren)**

Das Keton **E-4** (275 mg, 1.26 mmol) und Tryptophol **F-1** (206 mg, 1.26 mmol) wurden in abs. Dichlormethan (10 ml) gelöst, unter Argon mit Methansulfonsäure (0.13 ml, 2.05 mmol) versetzt und 20 h bei Raumtemperatur gerührt. Nach Zugabe von 1 N NaOH (10 ml) und $CH_2Cl_2$ (20 ml) wurde noch 10 min nachgerührt, die Phasen getrennt, die wässrige Phase zweimal mit $CH_2Cl_2$ extrahiert, die vereinigten organischen Phasen mit Wasser gewaschen, über $Na_2SO_4$ getrocknet und die Lösung i. Vak. eingeengt. Der verbliebene Rückstand wurde durch FlasH-Chromatographie mit $CHCl_3$/MeOH (20:1) gereinigt.
Ausbeute: 327 mg (73 %)

Bei der Umsetzung mit einer molaren Menge an Citronensäure in Ethanol fiel das Citrat **AA-22** als Feststoff aus.
*Ausbeute:* 281 mg **(AA-22)**
*Schmelzpunkt:* 207-208 °C
*$^1$H-NMR (DMSO-$d_6$):* 1.35-1.56 (8 H, m); 1.71 (2 H; t); 2.14 (2 H, t); 2.26 (6 H, s); 2.64 (2 H, t); 3.25 (3 H, s); 3.36 (2 H s); 3.89 (2 H, t); 6.95 (2 H, m); 7.32 (2 H, m); 10.72 (1 H, bs), freie Base.
*$^{13}$C-NMR (DMSO-$d_6$):* 22.13; 24.27; 25.80; 27.78; 29.26; 37.16; 44.12; 57.81; 59.09: 71.16; 72.18; 105.25; 111.38; 117.58; 118.35; 120.62; 126.36; 135.63; 138.96; 171.95; 177.09, Citrat.

**Beispiel AA-23:**

**6'-Fluor-4',9'-dihydro-N,N-dimethyl-4-methoxypropyl-spiro[cyclohexan-1,1'(3'H)-pyrano[3,4-b]indol]-4-amin, 2-hydroxy-1,2,3-propantricarboxylat (1:1) (Unpolareres Diastereomer)**

Das Keton **E-4** (426 mg, 2 mmol) und 5-Fluor-tryptophol **F-2** (362 mg, 2 mmol) wurden in abs. Dichlormethan (10 ml) gelöst, unter Argon mit Methansulfonsäure (0.14 ml, 2.2 mmol) versetzt und bei Raumtemperatur 24 h gerührt. Nach Zugabe von 1 N NaOH (10 ml) wurden die Phasen getrennt, die wässrige Phase mit $CH_2Cl_2$(3 x 10 ml) extrahiert, die vereinigten organischen Phasen mit Wasser (10 ml) gewaschen, über $Na_2SO_4$ getrocknet und die Lösung i. Vak. eingeengt. Der verbliebene Rückstand wurde durch FlasH-Chromatographie mit $CHCl_3$/MeOH (20:1 reines Methanol) getrennt.

Ausbeute: 408 mg (54 %) unpolarere Verbindung
218 mg (29 %) polarere Verbindung

Bei der Umsetzung der unpolareren Verbindung mit einer molaren Menge an Citronensäure in Ethanol fiel das Citrat als farbloser Feststoff aus.
*Ausbeute:* 384 mg, unpolaren Verbindung **AA-23**
*Schmelzpunkt:* 210-213 °C
*$^1$H-NMR (DMSO-$d_6$):* 1.52 (4 H, m); 1.70 (4 H, m); 1.83 (2 H; m); 2.14 (2 H, m); 2.60-2.73 (12 H, m); 3.25 (3 H, s); 3.35 (2 H m); 3.89 (2 H, t); 6.83 (1 H, m); 7.13 (1 H, m); 7.36 (1 H, m); 10.91 (1 H, bs).

**Beispiel AA-24:**

**2',3',4',9'-Tetrahydro-N,N-dimethyl-4-(3-methoxypropyl)-spiro[cyclohexan-1,1'(1'H)-pyrido[3,4-b]indol]-4-amin, 2-hydroxy-1,2,3-propantricarboxylat (1:1) (Unpolareres Diastereomer)**

Das Keton **E-4** (426 mg, 2 mmol) und Tryptamin H-1 (320 mg, 2 mmol) wurden in abs. Methanol (10 ml) gelöst und 20 h bei Raumtemperatur gerührt. Anschließend wurde das Lösungsmittel i. Vak. entfernt, der Rückstand in DCE (20 ml) gelöst, mit Trifluoressigsäure (2 ml) versetzt und 5 h bei Raumtemperatur gerührt. Nach Zugabe von 1N NaOH (10 ml) und $CH_2Cl_2$ (10 ml) wurde noch 20 min nachgerührt, die Phasen getrennt, die wässrige Phase mit $CH_2Cl_2$ (2 x 10 ml) extrahiert, die vereinigten organischen Phasen mit Wasser (10 ml) gewaschen, über $Na_2SO_4$ getrocknet und die Lösung i. Vak. eingeengt. Der verbliebene Rückstand wurde durch FlasH-Chromatographie mit $CHCl_3$/MeOH (9:1 ohne Triethylamin $\Rightarrow$ 4:1 + 1 % Triethylamin) gereinigt.

Ausbeute: 350 mg (49 %) unpolarere Verbindung, verunreinigt mit Ausgangsketon
321 mg (45 %) polarere Verbindung, verunreinigt

Bei der Umsetzung der unpolaren Verbindung mit einer molaren Menge an Citronensäure in Ethanol fiel ein das Citrat **AA-24** farbloser Feststoff aus.
Ausbeute: 264 mg, unpolares Diastereomer **AA-24** (sauber)
Schmelzpunkt: 247-248 °C
$^1$H-NMR (DMSO-$d_6$): 1.44-1.55 (4 H, m); 1.79 (6 H; m); 2.33-2.63 (12 H, m); 2.86 (2 H, m); 3.25 (3 H, s); 3.38 (4 H m); 7.00 (1 H, m); 7.07 (1 H, m); 7.39 (2 H, m); 11.04 (1 H, bs).

**Beispiel AA-25:**

**4',9'-Dihydro-N,N-dimethyl-4-(4-methoxybutyl)-spiro[cyclohexan-1,1'(3'H)-pyrano[3,4-b]indol]-4-amin, 2-hydroxy-1,2,3-propantricarboxylat (1:1) (Eines von 2 möglichen Diastereomeren)**

Das Keton **E-3** (455 mg, 2 mmol) und Tryptophol **F-1** (326 mg, 2 mmol) wurden in abs. Dichlormethan (10 ml) gelöst, unter Argon mit Methansulfonsäure (0.14 ml, 2.2 mmol) versetzt und 24 h bei Raumtemperatur gerührt. Nach Zugabe von 1 N NaOH (15 ml) und $CH_2Cl_2$ (25 ml) wurde noch 10 min nachgerührt, dann die Phasen getrennt, die wässrige Phase zweimal mit $CH_2Cl_2$ (10 ml) ex-trahiert, die vereinigten organischen Phasen mit Wasser (10 ml) gewaschen, über $Na_2SO_4$ getrocknet und die Lösung i. Vak. eingeengt. Der verbliebene Rückstand wurde durch FlasH-Chromatographie mit $CHCl_3$/MeOH (20:1) gereinigt.
*Ausbeute:* 687 mg (93 %)
Bei der Umsetzung mit einer molaren Menge an Citronensäure in Ethanol fiel das Citrat **AA-25** als farbloser Feststoff aus.
*Ausbeute:* 152 mg, weißer Feststoff
*Schmelzpunkt:* 214-215 °C
*$^1$H-NMR (DMSO-$d_6$):* 1.33 (2 H, m); 1.51 (4 H; m); 1.75 (4 H, m) 1.95 (2 H, t); 2.14 (2 H, t); 2.66 (10 H, m); 3.31 (3 H, s); 3.36 (2 H t); 3.90 (2 H, s); 6.98 (2 H, m); 7.38 (2 H, m); 10.88 (1 H, bs), Citrat.
*$^{13}$C-NMR (DMSO-$d_6$):* 21.04; 22.16; 26.93; 29.90; 30.23; 30.91; 37.19; 55.17; 57.74; 58.75; 71.85; 104.91; 111.18; 117.41; 118.18; 120.33; 126.40; 135.81; 139.71, freie Base.

**Beispiel AA-26:**

**6'-Fluor-4',9'-dihydro-N,N-dimethyl-4-(4-methoxybutyl)-spiro[cyclohexan-1,1'(3'H)-pyrano[3,4-b]indol]-4-amin, 2-hydroxy-1,2,3-propantricarboxylat (1:1) (Polareres Diastereoisomer)**

Das Keton **E-3** (426 mg, 2 mmol) und 5-Fluor-tryptophol **F-2** (362 mg, 2 mmol) wurden in abs. Dichlormethan (10 ml) gelöst, unter Argon mit Methansulfonsäure (0.14 ml, 2.2 mmol) versetzt und bei Raumtemperatur 24 h gerührt. Nach Zugabe von 1N NaOH (10 ml) Reaktion der Lösung) wurden die Phasen getrennt, die wässrige Phase mit $CH_2Cl_2$ (3 x 10 ml) extrahiert, die vereinigten organischen Phasen mit Wasser (10 ml) gewaschen, über $Na_2SO_4$ getrocknet und die Lösung i. Vak. eingeengt. Der verbliebene Rückstand wurde durch FlasH-Chromatographie mit $CHCl_3$/MeOH (20: 1) getrennt.
*Ausbeute:* 613 mg (79 %)
Bei der Umsetzung mit einer molaren Menge an Citronensäure in Ethanol fiel das Citrat AA-26 als farbloser Feststoff aus.
*Schmelzpunkt:* 216-218 °C
*$^1$H-NMR (DMSO-$d_6$):* 1.12 (2 H, m); 1.50 (4 H; m); 1.68 (4 H, m) 1.86 (2 H, t); 2.06 (2 H, t); 2.56 (10 H, m); 3.22 (3 H, s); 3.34 (5 H m); 3.87 (2 H, s); 4.34 (1 H, bs); 6.81 (1 H, t); 7.11 (1 H, m); 7.34 (1 H, m); 10.81 (1 H, bs), Citrat.
*$^{13}$C-NMR (DMSO-$d_6$):* 21.04; 22.08; 26.88; 29.84; 30.23; 30.86; 37.10; 55.16; 57.74; 58.69; 71.84; 72.00; 102.16; 102.38; 105.37; 108.00; 111.89; 111.98; 126.65; 132.44; 141.91; 155.56; 157.85, freie Base.

**Beispiel AA-27:**

**2',3',4',9'-Tetrahydro-N,N-dimethyl-4-(4-methoxybutyl)-spiro[cyclohexan-1,1'(1'H)-pyrido[3,4-b]indol]-4-amin, 2-hydroxy-1,2,3-propantricarboxylat (1:1) (Unpolareres Diastereomer)**

Das Keton **E-3** (455 mg, 2 mmol) und Tryptamin **H-1** (320 mg, 2 mmol) wurden in abs. Methanol (10 ml) gelöst und bei Raumtemperatur 20 h gerührt. Anschließend wurde das Lösungsmittel i. Vak. entfernt, der Rückstand in DCE (20 ml) gelöst, mit Trifluoressigsäure (2 ml) versetzt und 5 h bei Raumtemperatur gerührt. Nach Zugabe von 1 N NaOH (10 ml) und $CH_2Cl_2$ (10 ml) wurde noch 30 min nachgerührt, die Phasen getrennt, die wässrige Phase mit $CH_2Cl_2$ (2 x 10 ml) extrahiert, die vereinigten organischen Phasen mit Wasser (10 ml) gewaschen, über $Na_2SO_4$ getrocknet und die

Lösung im Vakuum eingeengt. Der verbliebene Rückstand wurde durch FlasH-Chromatographie mit $CHCl_3$/MeOH (9: 1 ohne Triethylamin → 4:1 + 1 % Triethylamin) gereinigt.
*Ausbeute:* 273 mg (37 %), unpolarere Verbindung
335 mg (48 %), polarere Verbindung, verunreinigt

Bei der Umsetzung des unpolaren Diastereomers mit einer molaren Menge an Citronensäure in Ethanol fiel das Citrat **AA-27** als farbloser Feststoff aus.
*Ausbeute:* 204 mg, unpolares Diastereomer **AA-27**
***Schmelzpunkt:*** 236-240 °C
*$^1$H-NMR (DMSO-$d_6$):*1.40 (2 H, m); 1.63 (4 H, m); 1.80-2.08 (8 H; m); 2.52-266 (10 H, m); 3.12 (2 H, t); 3.26 (3 H s); 3.41 (2 H, m); 6.94 (1 H, m); 7.06 (1 H, m); 7.37 (2 H, m); 10.86 (1 H, bs).

**<u>REFERENZ-Beispiel AA-28:</u>**

**4',9'-Dihydro-N,N-dimethyl-4-cyclopentyl-spiro[cyclohexan-1,1'(3'H)-pyrano[3,4-b]indol]-4-amin, 2-hydroxy-1,2,3-propantricarboxylat (2:1) (Eines von 2 möglichen Diastereomeren)**

Das Keton **E-6** (235 mg, 1.1 mmol) und Tryptophol **F-1** (180 mg, 1.12 mmol) wurden in abs. Dichlormethan (5 ml) gelöst und unter Argon mit Methansulfonsäure (0.1 ml, 1.5 mmol) versetzt und 20 h bei Raumtemperatur gerührt. Nach Zugabe von 1N NaOH (5 ml) und $CH_2Cl_2$ (10 ml) wurde noch 10 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch FlasH-Chromatographie mit $CHCl_3$/ MeOH (20: 1) gereinigt.
Ausbeute: 361 mg, Substanzgemisch erhalten, bei der Umsetzung mit einer molaren Menge an Citronensäure in Ethanol fiel das Citrat **AA-28** als farbloser Feststoff aus.
*Ausbeute:* 302 mg (50 %), 1 Diastereomer **AA-28**
***Schmelzpunkt:*** 200-202 °C
*$^1$H-NMR (DMSO-$d_6$):* 1.35 (6 H, m); 1.61 (8 H, m); 1.98 (3 H, m); 2.36 (8 H; m); 2.84 (1 H, m); 2.59 (2 H; s); 3.74 (2 H, m); 6.83 (2 H, m); 7.23 (2 H, m); 10.63 (1 H, s). *$^{13}$C-NMR (DMSO-$d_6$):* 22.10; 23.87; 24.67; 28.15; 29.42; 38.26; 42.72; 43.46; 59.14; 71.29; **72.08; 105.32;** 111.24; **117.64; 118.41; 120.71;** 126.36; 135.51; 138.91; 171.40; 175.86.

**<u>REFERENZ-Beispiel AA-29:</u>**

**2',3',4',9'-Tetrahydro-N,N-dimethyl-4-cyclopentyl-spiro[cyclohexan-1,1'(1'H)-pyridö[3,4-b]indöl]-4-amin, 2-hydroxy-1,2,3-propantricarboxylat (1:1) (Eines von 2 möglichen Diastereomeren)**

Das Keton E-6 (209 mg, 1.0 mmol) und Tryptamin H-1 (160 mg, 1.0 mmol) wurden in abs. Methanol (10 ml) gelöst und bei Raumtemperatur 20 h gerührt. Anschließend wurde das Lösungsmittel i. Vak. entfernt, der Rückstand in Dichlorethan (10 ml) gelöst, mit Trifluor-essigsäure (1.0 ml) versetzt und 5 d bei Raumtemperatur gerührt. Nach Zugabe von 1 N NaOH (10 ml) und $CH_2Cl_2$ (10 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch FlasH-Chromatographie mit $CHCl_3$/MeOH (20: 1) gereinigt. Bei der Umsetzung mit einer molaren Menge an Citronensäure in Ethanol fiel das Citrat **AA-29** als ein farbloser Feststoff aus.
*Ausbeute:* 226 mg (64 %) 1 Diastereomer **AA-29**
*Citrat: Schmelzpunkt:* 229-230 °C
Da die NMR-Spektren des Citrates schlecht aufgelöst waren, wurden die NMR-Spektren der freien Basen angegeben.
*$^1$H-NMR* ***(DMSO-$d_6$):*** 1.43 (12 H, m); 1.80 (2 H, t); 2.07 (3 H, m); 2.35 (6 H, s); 2.55 (2 H, m); 3.00 (2 H, t); 3.37 (1 H, bs); 6.96 (2 H, m); 7.30 (2 H, m); 10. 55 (1 H, s). *$^{13}$C-NMR (DMSO-$d_6$):* 22.53; 24.57; 24.81; 28.04; 30.72; 37.85; 38.66; 43.97; 52.07; 57.12; 106.26; 111.00; 117.20; 117.90; 120.09; 126.89; 135.59; 141.62.

**<u>REFERENZ-Beispiel AA-30:</u>**

**4',9'-Dihydro-N,N-dimethyl-4-cyclohexyl-spiro[cyclohexan-1,1'(3'H)-pyrano[3,4-b]indol]-4-amin, 2-hydroxy-1,2,3-propantricarboxylat (2:1) (Eines von 2 möglichen Diastereomeren)**

Das Keton **E-5** (175 mg, 0.78 mmol) und Tryptophol **F-1** (126 mg, 0.78 mmol) wurden in abs. Dichlormethan (5 ml) gelöst und unter Argon mit Methansulfonsäure (0.07 ml, 1.1 mmol) versetzt und 72 h bei Raumtemperatur gerührt. Nach Zugabe von 1N NaOH (5 ml) und $CH_2Cl_2$ (10 ml) wurde noch 10 min nachgerührt, die Phasen getrennt, die

wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch FlasH-Chromatographie mit $CHCl_3$/MeOH (20:1) gereinigt. Bei der Umsetzung mit einer molaren Menge an Citronensäure in Ethanol fiel das Citrat **AA-30** farbloser Feststoff aus.
*Ausbeute:* 110 mg (39 %) 1 Diastereomer **AA-30**
*Citrat: Schmelzpunkt:* 230-231 °C
*$^1$H-NMR (DMSO-$d_6$):* 1.10 (6 H, m); 1.77 (12 H, m); 2.07 (2 H, m); 2.66 (10 H; m); 3.88 (2 H, m); 6.97 (2 H, m); 7.36 (2 H, m); 10.72 (1 H, s).
*$^{13}$C-NMR (DMSO-$d_6$):* 22.16; 24.64; 26.00; 28.77; 30.09; 43.01; 43.62; 59.01; 71.52; 72.16; 105.20; 111.24; 117.59; 118.35; 120.61; 126.43; 135.64; 139.26; 171.56; 176.14.

**REFERENZ-Beispiel AA-31:**

**6'-Fluor-4',9'-dihydro-N,N-dimethyl-4-cyclohexyl-spiro[cyclohexan-1,1'(3'H)-pyrano[3,4-b]indol]-4-amin, 2-hydroxy-1,2,3-propantricarboxylat (1:1) (Eines von 2 möglichen Diastereomeren)**

Das Keton **E-5** (137 mg, 0.61 mmol) und 5-Fluor-tryptophol F-2 (109 mg, 0.61 mmol) wurden in abs. Dichlormethan (4 ml) gelöst, unter Argon mit Methansulfonsäure (0.065 ml, 1.0 mmol) versetzt und bei Raumtemperatur 48 h gerührt. Nach Zugabe von 1 N NaOH (5 ml) und $CH_2Cl_2$ (10 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch FlasH-Chromatographie mit $CHCl_3$/MeOH (20:1) gereinigt. Bei der Umsetzung mit einer molaren Menge an Citronensäure in Ethanol fiel das Citrat **AA-31** als farbloser Feststoff aus.
*Ausbeute:* 172 mg (73 %), 1 Diastereomer **AA-31**
*Citrat: Schmelzpunkt:* 204-205 °C
*$^1$H-NMR (DMSO-$d_6$):* 1.11 (6 H, m); 1.43 (2 H, m); 1.56 (4 H, m); 1.77 (6 H, m); 2.06 (2 H, m); 2.57 (7 H; m); 3.00 (2 H, m); 6.90 (1 H, m); 6.98 (1 H, m); 7.30 (2 H, m); 10.51 (1 H, s).
*$^{13}$C-NMR (DMSO-$d_6$):* 22.04; 24.57; 25.97; 26.58; 28.72; 30.04; 38.38; 43.25; 58.93; 71.52; 72.11; 102.35; 102.58; 105.64; 108.52; 112.03; 126.56; 132.21; 171.32; 175.49.

**REFERENZ-Beispiel AA-32:**

**2',3',4',9'-Tetrahydro-N,N-dimethyl-4-cyclohexyl-spiro[cyclohexan-1,1'(1'H)-pyrido[3,4-b]indol]-4-amin, 2-hydroxy-1,2,3-propantricarboxylat (1:1) (Eines von 2 möglichen Diastereomeren)**

Das Keton **E-5** (175 mg, 0.78 mmol) und Tryptamin **H-1** (125 mg, 0.78 mmol) wurden in abs. Methanol (8 ml) gelöst und bei Raumtemperatur 20 h gerührt. Anschließend wurde das Lösungsmittel i. Vak. entfernt, der Rückstand in Dichlorethan (10 ml) gelöst, mit Trifluor-essigsäure (0.8 ml) versetzt und 4 h bei Raumtemperatur gerührt. Nach Zugabe von 1 N NaOH (5 ml) und $CH_2Cl_2$ (10 ml) wurde noch 20 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und die Lösung i. Vak. eingeengt. Der verbliebene Rückstand wurde durch FlasH-Chromatographie mit $CHCl_3$/MeOH (9:1) gereinigt.
*Ausbeute:* 160 mg (56 %) 1 Diastereomer
***Citrat:*** *Schmelzpunkt:* 228-229 °C
NMR-Spektren der freien Base:
*$^1$H-NMR (DMSO-$d_6$):* 1.13 (6 H, m); 1.72 (10 H, m); 1.97 (2 H, m); 2.59 (10 H; m); 3.88 (2 H, m); 6.86 (1 H, t); 7.14 (1 H, m); 7.32 (1 H, m); 10.74 (1 H, s).
*$^{13}$C-NMR (DMSO-$d_6$):* 22.58; 25.06; 26.32; 26.81; 28.85; 31.26; 38.22; 45.32; 51.91; 57.69; 72.11; 106.30; 110.97; 117.22; 117.91; 120.10; 126.94; 135.58; 141.69

Der soeben erhaltene Spiroether (140 mg, 0.38 mmol) wurde in heißem Ethanol (4 ml) gelöst und mit einer Lösung von Citronensäure (73 mg, 0.38 mmol) in Ethanol (2 ml) versetzt. Nach 2- stündigem Stehen im Kühlschrank wurde der entstandene Feststoff **AA-32** abgesaugt und i. Vak. getrocknet.
Ausbeute: 160 mg (75 %) **(AA-32)**
Schmelzpunkt: 228-229 °C

**Beispiel AA-33:**

**2',3',4',9'-Tetrahydro-N,N-dimethyl-4-butyl-spiro[cyclohexan-1,1'(1'H)-pyrido[3,4-b]indol]-4-amin, 2-hydroxy-1,2,3-propantricarboxylat (1:1) (Polareres Diastereomer)**

Der unter Beispiel **AA-9** hergestellte polarere Spiroether (90 mg, 0.26 mmol) wurde in heißem Ethanol (5 ml) gelöst. Die in heißem Ethanol gelöste Citronensäure (48 mg, 0.26 mmol) wurde zugegeben. Der Ansatz wurde auf Raumtemperatur abgekühlt, dabei fiel ein weißer Niederschlag aus. Der Niederschlag wurde abfiltriert und i. Vak. getrocknet.
Ausbeute: 89 mg (75 %) **(AA-33)**

**Beispiel AA-34:**

**6'-Fluor-4',9'-dihydro-N,N-dimethyl-4-ethyl-spiro[cyclohexan-1,1'(3'H)-pyrano[3,4-b]indol]-4-amin, 2-hydroxy-1,2,3-propantricarboxylat (1:1) (Polareres Diastereomer)**

Die freie Bases des polaren Spiroether aus Beispiel **AA-2** (142 mg, 0.429 mmol) wurde in heißem Ethanol (5 ml) gelöst und die in heißem Ethanol gelöste Citronensäure (78 mg, 0.429 mmol) addiert. Der Ansatz wurde auf Raumtemperatur abgekühlt und i. Vak. eingeengt.
*Ausbeute:* 212 mg (11 %) **(AA-34)**
*Schmelzpunkt:* 72-75 °C
*$^1$H-NMR (DMSO-$d_6$):* 1.05 (3 H, t); 1.64 (2 H, m); 1.94 (6 H, m); 2.48 (2 H, m); 2.55 (6 H, s); 3.89 (2 H, t); 6.87 (1 H, m); 7.14 (1 H, m); 7.29 (1 H, m); 11.04 (1 H, s).

**Beispiel AA-35:**

**2',3',4',9'-Tetrahydro-N,N-dimethyl-4-(3-methoxypropyl)-spiro[cyclohexan-1,1'(1'H)-pyrido[3,4-b]indol]-4-amin, 2-hydroxy-1,2,3-propantricarboxylat (1:1) (Polareres Diastereomer, Reinheit < 95%)**

Das Keton **E-4** (426 mg, 2 mmol) und Tryptamin **H-1** (320 mg, 2 mmol) wurden in abs. Methanol (10 ml) gelöst und 20 h bei Raumtemperatur gerührt. Anschließend wurde das Lösungsmittel i. Vak. entfernt, der Rückstand in DCE (20 ml) gelöst, mit Trifluoressigsäure (2 ml) versetzt und 5 h bei Raumtemperatur gerührt. Nach Zugabe von 1 N NaOH (10 ml) und $CH_2Cl_2$ (10 ml) wurde noch 20 min nachgerührt, die Phasen getrennt, die wässrige Phase mit $CH_2Cl_2$ (2 x 10 ml) extrahiert, die vereinigten organischen Phasen mit Wasser (10 ml) gewaschen, über $Na_2SO_4$ getrocknet und die Lösung i. Vak. eingeengt. Der verbliebene Rückstand wurde durch FlasH-Chromatographie mit $CHCl_3$/MeOH (9:1 ohne Triethylamin $\Rightarrow$ 4:1 + 1 % Triethylamin) gereinigt.
Ausbeute: 350 mg (49 %) unpolarere Verbindung, verunreinigt mit Ausgangsketon 321 mg (45 %) polare Verbindung, verunreinigt

Bei der Umsetzung der polaren Verbindung mit einer molaren Menge an Citronensäure in Ethanol fiel das Citrat **AA-35** als farbloser Feststoff aus.
Ausbeute: 267 mg, polares Diastereomer **AA-35**
Schmelzpunkt: 228-229 °C
*$^1$H-NMR (DMSO-$d_6$):* 1.65 (4 H, m); 1.88 (4 H; m); 2.05 (4 H, m); 2.47-2.59 (10 H, m); 2.69 (2 H, t); 3.18 (2 H, t); 3.30 (3 H s); 3.43 (2 H, m); 6.97 (1 H, m); 7.07 (1 H, m); 7.33 (2 H, m); 10.95 (1 H, bs).

**Beispiel AA-36:**

**6'-Fluor-4',9'-dihydro-N,N-dimethyl-4-methoxypropyl-spiro[cyclohexan-1,1'(3'H)-pyrano[3,4-b]indol]-4-amin, 2-hydroxy-1,2,3-propantricarboxylat (1:1) (Polareres Diastereomer)**

Das Keton **E-4** (426 mg, 2 mmol) und 5-Fluor-tryptophol **F-2** (362 mg, 2 mmol) wurden in abs. Dichlormethan (10 ml) gelöst, unter Argon mit Methansulfonsäure (0.14 ml, 2.2 mmol) versetzt und bei Raumtemperatur 24 h gerührt. Nach Zugabe von 1 N NaOH (10 ml) wurden die Phasen getrennt, die wässrige Phase mit $CH_2Cl_2$ (3 x 10 ml) extrahiert, die vereinigten organischen Phasen mit Wasser (10 ml) gewaschen, über $Na_2SO_4$ getrocknet und die Lösung i. Vak. eingeengt. Der verbliebene Rückstand wurde durch FlasH-Chromatographie mit $CHCl_3$/MeOH (20:1 $\Rightarrow$ reines Methanol) getrennt.

Ausbeute: 408 mg (54 %) unpolarere Verbindung
218 mg (29 %) polare Verbindung

Bei der Umsetzung der polaren Verbindung mit einer molaren Menge an Citronensäure in Ethanol fiel kein Niederschlag aus, daher wurde die Lösung eingeengt und ein weißer, amorpher Feststoff **AA-36** erhalten.
*Ausbeute:* 239 mg, polare Verbindung, **AA-36**
*$^1$H-NMR (DMSO-$d_6$):* 1.65 (4 H, m); 1.97 (8 H; m); 2.56-2.68 (12 H, m); 3.31 (3 H, s); 3.45 (2 H m); 3.89 (2 H, t); 6.88 (1 H, m); 7.17 (1 H, m); 7.32 (1 H, m); 11.06 (1 H, bs).

**Beispiel AA-37**

**2',3',4',9'-Tetrahydro-N,N-dimethyl-4-butyl-2'-ethylaminocarbonyl-spiro[cyclohexan-1,1'(1'H)-pyrido[3,4-b]indol]-4-amin (Polareres Diastereomer)**

Das polarere Spiroamin (freie Base aus **AA-9,** 133 mg, 0,39 mmol) wurde in abs. Acetonitril (30 ml) suspendiert und mit Ethylisocyanat (0,034 ml, 31 mg, 0,43 mmol) versetzt. Die Reaktionsmischung wurde 1,5 h zum Rückfluß erhitzt. Nach Abkühlen auf Raumtemperatur kristallisierte ein farbloser Feststoff aus. Nach Absaugen wurde der polarere Harnstoff **AA-37** in einer Ausbeute von 46 % (74 mg) mit einem Schmelzpunkt von 182-184°C erhalten.

**Beispiel AA-38:**

**4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat (1:1) (Polareres Diastereomer)**

Keton **E-2** (2.0 g/10.15 mmol) und Tryptophol **F-1** (1.63 g/10.15 mmol) wurden unter Argon in abs. Dichlormethan (70 ml) vorgelegt und anschließend mit Methansulfonsäure (720 µl/11.16 mmol) versetzt. Der Ansatz wurde 24 h bei Raumtemperatur gerührt. Zur Aufarbeitung wurde der Ansatz mit 1 N NaOH versetzt und mit Dichlormethan (3 x 15 ml) extrahiert. Die organische Phase wurde über $Na_2SO_4$ getrocknet und i. Vak. eingeengt. Der Rückstand wurde durch Flashchromatographie mit $CHCl_3$/MeOH (9:1,1:1) gereinigt.

Ausbeute: *Fraktion* 1: Unpolarere Diastereomer 2.18 g (mit Tryptophol verunreinigt)
*Fraktion* 2: Polareres Diastereomer 862 mg (25 %)

*Fraktion* 2 (862 mg, 2.52 mmol) wurde in heißem Ethanol (5 ml) gelöst. Die in heißem Ethanol gelöste Citronensäure (480 mg, 2.52 mmol) wurde zugegeben. Der Ansatz wurde auf Raumtemperatur abgekühlt, dabei fiel ein weißer Niederschlag aus. Der Niederschlag **AA-38** wurde abfiltriert und i. Vak. getrocknet.
Ausbeute: 476 mg (35 %), polar **AA-38**

**Beispiel AA-39:**

**2',3',4',9'-Tetrahydro-N,N-dimethyl-4-(4-methoxybutyl)-spiro[cyclohexan-1,1'(1'H)-pyrido[3,4-b]indol]-4-amin, 2-hydroxy-1,2,3-propantricarboxylat (1:1) (Polareres Diastereomer)**

Das Keton **E-3** (455 mg, 2 mmol) und Tryptamin **H-1** (320 mg, 2 mmol) wurden in abs. Methanol (10 ml) gelöst und bei Raumtemperatur 20 h gerührt. Anschließend wurde das Lösungsmittel i. Vak. entfernt, der Rückstand in DCE (20 ml) gelöst, mit Trifluoressigsäure (2 ml) versetzt und 5 h bei Raumtemperatur gerührt. Nach Zugabe von 1N NaOH (10 ml) und $CH_2Cl_2$ (10 ml) wurde noch 30 min nachgerührt, die Phasen getrennt, die wässrige Phase mit $CH_2Cl_2$ (2 x 10 ml) extrahiert, die vereinigten organischen Phasen mit Wasser (10 ml) gewaschen, über $Na_2SO_4$ getrocknet und die Lösung im Vakuum eingeengt. Der verbliebene Rückstand wurde durch FlasH-Chromatographie mit $CHCl_3$/MeOH (9: 1 ohne Triethylamin ⇒ 4:1 + 1 % Triethylamin) gereinigt.

Ausbeute: 273 mg (37 %), unpolarere Verbindung
335 mg (48 %), polare Verbindung, verunreinigt

Bei der Umsetzung des polaren Diastereomers mit einer molaren Menge an Citronensäure in Ethanol fiel das

Citrat **AA-39** als farbloser Feststoff aus.
*Ausbeute:* 223 mg, polares Diastereomer **AA-39**
*Schmelzpunkt:* 202-204 °C
*$^1$H-NMR (DMSO-$d_6$):* 1.41 (4 H, m); 1.53 (2 H, m); 1.73 (6 H; m); 2.31-2.61 (10 H, m); 2.84 (2 H, m); 3.35 (7 H, m); 7.01 (1 H, m); 7.09 (1 H, m); 7.41 (2 H, m); 10.95 (1 H, bs).

**Beispiel AA-40**

**6'-Fluor-4',9'-dihydro-N-benzyl-4-allyl-spiro[cyclohexan-1,1'(3'H)-pyrano[3,4-b]indol]-4-amin, 2-hydroxy-1,2,3-propantricarboxylat (2:1) (Eines von 2 möglichen Diastereomeren)**

Eine Lösung von **E-13** (398 mg, 1.64 mmol) und 2-(5-Fluor-1*H*-indol-3-yl)ethanol **F-2** (293 mg, 1.64 mmol) in absolutem Dichlormethan (20 ml) wurde bei Raumtemperatur mit Trifluormethansulfonsäure (328 mg, 556 µl, 2.18 mmol) versetzt und 16 h bei Raumtemperatur gerührt. Die Reaktionslösung wurde anschließend mit 0.5 M Natronlauge (10 ml) versetzt und 2 h bei Raumtemperatur gerührt. Die Phasen wurden getrennt und die wässrige Phase mit Dichlormethan (3 × 30 ml) extrahiert. Die vereinigten organischen Phasen wurden mit Natriumsulfat getrocknet und i. Vak. eingeengt.
Ausbeute: 649 mg (98 %), leicht gelblicher Feststoff
Schmelzpunkt: 45-48 °C
*$^1$H-NMR* (300 MHz, *$d_6$-DMSO):* 1.49-1.73 (m, 6H); 1.84 (t, *J* = 6.8 Hz, 1 H); 2.08 (dd, *J* = 15.5, 11.7 Hz, 2H); 2.21 (d, *J* = 7.0 Hz, 2H); 2.63 (t, *J* = 5.3 Hz, 2H); 3.67 (d, *J* = 6.4 Hz, 2H); 3.88 (t, *J* = 5.2 Hz, 2H); 4.95-5.16 (m, 2H); 5.94 (m, 1H); 6.79-6.90 (m, 1 H); 7.13 (dd, *J* = 9.9, 2.5 Hz, 1 H); 7.19-7.41 (m, 4H); 7.49 (d, *J* = 7.0 Hz, 2H); 10.86 (s, 1 H).
$^{13}$C-NMR (100 MHz, *$d_6$-DMSO):* 22.1; 29.3; 29.7; 38.9; 43.7; 45.1; 52.4; 58.8; 71.8; 102.4 (d, J = 23 Hz); 105.4; 108.2 (d, J 26 Hz); 111.7; 116.8; 126.4; 126.7; 127.9; 128.1; 132.1; 135.1; 141.8; 142.0; 156.4 (d, J = 231 Hz).

Einer der erhaltenen Spiroether (300 mg, 0.74 mmol) in Isopropanol wurde mit einer Lösung von Citronensäure (142 mg, 0.74 mmol) in Isopropanol (1.2 ml) bei 70 °C versetzt. Das Produkt fiel in der Kälte als Hemicitrat AA-40 aus.
Ausbeute: 389 mg (100 %), farblose Kristalle **AA-40**
Schmelzpunkt: 133 °C
$^1$H-NMR (300 MHz, *$d_6$-DMSO):* 1.58-1.80 (m, 6H); 1.96-2.18 (m, 2H); 2.32 (d, *J* = 7.2 Hz, 2H); 2.57 (d, *J* = 15.2 Hz, 1 H); 2.65 (dd, *J* = 12.8, 9.5 Hz, 3H); 3.84 (s, 2H); 3.88 (t, *J* = 5.2 Hz, 2H); 4.34 (s, 1H); 5.09-5.21 (m, 2H); 5.95 (tdd, *J* = 17.3, 10.0, 7.2 Hz, 1 H); 6.80-6.92 (m, 1 H); 7.14 (dd, *J* = 9.9, 2.5 Hz, 1 H); 7.24-7.45 (m, 4H); 7.49-7.57 (m, 2H); 10.72 (s, 1 H).
$^{13}$C-NMR (100 MHz, *$d_6$-DMSO):* 22.0; 28.6; 29.5; 38.8; 42.9; 43.5; 45.0; 54.5; 58.9; 71.5; 71.8; 102.4 (d, J = 23 Hz); 105.6; 108.3 (d, J = 23 Hz); 111.8; 117.8; 126.7; 127.0; 128.2; 128.8; 132.0; 134.2; 141.7; 156.3 (d, J = 231 Hz); 171.3; 175.8.

**Beispiel AA-41**

**6'-Fluor-4',9'-dihydro-N-phenyl-4-allyl-spiro[cyclohexan-1,1'(3'H)-pyrano[3,4-b]indol]-4-amin (Eines von 2 möglichen Diastereomeren)**

Eine Lösung von **E-10** (277 mg, 1.14 mmol) und 2-(5-Fluor-1*H*-indol-3-yl)ethanol **F-2** (170 mg, 1.14 mmol) in absolutem Dichlormethan (20 ml) wurde bei Raumtemperatur mit Trifluormethansulfonsäure (342 mg, 580 µl, 2.28 mmol) versetzt und 16 h bei Raumtemperatur gerührt. Die Reaktionslösung wurde anschließend mit 0.5 M Natronlauge (10 ml) versetzt und 2 h bei Raumtemperatur gerührt. Die Phasen wurden getrennt und die wässrige Phase mit Dichlormethan (3 × 30 ml) extrahiert. Die vereinigten organischen Phasen wurden mit Natriumsulfat getrocknet, i. Vak. eingeengt und der Rückstand durch Flashchromatographie (200 g, 20 × 5.6 cm) mit Cyclohexan / Ethylacetat (5 : 1→3 : 2) gereinigt.

**AA-41:**
Ausbeute: 296 mg (66 %), farbloser Feststoff
Schmelzpunkt: 52-54°C
$^1$H-NMR (300 MHz, *$d_6$-DMSO):* 1.60-2.14 (m, 8H); 2.64 (t, J = 5.1 Hz, 2H); 2.77 (d, *J* = 6.8 Hz, 2H); 3.90 (t, *J* = 5.1 Hz, 2H); 4.98 (s, 1H); 5.11 (dd, J = 13.7, 2.6 Hz, 2H); 5.73-5.91 (m, 1 H); 6.53 (t, *J* = 7.2 Hz, 1H); 6.80 (d, *J* = 7.7 Hz, 2H); 6.87 (dd, *J* = 9.6, 2.6 Hz, 1 H); 7.04 (t, *J* = 7.9 Hz, 2H); 7.15 (dd, *J* = 9.9, 2.6 Hz, 1H); 7.30 (dd, *J* = 8.7 Hz, 1 H); 11.06 (s, 1 H).
$^{13}$C-NMR (100 MHz, *$d_6$-DMSO):* 22.0; 30.4; 30.9; 35.4; 54.0; 58.9; 71.4; 102.5 (d, J = 23 Hz); 105.6; 108.3 (d, J = 26 Hz); 111.6; 115.5; 115.9; 117.1; 126.6; 128.5; 132.1; 135.1; 141.5; 147.1; 155.7 (d, J = 230 Hz).

**Beispiel AA-42**

**6'-Fluor-4',9'-dihydro-N-(4-methoxybenzyl)-4-allyl-spiro[cyclohexan-1,1'(3'H)-pyrano[3,4-b]indol]-4-amin (Eines von 2 möglichen Diastereomeren)**

Eine Lösung von **E-9** (843 mg, 3.08 mmol) und 2-(5-Fluor-1*H*-indol-3-yl)ethanol **F-2** (552 mg, 3.08 mmol) in absolutem Dichlormethan (30 ml) wurde bei Raumtemperatur mit Trifluormethansulfonsäure (600 mg, 4.0 mmol) versetzt und 72 h bei Raumtemperatur gerührt. Anschließend wurde weitere Trifluormethansulfonsäure (300 mg, 2.0 mmol) zugesetzt und erneut 16 h gerührt. Die Reaktionslösung wurde danach mit 0.5 M Natronlauge (10 ml) versetzt und 2 h bei Raumtemperatur gerührt. Die Phasen wurden getrennt und die wässrige Phase mit Dichlormethan (3 × 30 ml) extrahiert. Die vereinigten organischen Phasen wurden mit Natriumsulfat getrocknet und i. Vak. eingeengt.
Ausbeute: 1.32 g (99 %), gelblicher Feststoff **AA-42**
Schmelzpunkt: 54-56 °C
$^1$H-NMR (400 MHz, $d_6$-*DMSO*): 1.50 (d, J = 11.9 Hz, 2H); 1.72 (m, 4H); 1.91 (d, *J* = 14.4 Hz, 2H); 2.55 (d, *J* = 5.0 Hz, 2H); 2.64 (t, *J* = 5.0 Hz, 2H); 3.63 (d, *J* = 2.9 Hz, 2H); 3.72 (s, 3H); 3.88 (dd, *J* = 5.2, 4.8 Hz, 2H); 5.18 (m, 3H); 5.88-6.04 (m, 1H); 6.80-6.93 (m, 4H); 7.08-7.17 (m, 1H); 7.27 (m, 2H); 11.01 (s, 1 H).
$^{13}$C-NMR (100 MHz, $d_6$-*DMSO*): 22.0; 30.4; 31.1; 35.3; 44.1; 53.2; 54.9; 58.8; 71.8; 102.4 (d, J = 23 Hz); 105.6; 108.2 (d, J = 25 Hz); 108.5; 111.6; 113.4; 116.9; 126.6; 129.1; 132.1; 133.9; 135.5; 141.7; 156.3 (d, J = 233 Hz).

**Beispiel AA-43**

**N-{6'-Fluor-4',9'-dihydro-4-butyl-spiro[cyclohexane-1,1'(3'H)-pyrano[3,4-b]indol]-4-yl}-pyrrolidin, 2-hydroxy-1,2,3-propantricarboxylat (1:1) (Eines von 2 möglichen Diastereomeren)**

Eine Lösung von **E-14** (1.06 g, 4.7 mmol) und 2-(5-Fluor-1*H*-3-yl)ethanol **F-2** (854 mg, 4.7 mmol) in wasserfreiem Dichlormethan (60 ml) wurde unter Eiskühlung und Argon mit Trifluormethansulfonsäure (949 mg, 552 µL, 6.3 mmol) versetzt und 1 d bei Raumtemperatur gerührt. Danach wurde weitere Trifluormethansulfonsäure (300 mg, 174 µL, 2 mmol) zugesetzt und erneut 1 d bei Raumtemperatur gerührt. Anschließend wurde das Reaktionsgemisch mit 0.5 M Natronlauge (48 ml) versetzt und 20 min gerührt. Die Phasen wurden getrennt, die wässrige Phase mit Dichlormethan (2 × 20 ml) extrahiert und die vereinigten organischen Phasen mit Natriumsulfat getrocknet. Das Rohprodukt (1.8 g) wurde durch Flashchromatographie (180 g, 20 × 5.6 cm) mit Chloroform / Methanol (95:5) gereinigt.
Ausbeute: 370 mg (19 %), gelblicher Feststoff (Fraktion 1)
Das Produkt lag als Hydrochlorid vor. Vermutlich stammt der Chlorwasserstoff aus dem zur Chromatographie verwendeten Chloroform.
$^1$H-NMR ($CDCl_3$): 0.97 (t, 3H, J = 6.8 Hz), 1.35-1.41 (m, 4H); 1.46-1.52 (m, 2H); 1.57 (d, 2H, J = 14.6 Hz), 1.89-1.98 (m, 4H); 2.22 (dt, 2H, J = 14.6, 6.0 Hz), 2.35-2.45 (m, 2H); 2.72 (t, 2H, J = 5.3 Hz), 2.78 (dt, 2H, J = 14.6, 3.5 Hz); 3.10 (dt, 2H, J = 13.0, 6.9 Hz), 3.63 (dt, 2H, J = 12.2 und 6.6 Hz), 3.92 (t, 2H, J = 5.3 Hz), 6.81 (dt, 1H, J = 9.2 und 2.5 Hz), 7.06 (dd, 1 H, J = 9.7, 2.4 Hz), 7.37 (dd, 1H, J = 8.8, 4.5 Hz); 10.36 (br s, 1 H); 11.04 (s, 1 H).
$^{13}$C-NMR ($CDCl_3$): 13.9; 22.6; 23.4; 25.1; 26.6; 27.0; 29.5; 32.6; 48.2; 60.3; 66.5; 71.0; 102.4 (d, J = 23 Hz); 106.1 (d, J = 4 Hz); 109.2 (d, J = 10 Hz); 112.4 (d, J = 10 Hz); 126.3 (d, J = 10 Hz); 132.4; 139.8; 157.5 (d, J = 233 Hz).
Außerdem wurden noch verunreinigtes Produkt (Fraktion 2, 322 mg, 17 %) und nicht umgesetztes Keton (Fraktion 3, 227 mg, 23 %) erhalten.
Das $^1$H-NMR-Spektrum des rohen Produktgemisches zeigte, dass nur ein Diastereoisomer und das Alken entstanden waren, wobei letzteres aber nicht isoliert wurde.
Eine Lösung von Fraktion 1 (350 mg, 0.83 mmol) in Chloroform (20 ml) wurde mit Natriumhydrogencarbonat-Lösung gewaschen, die organische Phase mit Natriumsulfat getrocknet und i. Vak. eingeengt.
Ausbeute: 204 mg (70 %), amorpher, gelblicher Feststoff
Schmelzpunkt: 70 °C
$^1$H-NMR ($CDCl_3$): 0.93 (t, 3H, J = 6.7 Hz), 1.21-1.38 (m, 4H); 1.38-1.42 (m, 2H); 1.48 (d, 2H, J = 12.8 Hz); 1.74 (d, 2H, J = 12.8 Hz); 1.74-1.84 (m, 4H); 1.88 (dt, 2H, J = 13.5, 2.9 Hz); 2.04 (dt, 2H, J = 13.2, 3.2 Hz); 2.69 (t, 4 H, J = 5.8 Hz); 2.74 (t, 2 H, J = 5.4 Hz); 3.99 (t, 2H, J = 5.4 Hz); 6.87 (dt, 1H, J = 9.1, 2.5 Hz); 7.11 (dd, 1H, J = 9.5, 2.4 Hz); 7.23 (dd, 1 H, J = 8.7, 4.3 Hz); 7.90 (s, 1 H).
$^{13}$C-NMR ($CDCl_3$): 14.2; 22.5; 24.0; 24.1; 24.8; 27.0; 28.6; 30.8; 31.1; 44.1; 54.7; 59.7; 72.4; 103.2 (d, J = 24 Hz); 107.1 (d, J = 5 Hz); 109.4 (d, J = 26 Hz); 111.2 (d, J = 10 Hz); 127.6 (d, J = 10 Hz); 132.0; 141.7; 157.8 (d, J = 234 Hz).
Eine Lösung des soeben erhaltenen gelben Feststoffs (freie Base der Fraktion 1) (180 mg, 0.46 mmol) in heißen Ethanol (15 ml) wurde mit einer heißen Lösung von Citronensäure (90 mg, 0.46 mmol) in Ethanol (1.2 ml) versetzt. Dabei fiel ein weißer Niederschlag aus, der nach dem Abkühlen abfiltriert wurde.
Ausbeute: 137 mg (50 %), weißer Feststoff **(AA-43)**

Schmelzpunkt: 198-199 °C
$^1$H-NMR (DMSO-$d_6$): 0.92 (t, 3H, J = 6.7 Hz); 1.20-1.40 (m, 4H); 1.44-1.64 (m, 4H); 1.71 (br d, 2H, J = 12.7 Hz); 1.90 (br s, 6H); 2.12 (br t, 2H, J = 12.7 Hz); 2.57 (d, 2H, J = 15.0 Hz); 2.63 (t, 2H, J = 4 Hz); 2.66 (d, 2H, J = 15.0 Hz); 3.07 (br s, 4H); 3.89 (t, 2H, J = 5.1 Hz); 6.87 (dt, 1 H, J = 9.1, 2.4 Hz); 7.15 (dd, 1H, J = 9.9, 2.3 Hz); 7.37 (dd, 1 H, J = 8.5, 4.4 Hz); 10.64 (s, 1 H); ca. 11-12 (sehr br s, 2-3H).

**Beispiel AA-44**

**N-{6'-Fluor-4',9'-dihydro-4-butyl-spiro[cyclohexan-1,1'(3'H)-pyrano[3,4-b]indol]-4-yl}-piperidin (Unpolareres Diastereomer)**

Zu einer Lösung von **E-15** (860 mg, 3.6 mmol) und 2-(5-Fluor-1*H*-indol-3yl)ethanol **F-2** (645 mg, 3.6 mmol) in Dichlormethan (70 ml) wurde unter Eis-Wasser-Kühlung Trifluormethansulfonsäure (702 mg, 408 μl, 4.68 mmol) getropft. Das Reaktionsgemisch wurde 20 h bei Raumtemperatur gerührt, anschließend mit 0.5 *M* Natronlauge (36 ml) versetzt und danach 2.5 h bei Raumtemperatur gerührt. Die organische Phase wurde abgetrennt und die wässrige mit Dichlormethan (3 × 20 ml) extrahiert. Die vereinigten organischen Phasen wurden mit Natriumchlorid-Lösung (40 ml) gewaschen, mit Natriumsulfat getrocknet und i. Vak. eingeengt. Das Isomerengemisch (1.4 g) wurde durch Flashchromatographie (140 g, 23 × 5.4 cm) mit Ethylacetat / Cyclohexan (1:3→1:2) und danach mit Ethylacetat getrennt.

Fraktion 1 (unpolares Diastereoisomer)
Ausbeute: 98 mg (7 %), weißer Feststoff
Schmelzpunkt: 126-130 °C
$^1$H-NMR ($CDCl_3$): 0.92 (t, 3H, J = 6.8 Hz); 1.20-1.83 (m, 18H); 1.99-2.10 (m, 2H); 2.56 (m, 4H); 2.74 (t, 2H, J = 5.4 Hz); 3.99 (t, 2H, J = 5.4 Hz); 6.89 (dt, 1 H, J = 9.0, 2.5 Hz); 7.11 (dd, 1 H, J = 9.5, 2.5 Hz); 7.29-7.25 (m, 1 H), 7.62 (s, 1 H).
$^{13}$C-NMR ($CDCl_3$):14.2; 22.5; 23.9; 25.4; 27.0; 27.6 (2); 28.0 (2); 30.5 (2); 33.6; 45.7; 56.4; 59.6; 72.6; 103.2 (d, J = 23 Hz); 107.3 (d, J = 4 Hz); 109.5 (d, J = 26 Hz); 111.3 (d, J = 10 Hz); 127.6 (d, J = 10 Hz); 132.0; 141.6; 157.9 (d, J = 234 Hz).
Fraktion 2 (polares Diastereoisomer)
Ausbeute: 360 mg (25 %), farbloses Öl
$^1$H-NMR ($CDCl_3$): 0.97 (t, 3H, J = 6.4 Hz); 1.29-1.80 (m, 18H); 2.63-2.68 (m, 4H); 1.99 (t, 2H, J = 11.2 Hz); 2.54-2.63 (m, 4H); 2.74 (t, 2H, J = 5.4 Hz); 3.99 (t, 2H, J = 5.4 Hz); 6.89 (dt, 1H, J = 9.0, 2.4 Hz); 7.12 (dd, 1H, J = 9.4, 2.2 Hz); 7.21-7.25 (m, 1 H); 7.63 (s, 1 H).

**Beispiel AA-45**

**N-{6'-Fluor-4',9'-dihydro-4-butyl-spiro[cyclohexan-1,1'(3'H)-pyrano[3,4-b]indol]-4-yl)-piperidin, 2-hydroxy-1,2,3-propantricarboxylat (1:1) (Polareres Diastereomer)**

Zu einer heißen Lösung von des unter Beispiel **AA-44** hergestellten polareren Diastereomers (Fraktion 2, 220 mg, 0.55 mmol) in 2-Propanol (25 ml) wurde eine 0.5 M Lösung von Citronensäure in 2-Propanol (1.38 ml, 0.69 mmol) gegeben. Der ausgefallene Niederschlag wurde abfiltriert und i. Vak. getrocknet.
Ausbeute: 160 mg (49 %), weißer Feststoff **(AA-45)**
Schmelzpunkt: 236-238 °C
$^1$H-NMR (DMSO-$d_6$): 0.98 (t, 3H, J = 6.9 Hz); 1.21-2.06 (m, 20H); 2.56 (d, 2H, J = 15.1 Hz); 2.47 (d, 2H, J = 15.1 Hz); 2.65 (t, 2H, J = 5.1 Hz), 2.90 (br s, 4H), 3.90 (t, 2H, J = 5.1 Hz, 2H), 6.89 (ddd, 1H, J = 9.6, 8.9, 2.6 Hz); 7.16 (dd, 1 H, J = 9.9, 2.5 Hz); 7.29-7.35 (m, 1 H); 11.03 (s, 1 H).

**Beispiel AA-46**

**N-{6'-Fluor-4',9'-dihydro-4-butyl-spiro[cyclohexan-1,1'(3'H)-pyrano[3,4-b]indol]-4-yl}-n-methylpiperazin, 2-hydroxy-1,2,3-propantricarboxylat (1:2) (Eines von 2 möglichen Diastereomeren)**

Eine Lösung von E-16 (631 mg, 2.5 mmol) und 5-Fluortryptophol F-2 (449 mg, 2.5 mmol) in wasserfreiem Dichlormethan (25 ml) wurde unter Eiskühlung mit Trifluormethansulfonsäure (900 mg, 530 μl, 6 mmol) versetzt und über das Wochenende bei Raumtemperatur gerührt. Zur Umsatzkontrolle wurde eine Probe (0.5 ml) entnommen, diese mit 0.5 N Natronlauge gewaschen und die organische Phase mit Natriumsulfat getrocknet. Nach vollständiger Umsetzung wurde das Reaktionsgemisch mit 0.5 N Natronlauge (10 ml) versetzt, 2 h bei Raumtemperatur gerührt, die wässrige Phase mit Dichlormethan (2 × 20 ml) extrahiert, die vereinigten organischen Phasen wurden mit Natriumsulfat getrocknet

und i. Vak. eingeengt. Das Rohprodukt wurde mittels Flashchromatographie (200 g, 20 × 5.7 cm) mit Methanol gereinigt.

*Fraktion 1:*
Ausbeute: 144 mg (14.0 %),weißer Feststoff
Schmelzpunkt: 74-81 °C
$^1$H-NMR (DMSO-$d_6$): 0.88 (t, 3H, J= 6.7 Hz); 1.14-1.36 (m, 7H); 1.55 (t, 4H, J = 12.2 Hz); 1.68 (t, 2H, J = 12.2 Hz); 2.04 (t, 2H, J = 13.0 Hz); 2.23 (s, 3H); 2.42-2.48 (m, 4H); 2.52-2.57 (m, 3H); 2.62 (t, 2H, J= 5.4 Hz); 3.88 (t, 2H, J = 5.4 Hz); 6.86 (dt, 1 H, J = 9.3, 2.6 Hz); 7.12 (dd, 1H, J= 9.9, 2.5 Hz); 7.37 (dd, 1 H, J= 8.7, 4.6 Hz); 10.57 (s, 1 H).

Darüber hinaus wurde noch zwei Mischfraktionen von Fraktion 2 & 3 (652 und 213 mg, 84 %) als gelbes Öl erhalten die Spiroether und ein Nebenprodukt im Verhältnis von ca. 9:1 enthalten.

Eine Lösung von Fraktion 2 & 3 (796 mg, 1.93 mmol) in siedenden Ethanol (15 ml) wurde mit einer Lösung von Citronensäure (928 mg, 4.8 mmol) in heißem Ethanol (8 ml) versetzt. Nach einiger Zeit fiel ein weißer Niederschlag aus, der nach dem Abkühlen abfiltriert wurde.
Ausbeute: 675 mg (85 %), weißer Feststoff **(AA-46)**
Schmelzpunkt: 213-220 °C
$^1$H-NMR (DMSO-$d_6$): 0.90 (t, 3H, J = 6.9 Hz); 1.15-1.37 (m, 7H); 1.51-1.63 (m, 4H); 1.71 (t, 2H, J = 12.8 Hz); 1.99 (t, 2H, J = 13.0 Hz); 2.46-2.80 (m, 16H, überlagert vom DMSO-Signal); 3.12 (br s, 4H); 3.89 (t, 2H, J = 5.4 Hz); 6.89 (dt, 1 H, J = 9.4, 2.6 Hz); 7.15 (dd, 1 H, J = 9.8, 2.4 Hz); 7.35 (dd, 1 H, J = 8.7, 4.5 Hz); 10.49 (s, 1 H).
$^{13}$C-NMR (DMSO-$d_6$):14.0; 22.1; 23.2; 26.5; 26.7 (2C); 29.7 (2C); 34.1; 42.0; 42.8; 44.2 (2C); 54.3; 55.8; 58.7; 71.5; 72.0; 102.5 (d, J = 24 Hz); 105.8 (d, J = 5 Hz); 108.4 (d, J = 26 Hz); 111.8 (d, J = 11 Hz); 126.9 (d, J = 10 Hz); 132.3; 141.8; 156.8 (d, J = 231 Hz); 171.4 (2C); 176.8.

**Beispiel AA-47**

**4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat (1:1),Unpolareres Diastereomer**

Bei dem Beispiel **AA-47** handelt es sich um das Citrat des unter Beispiel **AA-38** angefallenen unpolareren Diastereomers (*Fraktion* 1). Dieses Citrat wurde nach dem Standardverfahren gefällt.

**Beispiel AA-48**

**6'-Hydroxy-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat (1:1) ,Unpolareres Diastereomer**

Indol **F-3** (2.17 g, 12.2 mmol) und Keton **E-2** (2.37 g, 12.2 mmol) wurden in abs. Dichlormethan (100 ml) unter Argon vorgelegt, unter Eiskühlung mit TMS-triflat (2.37 ml, 14.4 mmol) in Dichlormethan (5 ml) versetzt und 30 min bei RT gerührt. Der Ansatz wurde weitere 16 h bei RT gerührt. Zur Aufarbeitung wurde $H_2O$ (85 ml) und $K_2CO_3$ (1.90 g) addiert und bei RT 20 min nachgerührt. Die Phasen wurden getrennt. Die wässrige Phase wurde mit Dichlormethan (2 x 40 ml) extrahiert. Die organische Phase wurde über $Na_2SO_4$ getrocknet und i. Vak. eingeengt. Der Rückstand wurde durch Flash-Chromatographie mit $CHCl_3$/MeOH (9:1, 1:1, MeOH) gereinigt.

Ausbeute: unpolares Diastereomer 1.12 g (26 %)
polares Diastereomer 0.911 g (21 %)

Das soeben erhaltene unpolarere Diastereomer (991 mg, 2.78 mmol) wurde in heißem Ethanol gelöst und mit Citronensäure (529 mg, 2.78 mmol) gelöst in Ethanol (5 ml) versetzt. Der ausgefallene Niederschlag wurde abgesaugt und i. Vak. getrocknet.
Ausbeute: 567 mg (38 %)
Schmelzpunkt: 240-241 °C
$^1$H-NMR (DMSO-$d_6$): 0.92 (3 H, t); 1.29 (4 H, m); 1.46 (2 H, m); 1.75 (4 H, t); 1.85 (2 H, t); 2.10 (2 H, m); 2.54-2.69 (10 H, m); 3.87 (2 H; t); 6.54 (1 H, d); 6.68 (1 H, s); 7.16 (1 H, d); 8.51 (1 H, s, OH); 10.53 (1 H, s).
$^{13}$C-NMR (DMSO-$d_6$): 13.91; 22.20; 23.05; 25.90; 26.29; 29.29; 30.65; 37.20; 44.44; 59.06; 60.52; 71.28; 72.08; 101.80; 104.34; 110.52; 111.58; 127.02; 130.11; 139.56; 150.27; 172.05; 177.47.

**Beispiel AA-49**

**6'-Hydroxy-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat (1:1) , Polareres Diastereomer**

Das unter Beispiel AA-48 erhaltene polarere Diastereomer (900 mg, 2.52 mmol) wurde in heißem Ethanol/ Dioxan (5 ml, 30 ml) gelöst (schlecht löslich). Anschließend wurde Citronensäure (480 mg, 2.52 mmol) in heißem Ethanol (5 ml) gelöst und zugegeben. Der Ansatz wurde auf RT abgekühlt, dabei ist wenig Niederschlag ausgefallen, deshalb wurde Ether addiert. Der ausgefallene Niederschlag wurde abgesaugt und i Vak. getrocknet.
Ausbeute: 874 mg (63 %)
Schmelzpunkt: 160-170 °C
$^1$H-NMR (DMSO-$d_6$): 0.97 (3 H, t); 1.43 (4 H, m); 1.65 (2 H, m); 1.92 (9 H, m); 2.51-2.67 (10 H, m); 3.88 (2 H; t); 6.58 (1 H, d); 6.70 (1 H, s); 7.12 (1 H, d); 8.56 (1 H, s, OH); 10.63 (1 H, s).
$^{13}$C-NMR (DMSO-$d_6$): 14.00; 22.09; 22.68; 24.48; 24.74; 28.32; 30.91; 37.46; 44.27; 59.13; 64.84; 70.64; 71.1608; 101.96; 104.69; 110.80; 111:17; 127.03; 129.96; 138.62; 150.36; 171.21; 176.86.

**REFERENZ-Beispiel AA-50**

**6'-Fluor-4',9'-dihydro-N,N-dimethyl-4-Cyclopentylmethyl-spiro[cyclohexan 1,1'(3'H)-pyrano[3,4-blindol]-4-amin, 2-hydroxy-1,2,3-propantricarboxylat (2:1) Eines von 2 möglichen Diastereomeren**

Das Keton E-17 (223 mg, 1.0 mmol) und 5-Fluor-tryptophol (179 mg, 1.0 mmol) wurden in abs. Dichlormethan (10 ml) gelöst, unter Argon mit Methansulfonsäure (0.1 ml, 1.5 mmol) versetzt und bei RT 3 d gerührt. Nach Zugabe von 1 N NaOH (10 ml) und $CH_2Cl_2$ (20 ml) wurde noch 10 min nachgerührt, die Phasen getrennt, die wässrige Phase zweimal mit $CH_2Cl_2$ extrahiert, die vereinigten organischen Phasen mit Wasser gewaschen, getrocknet ($Na_2SO_4$) und i. Vak. eingeengt. Der verbliebene Rückstand wurde durch Flash-Chromatographie mit $CHCl_3$/MeOH (20:1) getrennt. Es wurden 388 mg Feststoff isoliert, der lt. NMR als Salz vorlag, er wurde in $CH_2Cl_2$ gelöst, mit 1 N NaOH-Lösung gewaschen, über $Na_2SO_4$ getrocknet und i. Vak. eingeengt.
Ausbeute: 310 mg (81 %), nur 1 Diastereomer entstanden
$^1$H-NMR (DMSO-$d_6$): 1.16 (2 H, m); 1.51-1.84 (14 H, m); 2.05 (2 H, m); 2.45 (5 H, m); 2.74 (6 H, s); 3.90 (2 H, m); 6.87 (1 H, t); 7.17 (1 H, m); 7.26 (1 H, m); 8.44 (1 H, bs); 11.5 (1 H, bs). Salz?
Das soeben erhaltene Amin (310 mg, 0.81 mmol) wurde in heißem Ethanol (10 ml) gelöst und mit einer Lösung von Citronensäure (155 mg, 0.81 mmol) in heißem Ethanol (5 ml) versetzt. Nach 2-stündigem Stehen im Kühlschrank wurde der entstandene Feststoff abgesaugt und i. Vak. getrocknet.
Ausbeute: 316 mg (81 %), Hemicitrat entstanden.
Schmelzpunkt: 222-223 °C
$^1$H-NMR (DMSO-$d_6$): 1.11 (2 H, m); 1.48-1.98 (15 H, m); 2.15 (2 H, m); 2.58 (6 H, s); 2.65 (11 H, m); 3.89 (2 H, m); 6.83 (1 H, m); 7.15 (1 H, m); 7.37 (1 H, m); 10. (1 H, bs); 11.01 (1 H, s), Hemicitrat.
$^{13}$C-NMR (DMSO-$d_6$): 20.1; 24.6; 26.3; 29.2; 34.4; 35.6; 36.9; 44.2; 59.1; 61.6; 71.2; 72.5; 102.5; 105.6;108.2; 112.2; 126.5; 132.3; 141.1; 155.6; 157.9; 172.1; 177.3.

**Beispiel AA-51**

**2',3',4',9'-Tetrahydro-N,N-dimethyl-4-butyl-2'-(2-phenylethencarbonyl)-spiro[cyclohexan-1,1'(1'H)-pyrido [3,4-b]indol]-4-amin, 2-hydroxy-1,2,3-propantricarboxylat (2:1) Unpolareres Diastereomer**

Zimtsäurechlorid (441 mg, 2,65 mmol) wurde unter Argon in abs. Tetrahydrofuran (30 ml) gelöst und bei Raumtemperatur mit der freien Base, des unter Beispiel AA-9 hergestellten unpolareren Spiroamins (300 mg, 0,88 mmol), gelöst in abs. Tetrahydrofuran (15 ml), innerhalb von 20 min versetzt. Dabei entstand eine kräftige Fällung. Nach einer Reaktionszeit von 1,5 h wurde die Reaktionsmischung mit Wasser (10 ml) verdünnt, unter Eiskühlung mit 1 N Natronlauge (10 ml) versetzt und dann 2 h gerührt. Tetrahydrofuran wurde im Vakuum entfernt. Dabei fiel ein Feststoff aus, der durch Filtration abgetrennt und mit Wasser (3 $\times$ 10 ml) gewaschen wurde. Das Rohprodukt (408 mg) wurde chromatographisch aufgetrennt [Kieselgel 60 (50 g); Ethylacetat (500 ml)]. Das unpolarere Amid wurde als farbloser Feststoff in einer Ausbeute von 76 % (314 mg) gewonnen.
Das soeben erhaltene unpolarere Amid (296 mg, 0,63 mmol) wurde bei 80 °C in Ethanol (14 ml) suspendiert und mit einer ethanolischen Lösung (3 ml) von Citronensäure (133 mg, 0,69 mmol) versetzt. Aus der klaren Lösung fiel beim Abkühlen auf Raumtemperatur ein Feststoff aus. Es wurde 16 h bei Raumtemperatur gerührt. Die Mischung wurde 2 h bei 5 °C aufbewahrt. Der farblose Feststoff wurde durch Filtration abgetrennt und mit Diethylether (3 $\times$ 3 ml) gewaschen.

Das unpolarere Citrat **AA-51** wurde so in einer Ausbeute von 85 % (302 mg) als Hemicitrat mit einem Schmelzpunkt von 154-157 °C erhalten.
$^{13}$C-NMR (101 MHz, DMSO-$D_6$) d ppm: (unpolareres Diastereoisomer) 13.9, 22.2, 23.0, 26.2, 27.5, 29.5, 30.6, 37.3, 42.4, 44.0, 59.0, 71.6, 105.9, 111.3, 117.5, 118.4, 120.6, 123.1, 126.2, 127.8, 128.7, 129.3, 135.1, 135.5, 139.9, 140.2, 169.4, 171.4, 176.6

**Beispiel AA-52**

**2',3',4',9'-tetrahydro-N,N-dimethyl-4-butyl-2'-(2-phenylethencarbonyl)-spiro[cyclohexan-1,1'(1'H)-pyrido[3,4-b] indol]-4-amin, 2-hydroxy-1,2,3-propantricarboxylat (1:1) Polareres Diastereomer**

Zimtsäurechlorid (C, 441 mg, 2,65 mmol) wurde unter Argon in abs. Tetrahydrofuran (30 ml) gelöst und bei Raumtemperatur mit der freien Base, des unter Beispiel AA-9 hergestellten polareren Spiroamins (300 mg, 0,88 mmol, gelöst in abs. Tetrahydrofuran (15 ml), innerhalb von 20 min versetzt. Dabei entstand eine leichte Fällung. Nach einer Reaktionszeit von 1,5 h wurde die Reaktionsmischung mit Wasser (10 ml) verdünnt, unter Eiskühlung mit 1N Natronlauge (10 ml) versetzt und 1 h gerührt. Tetrahydrofuran wurde im Vakuum entfernt. Dabei fiel ein Feststoff aus, der durch Filtration abgetrennt und mit Wasser (3 × 10 ml) gewaschen wurde. Das Rohprodukt (384 mg) wurde chromatographisch aufgetrennt [Kieselgel 60 (50 g); Ethylacetat/Methanol 1 : 4 (750 ml)]. Das polarere Amid wurde als beigefarbener Feststoff in einer Ausbeute von 43 % (177 mg) gewonnen.
$^{13}$C-NMR (101 MHz, $CDCl_3$) d ppm: (polareres Diastereoisomer) 14.0, 22.4, 23.5, 25.9, 27.3, 31.5, 31.6, 37.8, 43.3, 58.8, 106.7, 111.8, 117.6, 119.3, 121.6, 122.1, 126.6, 127.7, 128.8, 129.6, 135.0, 136.0, 138.8, 141.9, 170.9
Das soeben erhaltene polarere Amid (157 mg, 0,334 mmol) wurde in Ethanol (5 ml) gelöst und mit einer ethanolischen Lösung (2 ml) von Citronensäure (72 mg, 0,37 mmol) versetzt. Es wurde 16 h bei Raumtemperatur gerührt, wobei keine Fällung beobachtet wurde. Die Mischung wurde eingeengt, in Ethanol (2 ml) aufgenommen und langsam mit Diethylether (30 ml) versetzt. Nach 1,5 h wurde ein farbloser Feststoff durch Filtration abgetrennt und mit Diethylether (3 × 3 ml) gewaschen. Das polarere Citrat **AA-52** wurde so in einer Ausbeute von 73 % (161 mg) erhalten.

**Vergleichsuntersuchungen zur Löslichkeit:**

Zur Bestimmung der Löslichkeiten der Verbindungen wurde eine Versuchsreihe anhand einer Verdünnung einer Lösung von 20 mg/ml in DMSO mit einer wäßrigen Pufferlösung verwendet. Bei der Passage von Arzneimitteln durch den Verdauungstrakt werden die Arzneimittel unterschiedlichen pH-Werten ausgesetzt. Im Magen werden pH-Werte von 1-3 erwartet, und im Anschluß an die Magenpassage im Darm sind pH-Werte von 6-8 zu erwarten. Da Löslichkeiten pHabhängig sein können, wurden wäßrige Puffer bei verschiedenen pH-Werten verwendet (pH 1, 100 mM HCl; pH 2, 10 mM HCl; pH 4, 50 mM Zitronensäure, titriert mit 1 N NaOH; pH 6, 50 mM Natriumcitrat, titrated with 1 N HCl; pH 7, 50 mM Tris.HCl; pH 8, Tris.HCl), die bei Raumtemperatur die eingestellten pH-Werte in der endgültigen Lösung hielten.
Da DMSO bei steigender Konzentration die Bildung von metastabilen übersättigten wässrigen Lösungen begünstigt, wurden die Stammlösungen 1:100 in wäßrigem Puffer verdünnt. Die Lösungen wurden in geschlossenen Gefäßen mindestens 15 Stunden geschüttelt. Dabei wurden 10 $\mu$l DMSO Stammlösung in 990 $\mu$l wäßrigem Puffer verdünnt und in geeigneten Gefäßen (z. B. Eppendorf-Gefäße), sodass die Konzentration konstant bei 1 % v/v war. Anschließlich wurden die Lösungen abzentrifugiert und Proben des klaren Überstandes wurden in Probengefäße überführt, die zwei Equivalente 50% Acetonitril in 0,1 N HCl enthielten.
Die Kalibrierungslösungen wurden durch Verdünnung der DMSO-Stammlösungen in Methanol hergestellt (1: 100). Aus diesen Verdünnungen wurden weitere Verdünnungen in Methanol hergestellt (1:100, 1:200, 1:400 and 1:800). Proben wurden durch RP-HPLC mit UV-Detektion analysiert. Über Regressionsanalyse wurden lineare Kalibrationsgleichungen abgeleitet, generell mit Korrelationskoeffizienten von über 0,95. Mit Hilfe der experimentell ermittelten Kalibrationsleichungen wurden die Konzentrationen der Verbindungen in den Pufferlösungen ermittelt. Mit dem beschriebenen Experiment konnten maximale Konzentrationen von 200 $\mu$g/ml Substanz in Puffer ermittelt warden. Höhere Löslichkeiten konnten nicht quantifiziert werden.
Die Korrelation zwischen Löslichkeit und der Variation von $R^3$ wurde anhand der folgenden Verbindungen gezeigt.
$R^1$, $R^2$ = $CH_3$; $R^5$, $R^6$, $R^7$, $R^9$, $R^{10}$ = H, $R^8$ = F, X = O

| Beispiel | $R^3$ | Löslichkeit |
|---|---|---|
| AA-8 | 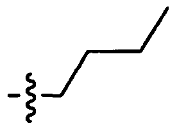 $R^8$ = OH | 200 µg/ml bei pH 1-9 |
| AA-23 | 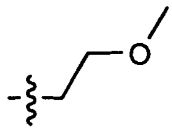 | Ca 180 µg/ml bei pH 1-7 |
| AA-5 | 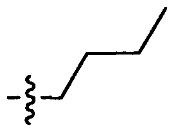 | 2,9 µg/ml (pH 1); 17,7 µg/ml (pH 2); 5,9 µg/ml (pH 4); 8,7 µg/ml (pH 6); 3,3 µg/ml (pH 7); 0,2 µg/ml (pH 8) |
| AA-2 | 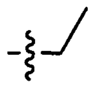 | Ca 30 µg/ml bei pH 1-7 |
| AA-22 | 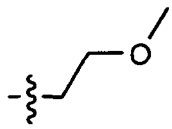 ; $R^8$ = H | 197,6 µg/ml (pH 1); 154,2 µg/ml (pH 2); 154,0 µg/ml (pH 4); 176,7 µg/ml (pH 6); 153,5 µg/ml (pH 7); 46,4 µg/ml (pH 8); 2,1 µg/ml (pH 9) |
| V-1 | 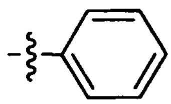 | 1,9 µg/ml (pH 2); 0,4 µg/ml (pH 6); 0,8 µg/ml (pH 7), 0,9 µg/ml (pH 8); 0,04 µg/ml (pH 9) |
| V-2 | 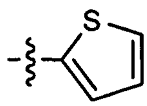 | > 5 µg/ml |
| V-3 | 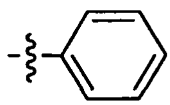 ; $R^1$ = H | 2,7 µg/ml (pH 1), 2,8 µg/ml (pH 2); 1,7 µg/ml (pH 4), 1,5 µg/ml (pH 6), 0,4 µg/ml (pH 7); 0,4 µg/ml (pH 8); 0,4 µg/ml (pH 9) |
| V-4 | 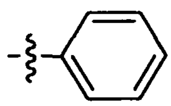 ; $R^8$ = OH | 1,9 µg/ml (pH 1); 6,4 µg/ml (pH 4); 3,8 µg/ml (pH 6); 1,7 µg/ml (pH 7); 5,1 µg/ml (pH 8); 0,5 µg/ml (pH 9) |
| V-5 | 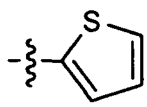 $R^8$ = OH | 1,9 µg/ml (pH 1); 3,1 µg/ml (pH 4); 3.2 µg/ml (pH 7); 2,0 µg/ml (pH 8); 0,6 µg/ml (pH 9) |

Die erfindungsgemäßen Verbindungen weisen eine außerordentlich hohe Affinität zum ORL1 bzw zum µ-Opioid-Rezeptor auf. Die Affinität liegt in der gleichen Größenordnung wie die beiden Vergleichsverbindungen. Sie besitzen jedoch eine höhere Löslichkeit.

Zwischen pH 1 und pH 8 wurde eine geringe pH-Abhängigkeit der Löslichkeit beobachtet. Unterhalb von pH 8 nimmt die Löslichkeit der Verbindungen ab. Untersuchungen zur Wirksamkeit der erfindungsgemäßen Verbindungen: Die in den folgenden Assays und Modellen erhobenen Daten sind in Tabelle 1 zusammengefaßt.

**Messung der ORL1-Bindung**

Die Cyclohexan-Derivate der allgemeinen Formel I wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 µg Membranprotein

je 200 μl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird in Tabelle 1 als nanomolarer $K_i$-Wert in oder % Inhibition bei c=1 μM angegeben.

**<u>Messung der μ-Bindung</u>**

Die Rezeptoraffinität zum humanen μ-Opiatrezeptor wurde in einem homogenen Ansatz in Mikrotiterplatten bestimmt. Hierzu wurden Verdünnungsreihen des jeweils zu prüfenden substituierten spirocyclischen Cyclohexan-Derivates mit einer Rezeptormembranpräparation (15-40 μg Protein pro 250 μl Inkubationsansatz) von CHO-K1-Zellen, welche den humanen μ-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 μl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen μ-Opiatrezeptor bei einer Konzentration der Prüfsubstanzen von 1 μmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

**<u>Analgesieprüfung im Tail-Flick-Test an der Maus</u>**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

In zwei Fällen wurde der Test in analoger Weise an der Ratte durchgeführt.

**Tabelle 1:**

| Beispiel | Struktur ohne Salz Salzform s. Beispielbeschreibung | Diastereomer | Ki (ORL1) Mittel [μM] *Fest oder %Hemmung [1 μM]* | Ki (μ) Mittel [μM] *Fest oder %Hemmung [1 μM]* | Tail-flick Maus i. v. % Hemmung [10 μg/kg) |
|---|---|---|---|---|---|
| AA-1 | 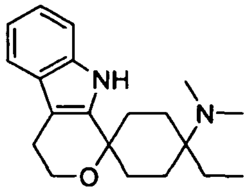 GRTE6490 | Eines von 2 möglichen Diastereomeren | 0.0031 | 0.0005 | 90% (100 μg/kg) |
| AA-2 | GRTE6559 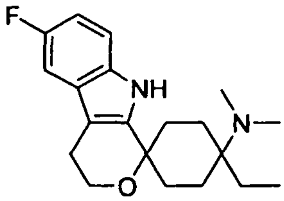 | Unpolareres Diastereomer | 0.0120 | 0.0003 | n. d. |
| AA-3 | 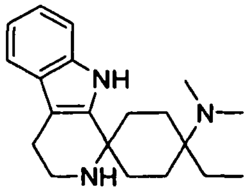 GRTE6562 | Eines von 2 möglichen Diastereomeren | 0.0012 | 0.0003 | n. d. |
| AA-4 | GRTE6282 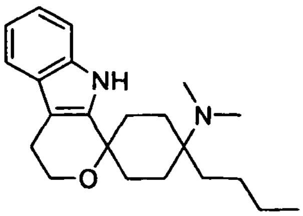 | Eines von 2 möglichen Diastereomeren | 0.0002 | 0.0005 | 75% |
| AA-5 | GRTE6283 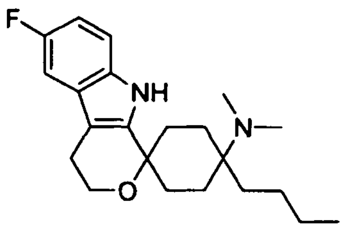 | Eines von 2 möglichen Diastereomeren | 0.0016 | 0.0009 | 60% |
| AA-6 | GRTE6859 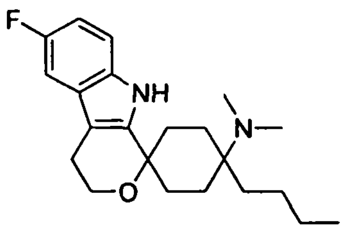 | Eines von 2 möglichen Diastereomeren | 0.0009 | 0.0007 | Ratte $ED_{50}$ = 11 μg/kg |
| AA-7 | GRTE7131 | | | | |

(fortgesetzt)

| Beispiel | Struktur ohne Salz Salzform s. Beispielbeschreibung | Diastereomer | Ki (ORL1) Mittel [µM] *Fest oder %Hemmung [1 µM]* | Ki (µ) Mittel [µM] *Fest oder %Hemmung [1 µM]* | Tail-flick Maus i. v. % Hemmung [10 µg/kg) |
|---|---|---|---|---|---|
| | 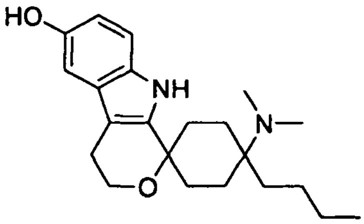 | Eines von 2 möglichen Diastereomeren | 0.0025 | 0.0002 | n. d. |
| AA-8 | GRTE7235 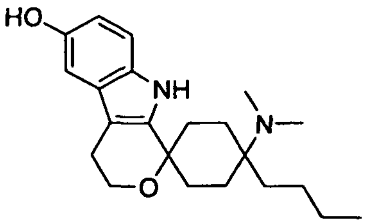 | Eines von 2 möglichen Diastereomeren | 0.2600 | 0.0250 | Ratte $ED_{50}$ = 385 µg/kg |
| AA-9 | GRTE6359 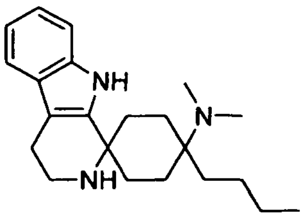 | Unpolareres Diastereomer | 0.0001 | 0.0003 | n. d. |
| AA-10 | GRTE6762 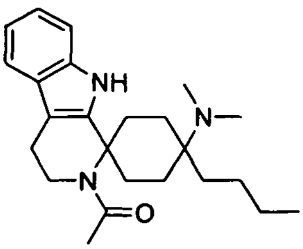 | Unpolareres Diastereomer | 0.0002 | 0.0003 | 75% |
| AA-11 | GRTE6874 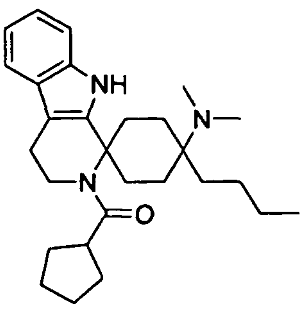 | Unpolareres Diastereomer | 0.0006 | 0.0012 | n. d. |

(fortgesetzt)

| Beispiel | Struktur ohne Salz Salzform s. Beispielbeschreibung | Diastereomer | Ki (ORL1) Mittel [μM] *Fest oder %Hemmung [1 μM]* | Ki (μ) Mittel [μM] *Fest oder %Hemmung [1 μM]* | Tail-flick Maus i. v. % Hemmung [10 μg/kg) |
|---|---|---|---|---|---|
| AA-12 | GRTE6785 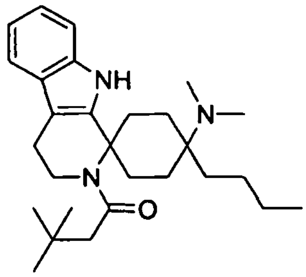 | Unpolareres Diastereomer | 0.0015 | 0.0009 | n. d. |
| AA-13 | GRTE6853 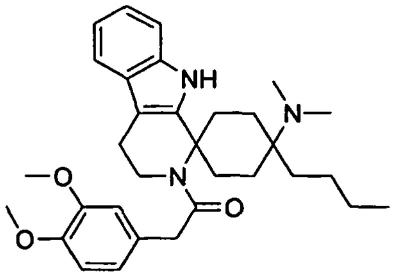 | Unpolareres Diastereomer | 0.0006 | 0.0006 | n. d. |
| AA-14 | GRTE6675 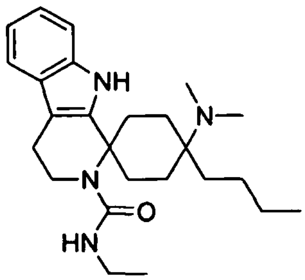 | Unpolareres Diastereomer | 0.0002 | 0.0003 | n. d. |
| AA-15 | GRTE6783 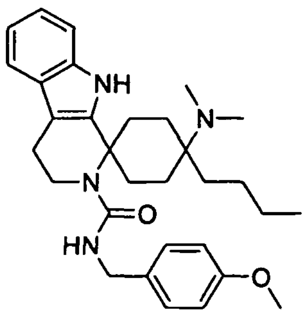 | polareres Diastereomer | 0.0002 | 0.0004 | n. d. |
| AA-16 | GRTE6786 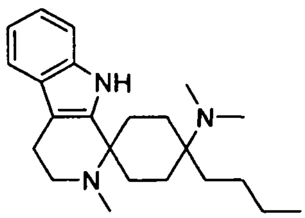 | Unpolareres Diastereomer | 0.0002 | 0.0004 | n. d. |

(fortgesetzt)

| Beispiel | Struktur ohne Salz Salzform s. Beispielbeschreibung | Diastereomer | Ki (ORL1) Mittel [µM] *Fest oder %Hemmung [1 µM]* | Ki (µ) Mittel [µM] *Fest oder %Hemmung [1 µM]* | Tail-flick Maus i. v. % Hemmung [10 µg/kg) |
|---|---|---|---|---|---|
| AA-17 | GRTE7243 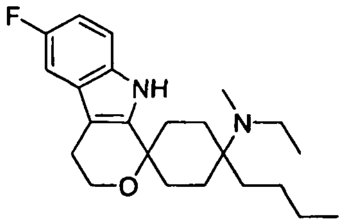 | Eines von 2 möglichen Diastereomeren | 0.0066 | 0.0018 | n. d. |
| AA-18 | GRTE7175 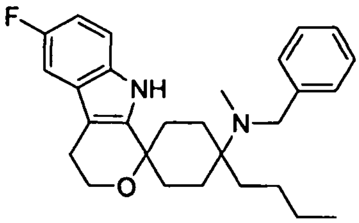 | | - | 0.3733 | n. d. |
| AA-19 | GRTE7055 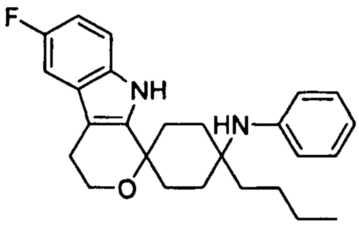 | Eines von 2 möglichen Diastereomeren | - | 1.9400 | n. d. |
| AA-20 | GRTE7123 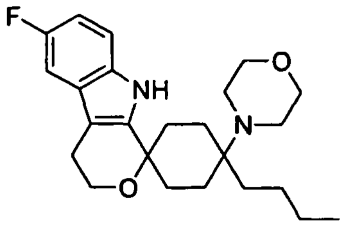 | Unpolareres Diastereomer | - | 36% | n. d. |
| AA-21 | GRTE7172 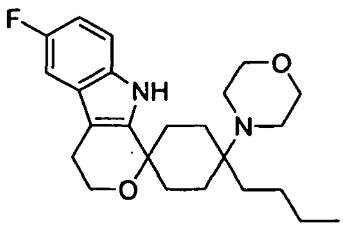 | | | 36% | n. d. |

(fortgesetzt)

| Beispiel | Struktur ohne Salz Salzform s. Beispielbeschreibung | Diastereomer | Ki (ORL1) Mittel [μM] *Fest oder %Hemmung [1 μM]* | Ki (μ) Mittel [μM] *Fest oder %Hemmung [1 μM]* | Tail-flick Maus i. v. % Hemmung [10 μg/kg) |
|---|---|---|---|---|---|
| AA-22 | GRTE6697 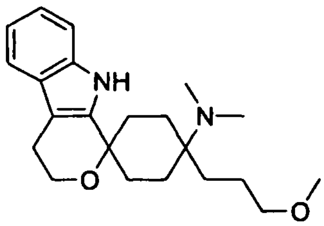 | Eines von 2 möglichen Diastereomeren | 0.0006 | 0.0002 | n. d. |
| AA-23 | GRTE6701 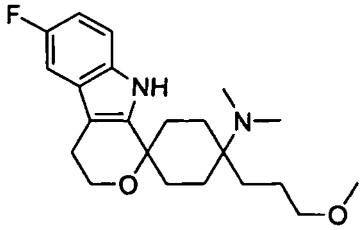 | Unpolareres Diastereomer | 0.0050 | 0.0003 | 100% (46,4 μg/kg) |
| AA-24 | GRTE6699 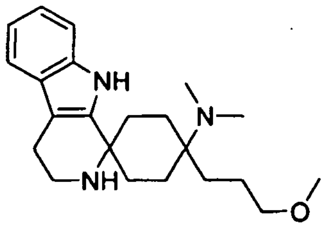 | Unpolareres Diastereomer | 0.0003 | 0.0003 | n. d. |
| AA-25 | GRTE6790 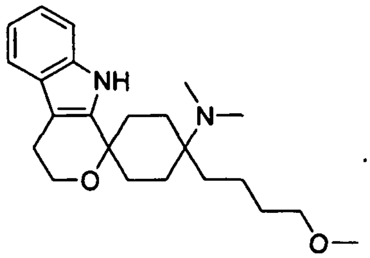 | Eines von 2 möglichen Diastereomeren | 0.0032 | 0.0002 | n. d. |
| AA-26 | GRTE6793 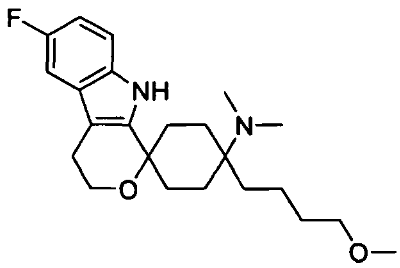 | polareres Diastereomer | 0.0243 | 0.0004 | n. d. |

(fortgesetzt)

| Beispiel | Struktur ohne Salz Salzform s. Beispielbeschreibung | Diastereomer | Ki (ORL1) Mittel [µM] *Fest oder %Hemmung [1 µM]* | Ki (µ) Mittel [µM] *Fest oder %Hemmung [1 µM]* | Tail-flick Maus i. v. % Hemmung [10 µg/kg) |
|---|---|---|---|---|---|
| AA-27 | GRTE6791 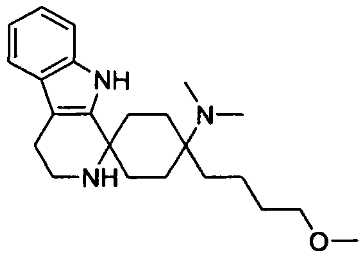 | Unpolareres Diastereomer | 0.0014 | 0.0004 | n. d. |
| AA-28 | GRTE6343 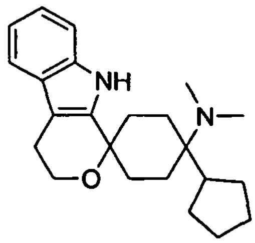 | Eines von 2 möglichen Diastereomeren | 0.0001 | 0.0006 | 90% |
| AA-29 | GRTE6564 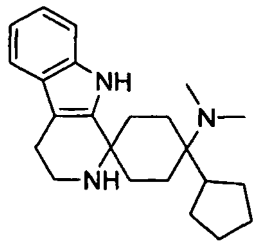 | Eines von 2 möglichen Diastereomeren | 0.0001 | 0.0002 | n. d. |
| AA-30 | GRTE6362 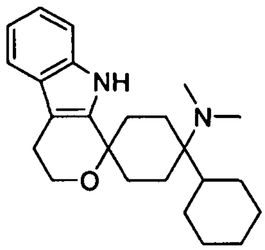 | Eines von 2 möglichen Diastereomeren | 0.0004 | 0.0007 | n. d. |
| AA-31 | GRTE6421 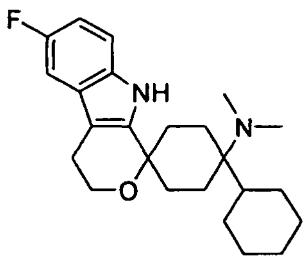 | Eines von 2 möglichen Diastereomeren | 0.0012 | 0.0015 | n. d. |

(fortgesetzt)

| Beispiel | Struktur ohne Salz Salzform s. Beispielbeschreibung | Diastereomer | Ki (ORL1) Mittel [μM] *Fest oder %Hemmung [1 μM]* | Ki (μ) Mittel [μM] *Fest oder %Hemmung [1 μM]* | Tail-flick Maus i. v. % Hemmung [10 μg/kg) |
|---|---|---|---|---|---|
| AA-32 | GRTE6363 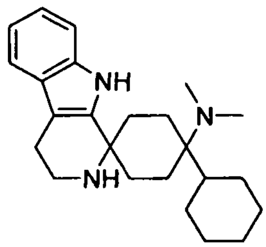 | Eines von 2 möglichen Diastereomeren | 0.0001 | 0.0002 | n. d. |
| AA-33 | GRTE6360 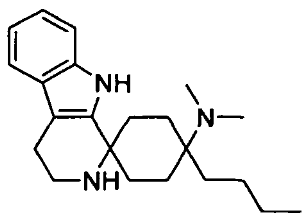 | Polareres Diastereomer | 0.2500 | 0.3400 | n. d. |
| AA-34 | GRTE6558 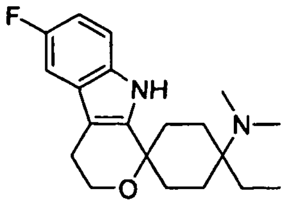 | Polareres Diastereomer | | 1.0767 | n. d. |
| AA-35 | GRTE6698 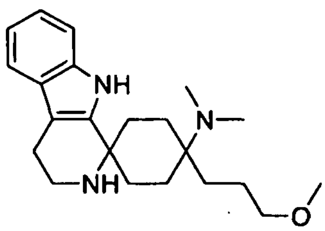 | polareres Diastereomer | | 26% | n. d. |
| AA-36 | GRTE6700 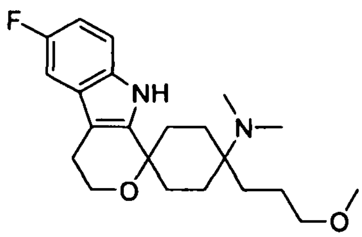 | polareres Diastereomer | 1.7500 | 0.0633 | n. d. |

(fortgesetzt)

| Beispiel | Struktur ohne Salz Salzform s. Beispielbeschreibung | Diastereomer | Ki (ORL1) Mittel [µM] *Fest oder %Hemmung [1 µM]* | Ki (µ) Mittel [µM] *Fest oder %Hemmung [1 µM]* | Tail-flick Maus i. v. % Hemmung [10 µg/kg) |
|---|---|---|---|---|---|
| AA-37 | GRTE6759 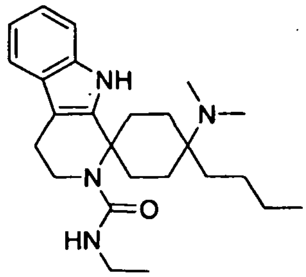 | polareres Diastereomer | | 0.2750 | n. d. |
| AA-38 | GRTE6799 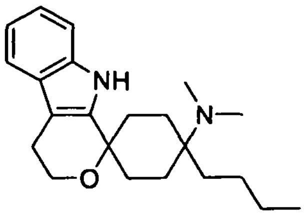 | polareres Diastereomer | 1.2600 | 0.3650 | n. d. |
| AA-39 | GRTE6792 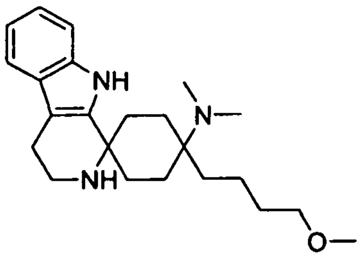 | polareres Diastereomer | 3.7900 | 1.0433 | n. d. |
| AA-40 | GRTE7053 **GRTE7053** 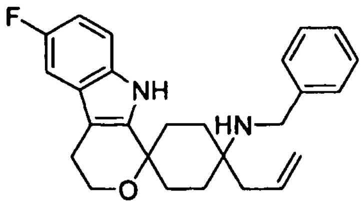 | Eines von 2 möglichen Diastereomeren | | 0.3167 | n. d. |
| AA-41 | GRTE7176 **GRTE7176** 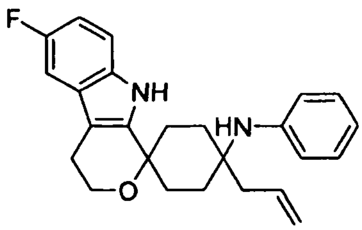 | | | 3.8033 | n. d. |

(fortgesetzt)

| Beispiel | Struktur ohne Salz Salzform s. Beispielbeschreibung | Diastereomer | Ki (ORL1) Mittel [μM] *Fest oder %Hemmung [1 μM]* | Ki (μ) Mittel [μM] *Fest oder %Hemmung [1 μM]* | Tail-flick Maus i. v. % Hemmung [10 μg/kg) |
|---|---|---|---|---|---|
| AA-42 | GRTE6283 **GRTE7236** 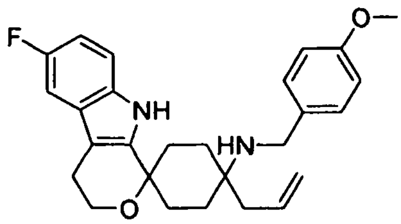 | Eines von 2 möglichen Diastereomeren | | 53% | n. d. |
| AA-43 | GRTE7236 **GRTE7336** 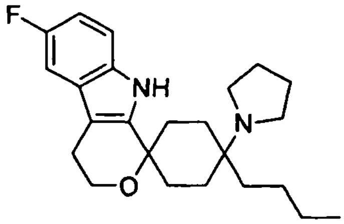 | Eines von 2 möglichen Diastereomeren | 47% | 94% | n. d. |
| AA-44 | GRTE7337 **GRTE7337** 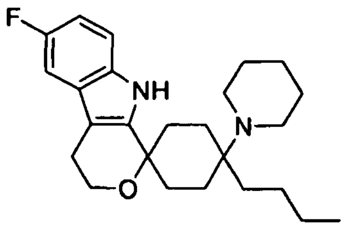 | Unpolareres Diastereomer | | 42% | n. d. |
| AA-45 | GRTE7338 **GRTE7338** 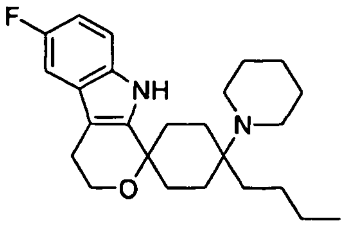 | Polareres Diastereomer | | 44% | n. d. |
| AA-46 | GRTE7342 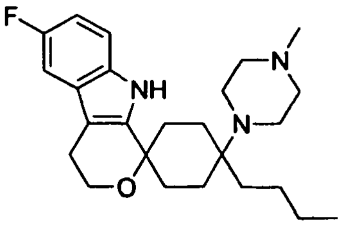 | Eines von 2 möglichen Diastereomeren | | 34% | n. d. |

(fortgesetzt)

| Beispiel | Struktur ohne Salz Salzform s. Beispielbeschreibung | Diastereomer | Ki (ORL1) Mittel [µM] *Fest oder %Hemmung [1 µM]* | Ki (µ) Mittel [µM] *Fest oder %Hemmung [1 µM]* | Tail-flick Maus i. v. % Hemmung [10 µg/kg) |
|---|---|---|---|---|---|
| AA-47 | 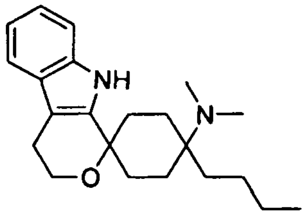 | Unpolareres Diastereomer | 0.0003 | 0.0006 | n. d. |
| AA-48 | 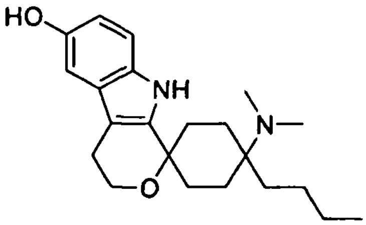 | Unpolareres Diastereomer | 98% | 0.0005 | n. d. |
| AA-49 | 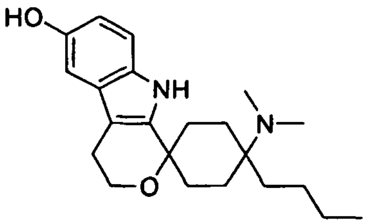 | Polareres Diastereomer | 27% | 1.3 | n. d. |
| AA-50 | 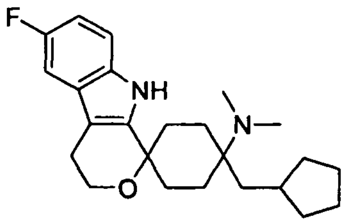 | Eines von 2 möglichen Diastereomeren | 0.0052 | 0.0027 | n. d. |
| AA-51 | 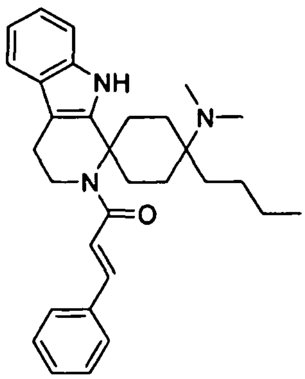 | Unpolareres Diastereomer | 0.0043 | 0.0030 | n. d. |

(fortgesetzt)

| Beispiel | Struktur ohne Salz Salzform s. Beispielbeschreibung | Diastereomer | Ki (ORL1) Mittel [µM] *Fest oder %Hemmung [1 µM]* | Ki (µ) Mittel [µM] *Fest oder %Hemmung [1 µM]* | Tail-flick Maus i. v. % Hemmung [10 µg/kg) |
|---|---|---|---|---|---|
| AA-52 | 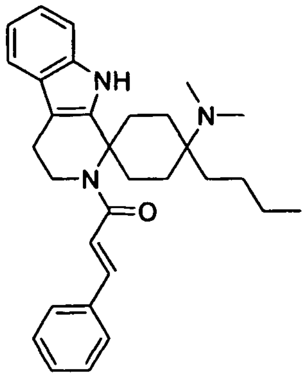 | Polareres Diastereomer | 0.6600 | 0.1200 | n. d. |

**Parenterale Lösung eines erfindungsgemäßen spirocyclischen Cyclohexan-Derivats**

38 g eines der erfindungsgemäßen spirocyclischen Cyclohexan-Derivate, hier Beispiel 3, wird in 1 l Wasser für Injektionszwecke bei Raumtemperatur gelöst und anschließend durch Zugabe von wasserfreier Glukose für Injektionszwecke auf isotone Bedingungen eingestellt.

**Patentansprüche**

**1.** Spirocyclische Cyclohexan-Derivate der allgemeinen Formel I,

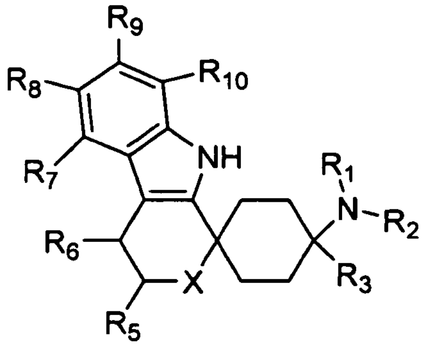

I,

worin

$R^1$ und $R^2$, unabhängig voneinander für H; $C_{1-5}$-Alkyl jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl, unsubstituiert oder einfach oder mehrfach substituiert; oder über $C_{1-3}$-Alkyl gebundenes Aryl, $C_{3-8}$-Cycloalkyl oder Heteroaryl, jeweils einfach oder mehrfach substituiert oder unsubstituiert, stehen;
oder die Reste $R^1$ und $R^2$ zusammen für $CH_2CH_2OCH_2CH_2$, $CH_2CH_2NR^{11}CH_2CH_2$ oder $(CH_2)_{3-6}$ stehen, wobei $R^{11}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert;

oder über $C_{1-3}$-Alkyl gebundenes Aryl, $C_{3-8}$-Cycloalkyl oder Heteroaryl, jeweils einfach oder mehrfach substituiert oder unsubstituiert, bedeutet;
$R^3$ für $C_{1-8}$-Alkyl, jeweils gesättigt oder ungesättigt, verzweigt oder unverzweigt, einfach oder mehrfach substituiert oder unsubstituiert;
$R^5$ für =O; H; $C_{1-5}$-Alkyl, gesättigt oder ungesättigt, verzweigt oder unverzweigt, unsubstituiert oder einfach oder mehrfach substituiert; $COOR^{13}$, $CONR^{13}$, $OR^{13}$; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;
$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; $C_{3-8}$-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, steht;
oder $R^5$ und $R^6$ gemeinsam $(CH_2)_n$ mit n = 2, 3, 4, 5 oder 6 bedeuten, wobei einzelne Wasserstoffatome auch durch F, Cl, Br, I, $NO_2$, $CF_3$, $OR^{13}$, CN oder $C_{1-5}$-Alkyl ersetzt sein können;
$R^7$, $R^8$, $R^9$ und $R^{10}$ unabhängig voneinander für
H, F, Cl, Br, I, $NO_2$, $CF_3$, $OR^{13}$, $SR^{13}$, $SO_2R^{13}$, $NHC(=O)NR^{14}R^{15}$, $SO_2NR^{14}R^{15}$, $SO_2OR^{13}$, CN, $COOR^{13}$, $NR^{14}R^{15}$; $C_{1-5}$-Alkyl, $C_{3-8}$-Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, stehen;
wobei $R^{13}$ H; $C_{1-5}$-Alkyl jeweils gesättigt oder ungesättigt, verzweigt oder unverzweigt, unsubstituiert oder einfach oder mehrfach substituiert; $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeutet;
$R^{14}$ und $R^{15}$ unabhängig voneinander H; $C_{1-5}$-Alkyl, jeweils gesättigt oder ungesättigt, verzweigt oder unverzweigt, unsubstituiert oder einfach oder mehrfach substituiert; oder $C_{3-8}$-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl gebundenes Aryl, $C_{3-8}$-Cycloalkyl oder Heteroaryl, unsubstituiert oder einfach oder mehrfach substituiert, bedeuten;
oder $R^{14}$ und $R^{15}$ zusammen $CH_2CH_2OCH_2CH_2$, $CH_2CH_2NR^{16}CH_2CH_2$ oder $(CH_2)_{3-6}$ bilden,

wobei $R^{16}$ H; $C_{1-5}$-Alkyl gesättigt oder ungesättigt, verzweigt oder unverzweigt, unsubstituiert oder einfach oder mehrfach substituiert, bedeutet;

X für O, S, SO, $SO_2$ oder $NR^{17}$ steht;
$R^{17}$ für H; $C_{1-5}$-Alkyl, gesättigt oder ungesättigt, verzweigt oder unverzweigt; $COR^{12}$ oder $SO_2R^{12}$,
wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl gebundenes Aryl, $C_{3-13}$-Cycloalkyl oder Heteroaryl, jeweils einfach oder mehrfach substituiert oder unsubstituiert; $OR^{13}$; $NR^{14}R^{15}$ bedeutet;
worin "Alkyl substituiert" oder "Cycloalkyl substituiert" Alkyl oder Cycloalkyl substituiert mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ bedeutet und
"Aryl substituiert" oder "Heteroaryl substituiert" Aryl oder Heteroaryl substituiert mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ bedeutet,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen,
mit Ausnahme der Verbindung
2',3',4',9'-Tetrahydro-N,N-dimethyl-4-butyl-spiro[cyclohexan-1,1'(1'H)-pyrido[3,4-b]indol]-4-amin.

**2.** Spirocyclische Cyclohexan-Derivate gemäß Anspruch 1, worin
$R^1$ und $R^2$ unabhängig voneinander für H, $C_{1-5}$-Alkyl, verzweigt oder unverzweigt, gesättigt oder ungesättigt, unsubstituiert oder einfach oder mehrfach substituiert, oder Phenyl oder Benzyl, unsubstituiert oder einfach oder mehrfach substituiert, stehen,
oder gemeinsam für einen Ring stehen und $(CH_2)_{3-6}$ bedeuten.

**3.** Spirocyclische Cyclohexan-Derivate gemäß Anspruch 1, worin $R^3$ für Ethyl, n-Propyl, 2-Propyl, Allyl, n-Butyl, iso-Butyl, sec.-Butyl, tert.-Butyl, n-Pentyl, iso-Pentyl, neo-Pentyl oder n-Hexyl steht, jeweils unsubstituiert oder einfach oder mehrfach substituiert mit OH, $OCH_3$ oder $OC_2H_5$.

**4.** Spirocyclische Cyclohexan-Derivate gemäß Anspruch 1, worin der Rest $R^5$ für H, $CH_3$, COOH, $COOCH_3$, $CH_2OPhenyl$, wobei der Phenylrest mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ substituiert sein kann, oder $CH_2OH$ steht.

**5.** Spirocyclische Cyclohexan-Derivate gemäß Anspruch 1, worin $R^6$ H; Methyl, Ethyl, $CF_3$, Benzyl oder Phenyl bedeuten kann, wobei der Benzyl- oder Phenylrest mit F, Cl, Br, I, CN, $CH_3$, $C_2H_5$, $NH_2$, $NO_2$, SH, $CF_3$, OH, $OCH_3$, $OC_2H_5$ oder $N(CH_3)_2$ substituiert sein kann.

**6.** Spirocyclische Cyclohexan-Derivate gemäß Anspruch 1, worin $R^7$ $R^8$ $R^9$ und $R^{10}$ unabhängig voneinander H; $C_{1-5}$-Alkyl, verzweigt oder unverzweigt, unsubstituiert oder ein- oder mehrfach substituiert; F, Cl, Br, I, $CF_3$, OH, $OCH_3$, $NH_2$, COOH, $COOCH_3$, $NHCH_3$ Thienyl, Pyrimidinyl, Pyridyl, $N(CH_3)_2$ oder $NO_2$ bedeuten.

**7.** Spirocyclische Cyclohexan-Derivate gemäß einem der Ansprüche 1 bis 6, worin X für O steht.

**8.** Spirocyclische Cyclohexan-Derivate gemäß einem der Ansprüche 1 bis 6, worin X für $NR^{17}$ steht.

**9.** Spirocyclische Cyclohexan-Derivate gemäß Anspruch 1 aus der Gruppe:

4’,9’-Dihydro-N,N-dimethyl-4-ethyl-spiro[cyclohexan-1,1’(3’H)-pyrano[3,4-b]indol]-4-amin, 2-hydroxy-1,2,3-propantricarboxylat
6’-Fluor-4’,9’-dihydro-N,N-dimethyl-4-ethyl-spiro[cyclohexan-1,1’(3’H)-pyrano[3,4-b]indol]-4-amin, 2-hydroxy-1,2,3-propantricarboxylat
2’,3’,4’,9’-Tetrahydro-N,N-dimethyl-4-ethyl-spiro[cyclohexan-1,1’(1’H)-pyrido[3,4-b]indol]-4-amin, 2-hydroxy-1,2,3-propantricarboxylat
4’,9’-Dihydro-N, N-dimethyl-4-butyl-spiro[cyclohexan-1,1’(3’H)-pyrano[3,4-b]indol]-4-amin, 2-hydroxy-1,2,3-propantricarboxylat
6’-Fluor-4’,9’-dihydro-N,N-dimethyl-4-butyl-spiro[cyclohexan-1,1’(3’H)-pyrano[3,4-b]indol]-4-amin, 2-hydroxy-1,2,3-propantricarboxylat
6’-Fluor-4’,9’-dihydro-N,N-dimethyl-4-butyl-spiro[cyclohexan-1,1’(3’H)-pyrano[3,4-b]indol]-4-amin, 2-hydroxy-1,2,3-propantricarboxylat
6’-Hydroxy-4’,9’-dihydro-N,N-dimethyl-4-butyl-spiro[cyclohexan-1,1’(3’H)-pyrano[3,4-b]indol]-4-amin, 2,2,2-trifluoracetat
6’-Hydroxy-4’,9’-dihydro-N,N-dimethyl-4-butyl-spiro[cyclohexan-1,1’(3’H)-pyrano[3,4-b]indol]-4-amin, 2-hydroxy-1,2,3-propantricarboxylat
2’,3’,4’,9’-Tetrahydro-N,N-dimethyl-4-butyl-spiro[cyclohexan-1,1’(1’H)-pyrido[3,4-b]indol]-4-amin, 2-hydroxy-1,2,3-propantricarboxylat
2’,3’,4’,9’-Tetrahydro-N,N-dimethyl-4-butyl-2’-methylcarbonyl-spiro[cyclohexan-1,1’(1’H)-pyrido[3,4-b]indol]-4-amin, 2-hydroxy-1,2,3-propantricarboxylat
2’,3’,4’,9’-Tetrahydro-N, N-dimethyl-4-butyl-2’-cyclopentylcarbonyl-spiro[cyclohexan-1,1’(1’H)-pyrido[3,4-b]indol]-4-amin, 2-hydroxy-1,2,3-propantricarboxylat
2’,3’,4’,9’-Tetrahydro-N,N-dimethyl-4-butyl-2’-(2,2)-dimethylpropancarbonyl-spiro[cyclohexan-1,1’(1’H)-pyrido [3,4-b]indol]-4-amin, 2-hydroxy-1,2,3-propantricarboxylat
2’,3’,4’,9’-Tetrahydro-N,N-dimethyl-4-butyl-2’-(3,4-dimethoxybenzylcarbonyl)-spiro[cyclohexan-1,1’(1’H)-pyrido[3,4-b]indol]-4-amin, 2-hydroxy-1,2,3-propantricarboxylat
2’,3’,4’,9’-Tetrahydro-N,N-dimethyl-4-butyl-2’-ethylaminocarbonyl-spiro[cyclohexan-1,1’(1’H)-pyrido[3,4-b]indol]-4-amin, 2-hydroxy-1,2,3-propantricarboxylat
2’,3’,4’,9’-Tetrahydro-N,N-dimethyl-4-butyl-2’-4-methoxybenzylaminocarbonyl-spiro[cyclohexan-1,1’(1’H)-pyrido[3,4-b]indol]-4-amin
2’,3’,4’,9’-Tetrahydro-N,N-dimethyl-4-butyl-2’-methyl-spiro[cyclohexan-1,1’(1’H)-pyrido[3,4-b]indol]-4-amin, 2-hydroxy-1,2,3-propantricarboxylat
6’-Fluor-4’,9’-dihydro-N-ethyl-N-methyl-4-butyl-spiro[cyclohexan-1,1’(3’H)-pyrano[3,4-b]indol]-4-amin, 2-hydroxy-1,2,3-propantricarboxylat
6’-Fluor-4’,9’-dihydro-N-benzyl-N-methyl-4-butyl-spiro[cyclohexan-1,1’(3’H)-pyrano[3,4-b]indol]-4-amin
6’-Fluor-4’,9’-dihydro-N-phenyl-4-butyl-spiro[cyclohexan-1,1’(3’H)-pyrano[3,4-b]indol]-4-amin

4-Butyl-6'-fluor-4-(N-morpholino)-1',3',4',9'-tetrahydrospiro[cyclohexan-1,1'-pyrano[3,4-b]indol]
4-Butyl-6'-fluor-4-(N-morpholino)-1',3',4',9'-tetrahydrospiro[cyclohexan-1,1'-pyrano[3,4-b]indol]
4',9'-Dihydro-N,N-dimethyl-4-methoxypropyl-spiro[cyclohexan-1,1'(3'H)-pyrano[3,4-b]indol]-4-amin, 2-hydroxy-1,2,3-propantricarboxylat
6'-Fluor-4',9'-dihydro-N,N-dimethyl-4-methoxypropyl-spiro[cyclohexan-1,1'(3'H)-pyrano[3,4-b]indol]-4-amin, 2-hydroxy-1,2,3-propantricarboxylat
2',3',4',9'-Tetrahydro-N,N-dimethyl-4-(3-methoxypropyl)-spiro[cyclohexan-1,1'(1'H)-pyrido[3,4-b]indol]-4-amin, 2-hydroxy-1,2,3-propantricarboxylat
4',9'-Dihydro-N, N-dimethyl-4-(4-methoxybutyl)-spiro[cyclohexan-1,1'(3'H)-pyrano[3,4-b]indol]-4-amin, 2-hydroxy-1,2,3-propantricarboxylat
6'-Fluor-4',9'-dihydro-N,N-dimethyl-4-(4-methoxybutyl)-spiro[cyclohexan-1,1'(3'H)-pyrano[3,4-b]indol]-4-amin, 2-hydroxy-1,2,3-propantricarboxylat
2',3',4',9'-Tetrahydro-N,N-dimethyl-4-(4-methoxybutyl)-spiro[cyclohexan-1,1'(1'H)-pyrido[3,4-b]indol]-4-amin, 2-hydroxy-1,2,3-propantricarboxylat
2',3',4',9'-Tetrahydro-N,N-dimethyl-4-butyl-spiro[cyclohexan-1,1'(1'H)-pyrido[3,4-b]indol]-4-amin, 2-hydroxy-1,2,3-propantricarboxylat
6'-Fluor-4',9'-dihydro-N,N-dimethyl-4-ethyl-spiro[cyclohexan-1,1'(3'H)-pyrano[3,4-b]indol]-4-amin, 2-hydroxy-1,2,3-propantricarboxylat
2',3',4',9'-Tetrahydro-N,N-dimethyl-4-(3-methoxypropyl)-spiro[cyclohexan-1,1'(1'H)-pyrido[3,4-b]indol]-4-amin, 2-hydroxy-1,2,3-propantricarboxylat 6'-Fluor-4',9'-dihydro-N,N-dimethyl-4-methoxypropyl-spiro[cyclohexan-1,1'(3'H)-pyrano[3,4-b]indol]-4-amin, 2-hydroxy-1,2,3-propantricarboxylat
2',3',4',9'-Tetrahydro-N,N-dimethyl-4-butyl-2'-ethylaminocarbonyl-spiro[cydohexan-1,1'(1'H)-pyrido[3,4-b]indol]-4-amin
4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat
2',3',4',9'-Tetrahydro-N,N-dimethyl-4-(4-methoxybutyl)-spiro[cyclohexan-1,1'(1'H)-pyrido[3,4-b]indol]-4-amin, 2-hydroxy-1,2,3-propantricarboxylat
6'-Fluor-4',9'-dihydro-N-benzyl-4-allyl-spiro[cyclohexan-1,1'(3'H)-pyrano[3,4-b]indol]-4-amin, 2-hydroxy-1,2,3-propantricarboxylat
6'-Fluor-4',9'-dihydro-N-phenyl-4-allyl-spiro[cyclohexan-1,1'(3'H)-pyrano[3,4-b]indol]-4-amin
6'-Fluor-4',9'-dihydro-N-(4-methoxybenzyl)-4-allyl-spiro[cyclohexan-1,1'(3'H)-pyrano[3,4-b]indol]-4-amin
N-{6'-Fluor-4',9'-dihydro-4-butyl-spiro[cyclohexane-1,1'(3'H)-pyrano[3,4-b]indol]-4-yl}-pyrrolidin, 2-hydroxy-1,2,3-propantricarboxylat
N-{6'-Fluor-4',9'-dihydro-4-butyl-spiro[cyclohexan-1,1'(3'H)-pyrano[3,4-b]indol]-4-yl}-piperidin
N-{6'-Fluor-4',9'-dihydro-4-butyl-spiro[cyclohexan-1,1'(3'H)-pyrano[3,4-b]indol]-4-yl}-piperidin, 2-hydroxy-1,2,3-propantricarboxylat
N-{6'-Fluor-4',9'-dihydro-4-butyl-spiro[cyclohexan-1,1'(3H)-pyrano[3,4-b]indol]-4-yl}-n-methylpiperazin, 2-hydroxy-1,2,3-propantricarboxylat
4',9'-Dihydro-N,N-dimethyl-4-butyl-spiro[cyclohexan-1,1'(3'H)-pyrano[3,4-b]indol]-4-amin, 2-hydroxy-1,2,3-propantricarboxylat
6'-Hydroxy-4',9'-dihydro-N,N-dimethyl-4-butyl-spiro[cyclohexan-1,1'(3'H)-pyrano[3,4-b]indol]-4-amin, 2-hydroxy-1,2,3-propantricarboxylat
2',3',4',9'-Tetrahydro-N,N-dimethyl-4-butyl-2'-(2-phenylethencarbonyl)-spiro[cyclohexan-1,1'(1'H)-pyrido[3,4-b]indol]-4-amin, 2-hydroxy-1,2,3-propantricarboxylat,

gegebenenfalls auch als Gemisch.

10. Verfahren zur Herstellung von spirocyclischen Cyclohexanderivaten gemäß einem der Ansprüche 1-9, **dadurch gekennzeichnet, dass** ein Edukt der allgemeinen Formel E

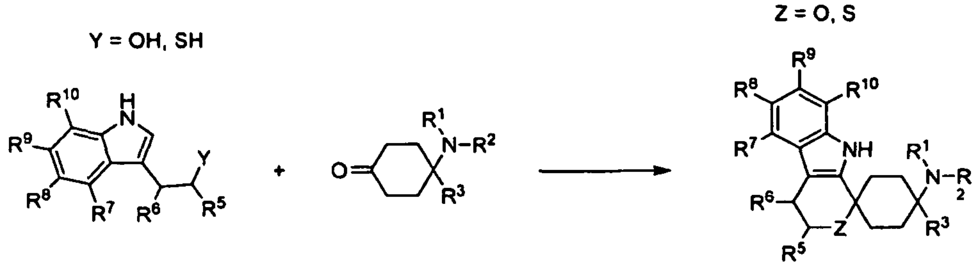


F E G

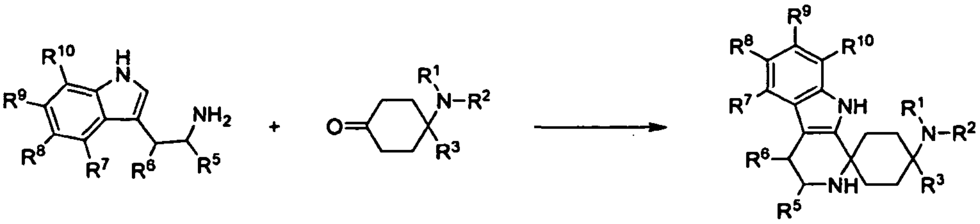

H E I

unter Zugabe von Säure, oder deren Trimethylsilylester, beispielsweise Trifluormethansulfonsäuretrimethylsilylester, Trifluormethansulfonsäure, Essigsäure, Phosphorsäure, Methansulfonsäure oder Trifluoressigsäure, in einem geeigneten Lösungsmittel, beispielsweise Dichlorethan, Dichlormethan, Chloroform, Acetonitril, Diethylether oder Nitromethan, mit einem Edukt der allgemeinen Formel F oder H umgesetzt wird, wobei die Reste $R^1$ bis $R^3$ und $R^5$ bis $R^{10}$ die in Anspruch 1 angegebenen Bedeutungen haben.

**11.** Verfahren zur Herstellung von spirocyclischen Cyclohexanderivaten gemäß Anspruch 1, bei denen X $NR^{17}$ und $R^{17}$ $COR^{12}$ oder $SO_2R^{12}$ bedeutet, **dadurch gekennzeichnet, dass** ein spirocyclisches Cyclohexanderivat, bei dem X NH bedeutet, unter Zugabe von Base, beispielsweise Triethylamin, mit einem Anhydrid oder einem Säurechlorid umgesetzt wird, vorzugsweise unter Mikrowelleneinstrahlung.

**12.** Verfahren zur Herstellung von spirocyclischen Cyclohexanderivaten gemäß Anspruch 1, bei denen X SO oder $SO_2$ bedeutet, **dadurch gekennzeichnet, dass** ein spirocyclisches Cyclohexanderivat, bei dem X S bedeutet, mit Hilfe eines Oxidationsmittels, beispielsweise $H_2O_2$, oxidiert wird.

**13.** Arzneimittel enthaltend wenigstens ein spirocyclisches Cyclohexan -Derivat gemäß einem der Ansprüche 1 bis 9 sowie gegebenenfalls enthaltend geeignete Zusatz- und/oder Hilfsstoffe und/oder gegebenenfalls weiterer Wirkstoffe.

**14.** Ein spirocyclisches Cyclohexan-Derivat gemäß einem der Ansprüche 1 bis 9 zur Verwendung für die Behandlung von Schmerz, insbesondere von akutem, neuropathischem oder chronischem Schmerz, von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

**1.** Spirocyclic cyclohexane derivatives of the general formula I

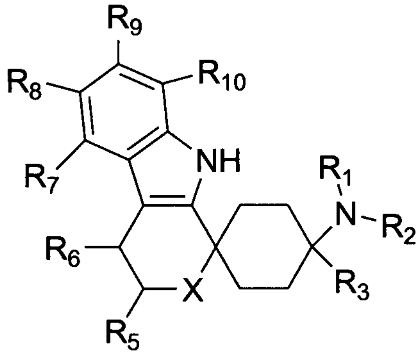

I

wherein

$R^1$ and $R^2$ independently of one another represent H; $C_{1-5}$-alkyl, in each case saturated or unsaturated, branched or unbranched, mono- or poly-substituted or unsubstituted; $C_{3-8}$-cycloalkyl, in each case saturated or unsaturated, mono- or poly-substituted or unsubstituted; aryl, unsubstituted or mono- or poly-substituted; or $C_{1-3}$-alkyl-bonded aryl, $C_{3-8}$-cycloalkyl or heteroaryl, in each case mono- or poly-substituted or unsubstituted;
or the radicals $R^1$ and $R^2$ together represent $CH_2CH_2OCH_2CH_2$, $CH_2CH_2NR^{11}CH_2CH_2$ or $(CH_2)_{3-6}$,
wherein $R^{11}$ denotes H; $C_{1-5}$-alkyl, in each case saturated or unsaturated, branched or unbranched, mono- or poly-substituted or unsubstituted; $C_{3-8}$-cycloalkyl, in each case saturated or unsaturated, mono- or poly-substituted or unsubstituted; aryl or heteroaryl, in each case mono- or poly-substituted or unsubstituted; or $C_{1-3}$-alkyl-bonded aryl, $C_{3-8}$-cycloalkyl or heteroaryl, in each case mono- or poly-substituted or unsubstituted;
$R^3$ represents $C_{1-8}$-alkyl, in each case saturated or unsaturated, branched or unbranched, mono- or poly-substituted or unsubstituted;
$R^5$ represents =O; H; $C_{1-5}$-alkyl, saturated or unsaturated, branched or unbranched, unsubstituted or mono- or poly-substituted; $COOR^{13}$, $CONR^{13}$, $OR^{13}$; $C_{3-8}$-cycloalkyl, saturated or unsaturated, unsubstituted or mono- or poly-substituted; aryl or heteroaryl, unsubstituted or mono- or poly-substituted; or $C_{1-3}$-alkyl-bonded aryl, $C_{3-8}$-cycloalkyl or heteroaryl, unsubstituted or mono- or poly-substituted;
$R^6$ represents H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-alkyl, saturated or unsaturated, branched or unbranched, unsubstituted or mono- or poly-substituted; $C_{3-8}$-cycloalkyl, saturated or unsaturated, unsubstituted or mono- or poly-substituted; aryl or heteroaryl, unsubstituted or mono- or poly-substituted; or $C_{1-3}$-alkyl-bonded aryl, $C_{3-8}$-cycloalkyl or heteroaryl, unsubstituted or mono- or poly-substituted;
or $R^5$ and $R^6$ together denote $(CH_2)_n$ where n = 2, 3, 4, 5 or 6, wherein individual hydrogen atoms can also be replaced by F, Cl, Br, I, $NO_2$, $CF_3$, $OR^{13}$, CN or $C_{1-5}$-alkyl;
$R^7$, $R^8$, $R^9$ and $R^{10}$ independently of one another represent
H, F, Cl, Br, I, $NO_2$, $CF_3$, $OR^{13}$, $SR^{13}$, $SO_2R^{13}$, $NHC(=O)NR^{14}R^{15}$, $SO_2NR^{14}R^{15}$, $SO_2OR^{13}$, CN, $COOR^{13}$, $NR^{14}R^{15}$; $C_{1-5}$-alkyl, $C_{3-8}$-cycloalkyl, unsubstituted or mono- or poly-substituted; aryl or heteroaryl, unsubstituted or mono- or poly-substituted; or $C_{1-3}$-alkyl-bonded aryl, $C_{3-8}$-cycloalkyl or heteroaryl, unsubstituted or mono- or poly-substituted;
wherein $R^{13}$ denotes H; $C_{1-5}$-alkyl, in each case saturated or unsaturated, branched or unbranched, unsubstituted or mono- or poly-substituted; $C_{3-8}$-cycloalkyl, in each case saturated or unsaturated, unsubstituted or mono- or poly-substituted; aryl or heteroaryl, unsubstituted or mono- or poly-substituted; or $C_{1-3}$-alkyl-bonded aryl, $C_{3-8}$-cycloalkyl or heteroaryl, unsubstituted or mono- or poly-substituted;
$R^{14}$ and $R^{15}$ independently of one another denote H; $C_{1-5}$-alkyl, in each case saturated or unsaturated, branched or unbranched, unsubstituted or mono- or poly-substituted; or $C_{3-8}$-cycloalkyl, in each case saturated or unsaturated, unsubstituted or mono- or poly-substituted; aryl or heteroaryl, unsubstituted or mono- or poly-substituted; or $C_{1-3}$-alkyl-bonded aryl, $C_{3-8}$-cycloalkyl or heteroaryl, unsubstituted or mono- or poly-substituted;
or $R^{14}$ and $R^{15}$ together form $CH_2CH_2OCH_2CH_2$, $CH_2CH_2NR^{16}CH_2CH_2$ or $(CH_2)_{3-6}$,

wherein $R^{16}$ denotes H; $C_{1-5}$-alkyl, saturated or unsaturated, branched or unbranched, unsubstituted or mono- or poly-substituted;

X represents O, S, SO, $SO_2$ or $NR^{17}$;
$R^{17}$ represents H; $C_{1-5}$-alkyl, saturated or unsaturated, branched or unbranched; $COR^{12}$ or $SO_2R^{12}$,

wherein $R^{12}$ denotes H; $C_{1-5}$-alkyl, in each case saturated or unsaturated, branched or unbranched, mono- or poly-substituted or unsubstituted; $C_{3-8}$-cycloalkyl, in each case saturated or unsaturated, mono- or poly-substituted or unsubstituted; aryl or heteroaryl, in each case mono- or poly-substituted or unsubstituted; or $C_{1-3}$-alkyl-bonded aryl, $C_{3-8}$-cycloalkyl or heteroaryl, in each case mono- or poly-substituted or unsubstituted; $OR^{13}$; $NR^{14}R^{15}$;
wherein "alkyl substituted" or "cycloalkyl substituted" denotes alkyl or cycloalkyl substituted by F, Cl, Br, I, CN, $CH_3$, $C_2H_5$, $NH_2$, $NO_2$, SH, $CF_3$, OH, $OCH_3$, $OC_2H_5$ or $N(CH_3)_2$ and
"aryl substituted" or "heteroaryl substituted" denotes aryl or heteroaryl substituted by F, Cl, Br, I, CN, $CH_3$, $C_2H_5$, $NH_2$, $NO_2$, SH, $CF_3$, OH, $OCH_3$, $OC_2H_5$ or $N(CH_3)_2$,
in the form of the racemate; of the enantiomers, diastereoisomers, mixtures of the enantiomers or diastereoisomers or of an individual enantiomer or diastereoisomer; of the bases and/or salts of physiologically acceptable acids or cations,
with the exception of the compound
2',3',4',9'-tetrahydro-N,N-dimethyl-4-butyl-spiro[cyclohexane-1,1'(1'H)-pyrido[3,4-b]indol]-4-amine.

**2.** Spirocyclic cyclohexane derivatives according to claim 1, wherein
$R^1$ and $R^2$ independently of one another represent H, $C_{1-5}$-alkyl, branched or unbranched, saturated or unsaturated, unsubstituted or mono- or poly-substituted, or phenyl or benzyl, unsubstituted or mono- or poly-substituted, or together represent a ring and denote $(CH_2)_{3-6}$.

**3.** Spirocyclic cyclohexane derivatives according to claim 1, wherein $R^3$ represents ethyl, n-propyl, 2-propyl, allyl, n-butyl, isobutyl, sec-butyl, tert-butyl, n-pentyl, isopentyl, neopentyl or n-hexyl, in each case unsubstituted or mono- or poly-substituted by OH, $OCH_3$ or $OC_2H_5$.

**4.** Spirocyclic cyclohexane derivatives according to claim 1, wherein
the radical $R^5$ represents H, $CH_3$, COOH, $COOCH_3$, $CH_2$Ophenyl, wherein the phenyl radical can be substituted by F, Cl, Br, I, CN, $CH_3$, $C_2H_5$, $NH_2$, $NO_2$, SH, $CF_3$, OH, $OCH_3$, $OC_2H_5$ or $N(CH_3)_2$, or $CH_2OH$.

**5.** Spirocyclic cyclohexane derivatives according to claim 1, wherein
$R^6$ can denote H; methyl, ethyl, $CF_3$, benzyl or phenyl, wherein the benzyl or phenyl radical can be substituted by F, Cl, Br, I, CN, $CH_3$, $C_2H_5$, $NH_2$, $NO_2$, SH, $CF_3$, OH, $OCH_3$, $OC_2H_5$ or $N(CH_3)_2$.

**6.** Spirocyclic cyclohexane derivatives according to claim 1, wherein $R^7$, $R^8$, $R^9$ and $R^{10}$ independently of one another denote H; $C_{1-5}$-alkyl, branched or unbranched, unsubstituted or mono- or poly-substituted; F, Cl, Br, I, $CF_3$, OH, $OCH_3$, $NH_2$, COOH, $COOCH_3$, $NHCH_3$, thienyl, pyrimidinyl, pyridyl, $N(CH_3)_2$ or $NO_2$.

**7.** Spirocyclic cyclohexane derivatives according to any one of claims 1 to 6, wherein X represents O.

**8.** Spirocyclic cyclohexane derivatives according to any one of claims 1 to 6, wherein X represents $NR^{17}$.

**9.** Spirocyclic cyclohexane derivatives according to claim 1 from the group:

4',9'-dihydro-N, N-dimethyl-4-ethyl-spiro[cyclohexane-1,1'(3'H)-pyrano[3,4-b]indol]-4-amine, 2-hydroxy-1,2,3-propanetricarboxylate
6'-fluoro-4',9'-dihydro-N,N-dimethyl-4-ethyl-spiro[cyclohexane-1,1'(3'H)-pyrano[3,4-b]indol]-4-amine, 2-hydroxy-1,2,3-propanetricarboxylate
2',3',4',9'-tetrahydro-N,N-dimethyl-4-ethyl-spiro[cyclohexane-1,1'(1'H)-pyrido[3,4-b]indol]-4-amine, 2-hydroxy-1,2,3-propanetricarboxylate
4',9'-dihydro-N,N-dimethyl-4-butyl-spiro[cyclohexane-1,1'(3'H)-pyrano[3,4-b]indol]-4-amine, 2-hydroxy-1,2,3-propanetricarboxylate
6'-fluoro-4',9'-dihydro-N,N-dimethyl-4-butyl-spiro[cyclohexane-1,1'(3'H)-pyrano[3,4-b]indol]-4-amine, 2-hydroxy-1,2,3-propanetricarboxylate
6'-fluoro-4',9'-dihydro-N,N-dimethyl-4-butyl-spiro[cyclohexane-1,1'(3'H)-pyrano[3,4-b]indol]-4-amine, 2-hydroxy-1,2,3-propanetricarboxylate
6'-hydroxy-4',9'-dihydro-N,N-dimethyl-4-butyl-spiro[cyclohexane-1,1'(3'H)-pyrano[3,4-b]indol]-4-amine, 2,2,2-trifluoroacetate
6'-hydroxy-4',9'-dihydro-N,N-dimethyl-4-butyl-spiro[cyclohexane-1,1'(3'H)-pyrano[3,4-b]indol]-4-amine, 2-hydroxy-1,2,3-propanetricarboxylate

2’,3’,4’,9’-tetrahydro-N,N-dimethyl-4-butyl-spiro[cyclohexane-1,1’(1’H)-pyrido[3,4-b]indol]-4-amine, 2-hydroxy-1,2,3-propanetricarboxylate
2’,3’,4’,9’-tetrahydro-N,N-dimethyl-4-butyl-2’-methylcarbonyl-spiro[cyclohexane-1,1’(1’H)-pyrido[3,4-b]indol]-4-amine, 2-hydroxy-1,2,3-propanetricarboxylate
2’,3’,4’,9’-tetrahydro-N,N-dimethyl-4-butyl-2’-cyclopentylcarbonyl-spiro[cyclohexane-1,1’(1’H)-pyrido[3,4-b]indol]-4-amine, 2-hydroxy-1,2,3-propanetricarboxylate
2’,3’,4’,9’-tetrahydro-N,N-dimethyl-4-butyl-2’-(2,2)-dimethylpropanecarbonyl-spiro[cyclohexane-1,1’(1’H)-pyrido[3,4-b]indol]-4-amine, 2-hydroxy-1,2,3-propanetricarboxylate
2’,3’,4’,9’-tetrahydro-N,N-dimethyl-4-butyl-2’-(3,4-dimethoxybenzylcarbonyl)-spiro[cyclohexane-1,1’(1’H)-pyrido[3,4-b]indol]-4-amine, 2-hydroxy-1,2,3-propanetricarboxylate
2’,3’,4’,9’-tetrahydro-N,N-dimethyl-4-butyl-2’-ethylaminocarbonyl-spiro[cyclohexane-1,1’(1’H)-pyrido[3,4-b]indol]-4-amine, 2-hydroxy-1,2,3-propanetricarboxylate
2’,3’,4’,9’-tetrahydro-N,N-dimethyl-4-butyl-2’-4-methoxybenzylaminocarbonyl-spiro[cyclohexane-1,1’(1’H)-pyrido[3,4-b]indol]-4-amine
2’,3’,4’,9’-tetrahydro-N, N-d imethyl-4-butyl-2’-methyl-spiro[cyclohexane-1,1’(1’H)-pyrido[3,4-b]indol]-4-amine, 2-hydroxy-1,2,3-propanetricarboxylate
6’-fluoro-4’,9’-dihydro-N-ethyl-N-methyl-4-butyl-spiro[cyclohexane-1,1’(3’H)-pyrano[3,4-b]indol]-4-amine, 2-hydroxy-1,2,3-propanetricarboxylate
6’-fluoro-4’,9’-dihydro-N-benzyl-N-methyl-4-butyl-spiro[cyclohexane-1,1’(3’H)-pyrano[3,4-b]indol]-4-amine
6’-fluoro-4’,9’-dihydro-N-phenyl-4-butyl-spiro[cyclohexane-1,1’(3’H)-pyrano[3,4-b]indol]-4-amine
4-butyl-6’-fluoro-4-(*N*-morpholino)-1’,3’,4’,9’-tetrahydrospiro[cyclohexane-1,1’-pyrano[3,4-b]indole]
4-butyl-6’-fluoro-4-(*N*-morpholino)-1’,3’,4’,9’-tetrahydrospiro[cyclohexane-1,1’-pyrano[3,4-b]indole]
4’,9’-dihydro-N,N-dimethyl-4-methoxypropyl-spiro[cyclohexane-1,1’(3’H)-pyrano[3,4-b]indol]-4-amine, 2-hydroxy-1,2,3-propanetricarboxylate
6’-fluoro-4’,9’-d ihyd ro-N, N-d imethyl-4-methoxypropyl-spiro[cyclohexane-1,1’(3’H)-pyrano[3,4-b]indol]-4-amine, 2-hydroxy-1,2,3-propanetricarboxylate
2’,3’,4’,9’-tetrahydro-N,N-dimethyl-4-(3-methoxypropyl)-spiro[cyclohexane-1,1’(1’H)-pyrido[3,4-b]indol]-4-amine, 2-hydroxy-1,2,3-propanetricarboxylate
4’,9’-dihydro-N,N-dimethyl-4-(4-methoxybutyl)-spiro[cyclohexane-1,1’(3’H)-pyrano[3,4-b]indol]-4-amine, 2-hydroxy-1,2,3-propanetricarboxylate
6’-fluoro-4’,9’-dihydro-N,N-dimethyl-4-(4-methoxybutyl)-spiro[cyclohexane-1,1’(3’H)-pyrano[3,4-b]indol]-4-amine, 2-hydroxy-1,2,3-propanetricarboxylate
2’,3’,4’,9’-tetrahydro-N,N-dimethyl-4-(4-methoxybutyl)-spiro[cyclohexane-1,1’(1’H)-pyrido[3,4-b]indol]-4-amine, 2-hydroxy-1,2,3-propanetricarboxylate
2’,3’,4’,9’-tetrahydro-N,N-dimethyl-4-butyl-spiro[cyclohexane-1,1’(1’H)-pyrido[3,4-b]indol]-4-amine, 2-hydroxy-1,2,3-propanetricarboxylate
6’-fluoro-4’,9’-dihydro-N,N-dimethyl-4-ethyl-spiro[cyclohexane-1,1’(3’H)-pyrano[3,4-b]indol]-4-amine, 2-hydroxy-1,2,3-propanetricarboxylate
2’,3’,4’,9’-tetrahydro-N,N-dimethyl-4-(3-methoxypropyl)-spiro[cyclohexane-1,1’(1’H)-pyrido[3,4-b]indol]-4-amine, 2-hydroxy-1,2,3-propanetricarboxylate
6’-fluoro-4’,9’-dihydro-N,N-dimethyl-4-methoxypropyl-spiro[cyclohexane-1,1’(3’H)-pyrano[3,4-b]indol]-4-amine, 2-hydroxy-1,2,3-propanetricarboxylate
2’,3’,4’,9’-tetrahydro-N,N-dimethyl-4-butyl-2’-ethylaminocarbonyl-spiro[cyclohexane-1,1’(1’H)-pyrido[3,4-b]indol]-4-amine
4’,9’-dihydro-N,N-dimethyl-4-butyl-spiro[cyclohexane-1,1’(3’H)-pyrano[3,4-b]indol]-4-amine, 2-hydroxy-1,2,3-propanetricarboxylate
2’,3’,4’,9’-tetrahydro-N,N-dimethyl-4-(4-methoxybutyl)-spiro[cyclohexane-1,1’(1’H)-pyrido[3,4-b]indol]-4-amine, 2-hydroxy-1,2,3-propanetricarboxylate
6’-fluoro-4’,9’-dihydro-N-benzyl-4-allyl-spiro[cyclohexane-1,1’(3’H)-pyrano[3,4-b]indol]-4-amine, 2-hydroxy-1,2,3-propanetricarboxylate
6’-fluoro-4’,9’-dihydro-N-phenyl-4-allyl-spiro[cyclohexane-1,1’(3’H)-pyrano[3,4-b]indol]-4-amine
6’-fluoro-4’,9’-dihydro-N-(4-methoxybenzyl)-4-allyl-spiro[cyclohexane-1,1’(3’H)-pyrano[3,4-b]indol]-4-amine
N-{6’-fluoro-4’,9’-dihydro-4-butyl-spiro[cyclohexane-1,1’(3’H)-pyrano[3,4-b]indol]-4-yl}-pyrrolidine, 2-hydroxy-1,2,3-propanetricarboxylate
N-{6’-fluoro-4’,9’-dihydro-4-butyl-spiro[cyclohexane-1,1’(3’H)-pyrano[3,4-b]indol]-4-yl}-piperidine
N-{6’-fluoro-4’,9’-dihydro-4-butyl-spiro[cyclohexane-1,1’(3’H)-pyrano[3,4-b]indol]-4-yl}-piperidine, 2-hydroxy-1,2,3-propanetricarboxylate
N-{6’-fluoro-4’,9’-dihydro-4-butyl-spiro[cyclohexane-1,1’(3’H)-pyrano[3,4-b]indol]-4-yl}-n-methylpiperazine, 2-hydroxy-1,2,3-propanetricarboxylate
4',9'-dihydro-N,N-dimethyl-4-butyl-spiro[cyclohexane-1,1'(3'H)-pyrano[3,4-b]indol]-4-amine, 2-hydroxy-1,2,3-propanetricarboxylate
6'-hydroxy-4',9'-dihydro-N,N-dimethyl-4-butyl-spiro[cyclohexane-1,1'(3'H)-pyrano[3,4-b]indol]-4-amine, 2-hydroxy-1,2,3-propanetricarboxylate
2',3',4',9'-tetrahydro-N,N-dimethyl-4-butyl-2'-(2-phenylethenecarbonyl)-spiro[cyclohexane-1,1'(1'H)-pyrido[3,4-b]indol]-4-amine, 2-hydroxy-1,2,3-propanetricarboxylate,

optionally also in the form of a mixture.

**10.** Process for the preparation of spirocyclic cyclohexane derivatives according to any one of claims 1 to 9, **characterised in that** a starting material of the general formula E

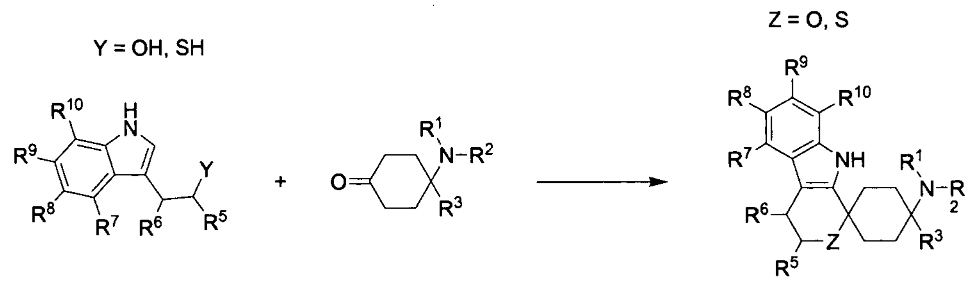


F E G

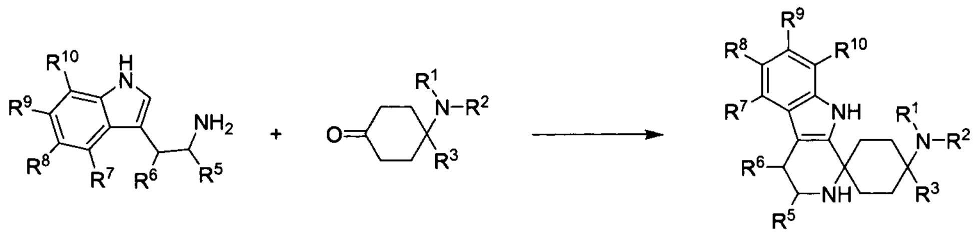

H E I

is reacted, with the addition of acid or trimethylsilyl esters thereof, for example trifluoromethanesulfonic acid trimethylsilyl ester, trifluoromethanesulfonic acid, acetic acid, phosphoric acid, methanesulfonic acid or trifluoroacetic acid, in a suitable solvent, for example dichloroethane, dichloromethane, chloroform, acetonitrile, diethyl ether or nitromethane, with a starting material of the general formula F or H, wherein the radicals $R^1$ to $R^3$ and $R^5$ to $R^{10}$ have the meanings given in claim 1.

**11.** Process for the preparation of spirocyclic cyclohexane derivatives according to claim 1 in which X denotes $NR^{17}$ and $R^{17}$ denotes $COR^{12}$ or $SO_2R^{12}$, **characterised in that** a spirocyclic cyclohexane derivative in which X denotes NH is reacted, with the addition of base, for example triethylamine, with an anhydride or an acid chloride, preferably with microwave radiation.

**12.** Process for the preparation of spirocyclic cyclohexane derivatives according to claim 1 in which X denotes SO or $SO_2$, **characterised in that** a spirocyclic cyclohexane derivative in which X denotes S is oxidised with the aid of an oxidising agent, for example $H_2O_2$.

**13.** Medicament comprising at least one spirocyclic cyclohexane derivative according to any one of claims 1 to 9 and optionally comprising suitable additives and/or auxiliary substances and/or optionally further active ingredients.

**14.** A spirocyclic cyclohexane derivative according to any one of claims 1 to 9 for use for the treatment of pain, especially acute, neuropathic or chronic pain, anxiety, stress and stress-associated syndromes, depression, epilepsy, Alzheimer's disease, senile dementia, general cognitive dysfunctions, learning and memory disorders (as a nootropic), withdrawal symptoms, alcohol and/or drug and/or medicament abuse and/or dependency, sexual dysfunctions, cardiovascular diseases, hypotension, hypertension, tinnitus, pruritus, migraine, impaired hearing, deficient intestinal

motility, impaired food intake, anorexia, obesity, locomotor disorders, diarrhoea, cachexia, urinary incontinence or as a muscle relaxant, anticonvulsive or anaesthetic or for co-administration in the case of treatment with an opioid analgesic or with an anaesthetic, for diuresis or antinatriuresis, anxiolysis, for modulation of motor activity, for modulation of neurotransmitter secretion and treatment of neurodegenerative diseases associated therewith, for the treatment of withdrawal symptoms and/or for reducing the addictive potential of opioids.

**Revendications**

1. Dérivés de cyclohexane spirocycliques de formule générale I

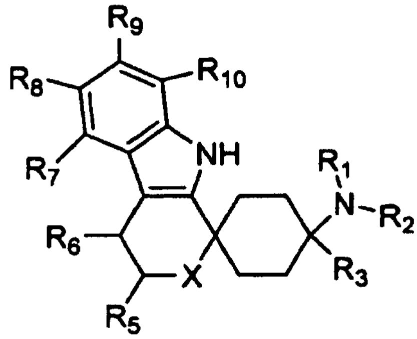

I,

dans laquelle

$R^1$ et $R^2$ représentent, indépendamment l'un de l'autre, H ; alkyle en $C_{1-5}$ à chaque fois saturé ou insaturé, ramifié ou non ramifié, substitué une ou plusieurs fois ou non substitué ; cycloalkyle en $C_{3-8}$, à chaque fois saturé ou insaturé, substitué une ou plusieurs fois ou non substitué ; aryle, non substitué ou substitué une ou plusieurs fois ; ou aryle, cycloalkyle en $C_{3-8}$ ou hétéroaryle relié par alkyle en $C_{1-3}$, à chaque fois substitué une ou plusieurs fois ou non substitué ;

ou les radicaux $R^1$ et $R^2$ représentent ensemble $CH_2CH_2OCH_2CH_2$, $CH_2CH_2NR^{11}CH_2CH_2$ ou $(CH_2)_{3-6}$,

$R^{11}$ représentant H ; alkyle en $C_{1-5}$, à chaque fois saturé ou insaturé, ramifié ou non ramifié, substitué une ou plusieurs fois ou non substitué ; cycloalkyle en $C_{3-8}$, à chaque fois saturé ou insaturé, substitué une ou plusieurs fois ou non substitué ; aryle ou hétéroaryle, à chaque fois substitué une ou plusieurs fois ou non substitué ; ou aryle, cycloalkyle en $C_{3-8}$ ou hétéroaryle relié par alkyle en $C_{1-3}$, à chaque fois substitué une ou plusieurs fois ou non substitué ;

$R^3$ représente alkyle en $C_{1-8}$, à chaque fois saturé ou insaturé, ramifié ou non ramifié, substitué une ou plusieurs fois ou non substitué ;

$R^5$ représente =O ; H ; alkyle en $C_{1-5}$, saturé ou insaturé, ramifié ou non ramifié, non substitué ou substitué une ou plusieurs fois ; $COOR^{13}$, $CONR^{13}$, $OR^{13}$; cycloalkyle en $C_{3-8}$, saturé ou insaturé, non substitué ou substitué une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ;

$R^6$ représente H ; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$ ; alkyle en $C_{1-5}$, saturé ou insaturé, ramifié ou non ramifié, non substitué ou substitué une ou plusieurs fois ; cycloalkyle en $C_{3-8}$, saturé ou insaturé, non substitué ou substitué une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ;

ou $R^5$ et $R^6$ signifient ensemble $(CH_2)_n$ avec n = 2, 3, 4, 5 ou 6, des atomes d'hydrogène individuels pouvant également être remplacés par F, Cl, Br, I, $NO_2$, $CF_3$, $OR^{13}$, $C_N$ ou alkyle en $C_{1-5}$ ;

$R^7$, $R^8$, $R^9$ et $R^{10}$ représentent indépendamment les uns des autres

H, F, Cl, Br, I, $NO_2$, $CF_3$, $OR^{13}$, $SR^{13}$, $SO_2R^{13}$, NHC (=O) $NR^{14}R^{15}$, $SO_2NR^{14}R^{15}$, $SO_2OR^{13}$, CN, $COOR^{13}$, $NR^{14}R^{15}$; alkyle en $C_{1-5}$, cycloalkyle en $C_{3-8}$, non substitué ou substitué une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ;

$R^{13}$ signifiant H ; alkyle en $C_{1-5}$ à chaque fois saturé ou insaturé, ramifié ou non ramifié, non substitué ou substitué une ou plusieurs fois ; cycloalkyle en $C_{3-8}$, à chaque fois saturé ou insaturé, non substitué ou substitué

une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ;

$R^{14}$ et $R^{15}$ signifiant indépendamment l'un de l'autre H ; alkyle en $C_{1-5}$, à chaque fois saturé ou insaturé, ramifié ou non ramifié, non substitué ou substitué une ou plusieurs fois ; cycloalkyle en $C_{3-8}$, à chaque fois saturé ou insaturé, non substitué ou substitué une ou plusieurs fois ; aryle ou hétéroaryle, non substitué ou substitué une ou plusieurs fois ; ou aryle, cycloalkyle en $C_{3-8}$ ou hétéroaryle relié par alkyle en $C_{1-3}$, non substitué ou substitué une ou plusieurs fois ;

ou $R^{14}$ et $R^{15}$ formant ensemble $CH_2CH_2OCH_2CH_2$, $CH_2CH_2NR^{16}CH_2CH_2$ ou $(CH_2)_{3-6}$,

$R^{16}$ signifiant H ; alkyle en $C_{1-5}$ saturé ou insaturé, ramifié ou non ramifié, non substitué ou substitué une ou plusieurs fois ;

X représente O, S, SO, $SO_2$ ou $NR^{17}$ ;

$R^{17}$ représentant H ; alkyle en $C_{1-5}$, saturé ou insaturé, ramifié ou non ramifié ; $COR^{12}$ ou $SO_2R^{12}$,

$R^{12}$ signifiant H ; alkyle en $C_{1-5}$, à chaque fois saturé ou insaturé, ramifié ou non ramifié, substitué une ou plusieurs fois ou non substitué ; cycloalkyle en $C_{3-8}$, à chaque fois saturé ou insaturé, substitué une ou plusieurs fois ou non substitué ; aryle ou hétéroaryle, à chaque fois substitué une ou plusieurs fois ou non substitué ; ou aryle, cycloalkyle en $C_{3-8}$ ou hétéroaryle relié par alkyle en $C_{1-3}$, à chaque fois substitué une ou plusieurs fois ou non substitué ; $OR^{13}$ ; $NR^{14}R^{15}$ ; « alkyle substitué » ou « cycloalkyle substitué » signifiant alkyle ou cycloalkyle substitué avec F, Cl, Br, I, CN, $CH_3$, $C_2H_5$, $NH_2$, $NO_2$, SH, $CF_3$, OH, $OCH_3$, $OC_2H_5$ ou $N(CH_3)_2$, et « aryle substitué » ou « hétéroaryle substitué » signifiant aryle ou hétéroaryle substitué avec F, Cl, Br, I, CN, $CH_3$, $C_2H_5$, $NH_2$, $NO_2$, SH, $CF_3$, OH, $OCH_3$, $OC_2H_5$ ou $N(CH_3)_2$,

sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement compatibles,

à l'exception du composé 2',3',4',9'-tétrahydro-N,N-diméthyl-4-butyl-spiro[cyclohexane-1,1'(1'H)-pyrido[3,4-b] indol]-4-amine.

**2.** Dérivés de cyclohexane spirocycliques selon la revendication 1, dans lesquels
$R^1$ et $R^2$ représentent, indépendamment l'un de l'autre, H, alkyle en $C_{1-5}$ ramifié ou non ramifié, saturé ou insaturé, non substitué ou substitué une ou plusieurs fois, ou phényle ou benzyle, non substitué ou substitué une ou plusieurs fois,
ou représentent ensemble un cycle et signifient $(CH_2)_{3-6}$.

**3.** Dérivés de cyclohexane spirocycliques selon la revendication 1, dans lesquels
$R^3$ représente éthyle, n-propyle, 2-propyle, allyle, n-butyle, iso-butyle, sec.-butyle, tert.-butyle, n-pentyle, iso-pentyle, néo-pentyle ou n-hexyle, à chaque fois non substitué ou substitué une ou plusieurs fois avec OH, $OCH_3$ ou $OC_2H_5$.

**4.** Dérivés de cyclohexane spirocycliques selon la revendication 1, dans lesquels
le radical $R^5$ représente H, $CH_3$, COOH, $COOCH_3$, $CH_2O$-phényle, le radical phényle pouvant être substitué avec F, Cl, Br, I, CN, $CH_3$, $C_2H_5$, $NH_2$, $NO_2$, SH, $CF_3$, OH, $OCH_3$, $OC_2H_5$ ou $N(CH_3)_2$, ou $CH_2OH$.

**5.** Dérivés de cyclohexane spirocycliques selon la revendication 1, dans lesquels
$R^6$ peut signifier H ; méthyle, éthyle, $CF_3$, benzyle ou phényle, le radical benzyle ou phényle pouvant être substitué avec F, Cl, Br, I, CN, $CH_3$, $C_2H_5$, $NH_2$, $NO_2$, SH, $CF_3$, OH, $OCH_3$, $OC_2H_5$ ou $N(CH_3)_2$,

**6.** Dérivés de cyclohexane spirocycliques selon la revendication 1, dans lesquels $R^7$, $R^8$, $R^9$ et $R^{10}$ signifient indépendamment les uns des autres H ; alkyle en $C_{1-5}$, ramifié ou non ramifié, non substitué ou substitué une ou plusieurs fois ; F, Cl, Br, I, $CF_3$, OH, $OCH_3$, $NH_2$, COOH, $COOCH_3$, $NHCH_3$, thiényle, pyrimidinyle, pyridyle, $N(CH_3)_2$ ou $NO_2$.

**7.** Dérivés de cyclohexane spirocycliques selon l'une quelconque des revendications 1 à 6, dans lesquels X représente O.

**8.** Dérivés de cyclohexane spirocycliques selon l'une quelconque des revendications 1 à 6, dans lesquels X représente $NR^{17}$.

**9.** Dérivés de cyclohexane spirocycliques selon la revendication 1, choisis dans le groupe constitué par :

4',9'-dihydro-N,N-diméthyl-4-éthyl-spiro[cyclohexane-1,1'(3'H)-pyrano[3,4-b]indol]-4-amine, 2-hydroxy-1,2,3-propanetricarboxylate

6’-fluoro-4’,9’-dihydro-N,N-diméthyl-4-éthyl-spiro[cyclohexane-1,1’(3’H)-pyrano[3,4-b]indol]-4-amine, 2-hydroxy-1,2,3-propanetricarboxylate
2’,3’,4’,9’-tétrahydro-N,N-diméthyl-4-éthyl-spiro[cyclohexane-1,1’(1’H)-pyrido[3,4-b]indol]-4-amine, 2-hydroxy-1,2,3-propanetricarboxylate
4’,9’-dihydro-N,N-diméthyl-4-butyl-spiro[cyclohexane-1,1’(3’H)-pyrano[3,4-b]indol]-4-amine, 2-hydroxy-1,2,3-propanetricarboxylate
6’-fluoro-4’,9’-dihydro-N,N-diméthyl-4-butyl-spiro[cyclohexane-1,1’(3’H)-pyrano[3,4-b]indol]-4-amine, 2-hydroxy-1,2,3-propanetricarboxylate
6’-fluoro-4’,9’-dihydro-N,N-diméthyl-4-butyl-spiro[cyclohexane-1,1’(3’H)-pyrano[3,4-b]indol]-4-amine, 2-hydroxy-1,2,3-propanetricarboxylate
6’-hydroxy-4’,9’-dihydro-N,N-diméthyl-4-butyl-spiro[cyclohexane-1,1’(3’H)-pyrano[3,4-b]indol]-4-amine, 2,2,2-trifluoroacétate
6’-hydroxy-4’,9’-dihydro-N,N-diméthyl-4-butyl-spiro[cyclohexane-1,1’(3’H)-pyrano[3,4-b]indol]-4-amine, 2-hydroxy-1,2,3-propanetricarboxylate
2’,3’,4’,9’-tétrahydro-N,N-diméthyl-4-butyl-spiro[cyclohexane-1,1’(1’H)-pyrido[3,4-b]indol]-4-amine, 2-hydroxy-1,2,3-propanetricarboxylate
2’,3’,4’,9’-tétrahydro-N,N-diméthyl-4-butyl-2’-méthylcarbonyl-spiro[cyclohexane-1,1’(1’H)-pyrido[3,4-b]indol]-4-amine, 2-hydroxy-1,2,3-propanetricarboxylate
2’,3’,4’,9’-tétrahydro-N,N-diméthyl-4-butyl-2’-cyclopentylcarbonyl-spiro[cyclohexane-1,1’(1’H)-pyrido[3,4-b]-indol]-4-amine, 2-hydroxy-1,2,3-propanetricarboxylate
2’,3’,4’,9’-tétrahydro-N,N-diméthyl-4-butyl-2’-(2,2)-diméthylpropanecarbonyl-spiro[cyclohexane-1,1’(1’H)-pyrido[3,4-b]indol]-4-amine, 2-hydroxy-1,2,3-propanetricarboxylate
2’,3’,4’,9’-tétrahydro-N,N-diméthyl-4-butyl-2’-(3,4-diméthoxybenzylcarbonyl)-spiro[cyclohexane-1,1’(1’H)-pyrido[3,4-b]indol]-4-amine, 2-hydroxy-1,2,3-propanetricarboxylate
2’,3’,4’,9’-tétrahydro-N,N-diméthyl-4-butyl-2’-éthylaminocarbonyl-spiro[cyclohexane-1,1’(1’H)-pyrido[3,4-b]indol]-4-amine, 2-hydroxy-1,2,3-propanetricarboxylate
2’,3’,4’,9’-tétrahydro-N,N-diméthyl-4-butyl-2’-4-méthoxybenzylaminocarbonyl-spiro[cyclohexane-1,1’(1’H)-pyrido[3,4-b]indol]-4-amine
2’,3’,4’,9’-tétrahydro-N,N-diméthyl-4-butyl-2’-méthyl-spiro[cyclohexane-1,1’(1’H)-pyrido[3,4-b]indol]-4-amine, 2-hydroxy-1,2,3-propanetricarboxylate
6’-fluoro-4’,9’-dihydro-N-éthyl-N-méthyl-4-butyl-spiro[cyclohexane-1,1’(3’H)-pyrano[3,4-b]indol]-4-amine, 2-hydroxy-1,2,3-propanetricarboxylate
6’-fluoro-4’,9’-dihydro-N-benzyl-N-méthyl-4-butyl-spiro[cyclohexane-1,1’(3’H)-pyrano[3,4-b]indol]-4-amine
6’-fluoro-4’,9’-dihydro-N-phényl-4-butyl-spiro[cyclohexane-1,1’(3’H)-pyrano[3,4-b]indol]-4-amine
4-butyl-6’-fluoro-4-(N-morpholino)-1’,3’,4’,9’-tétrahydrospiro[cyclohexane-1,1’-pyrano[3,4-b]indole]
4-butyl-6’-fluoro-4-(N-morpholino)-1’,3’,4’,9’-tétrahydrospiro[cyclohexane-1,1’-pyrano[3,4-b]indole]
4’,9’-dihydro-N,N-diméthyl-4-méthoxypropyl-spiro[cyclohexane-1,1’(3’H)-pyrano[3,4-b]indol]-4-amine, 2-hydroxy-1,2,3-propanetricarboxylate
6’-fluoro-4’,9’-dihydro-N,N-diméthyl-4-méthoxypropyl-spiro[cyclohexane-1,1’(3’H)-pyrano[3,4-b]indol]-4-amine, 2-hydroxy-1,2,3-propanetricarboxylate
2’,3’,4’,9’-tétrahydro-N,N-diméthyl-4-(3-méthoxypropyl)-spiro[cyclohexane-1,1’(l’H)-pyrido[3,4-b]indol]-4-amine, 2-hydroxy-1,2,3-propanetricarboxylate
4’,9’-dihydro-N,N-diméthyl-4-(4-méthoxybutyl)-spiro[cyclohexane-1,1’(3’H)-pyrano[3,4-b]indol]-4-amine, 2-hydroxy-1,2,3-propanetricarboxylate
6’-fluoro-4’,9’-dihydro-N,N-diméthyl-4-(4-méthoxybutyl)-spiro[cyclohexane-1,1’(3’H)-pyrano[3,4-b]indol]-4-amine, 2-hydroxy-1,2,3-propanetricarboxylate
2’,3’,4’,9’-tétrahydro-N,N-diméthyl-4-(4-méthoxybutyl)--spiro[cyclohexane-1,1’(1’H)-pyrido[3,4-b]indol]-4-amine, 2-hydroxy-1,2,3-propanetricarboxylate
2’,3’,4’,9’-tétrahydro-N,N-diméthyl-4-butyl-spiro[cyclohexane-1,1’(1’H)-pyrido[3,4-b]indol]-4-amine, 2-hydroxy-1,2,3-propanetricarboxylate
6’-fluoro-4’,9’-dihydro-N,N-diméthyl-4-éthyl-spiro[cyclohexane-1,1’(3’H)-pyrano[3,4-b]indol]-4-amine, 2-hydroxy-1,2,3-propanetricarboxylate
2’,3’,4’,9’-tétrahydro-N,N-diméthyl-4-(3-méthoxypropyl)-spiro[cyclohexane-1,1’(l’H)-pyrido[3,4-b]indol]-4-amine, 2-hydroxy-1,2,3-propanetricarboxylate
6’-fluoro-4’,9’-dihydro-N,N-diméthyl-4-méthoxypropyl-spiro[cyclohexane-1,1’(3’H)-pyrano[3,4-b]indol]-4-amine, 2-hydroxy-1,2,3-propanetricarboxylate
2’,3’,4’,9’-tétrahydro-N,N-diméthyl-4-butyl-2’-éthylaminocarbonyl-spiro[cyclohexane-1,1’(l’H)-pyrido[3,4-b]indol]-4-amine

4',9'-dihydro-N,N-diméthyl-4-butyl-spiro[cyclohexane-1,1'(3'H)-pyrano[3,4-b]indol]-4-amine, 2-hydroxy-1,2,3-propanetricarboxylate
2',3',4',9'-tétrahydro-N,N-diméthyl-4-(4-méthoxybutyl)--spiro[cyclohexane-1,1'(l'H)-pyrido[3,4-b]indol]-4-amine, 2-hydroxy-1,2,3-propanetricarboxylate
6'-fluoro-4',9'-dihydro-N-benzyl-4-allyl-spiro[cyclohexane-1,1'(3'H)-pyrano[3,4-b]indol]-4-amine, 2-hydroxy-1,2,3-propanetricarboxylate
6'-fluoro-4',9'-dihydro-N-phényl-4-allyl-spiro[cyclohexane-1,1'(3'H)-pyrano[3,4-b]indol]-4-amine
6'-fluoro-4',9'-dihydro-N-(4-méthoxybenzyl)-4-allyl-spiro[cyclohexane-1,1'(3'H)-pyrano[3,4-b]indol]-4-amine
N-{6'-fluoro-4',9'-dihydro-4-butyl-spiro[cyclohexane-1,1'(3'H)-pyrano[3,4-b]indol]-4-yl}-pyrrolidine, 2-hydroxy-1,2,3-propanetricarboxylate
N-{6'-fluoro-4',9'-dihydro-4-butyl-spiro[cyclohexane-1,1'(3'H)-pyrano[3,4-b]indol]-4-yl}-pipéridine
N-{6'-fluoro-4',9'-dihydro-4-butyl-spiro[cyclohexane-1,1'(3'H)-pyrano[3,4-b]indol]-4-yl}-pipéridine, 2-hydroxy-1,2,3-propanetricarboxylate
N-{6'-fluoro-4',9'-dihydro-4-butyl-spiro[cyclohexane-1,1'(3'H)-pyrano(3,4-b]indol]-4-yl}-n-méthylpipérazine, 2-hydroxy-1,2,3-propanetricarboxylate
4',9'-dihydro-N,N-diméthyl-4-butyl-spiro[cyclohexane-1,1'(3'H)-pyrano[3,4-b]indol]-4-amine, 2-hydroxy-1,2,3-propanetricarboxylate
6'-hydroxy-4',9'-dihydro-N,N-diméthyl-4-butyl-spiro[cyclohexane-1,1'(3'H)-pyrano[3,4-b]indol]-4-amine, 2-hydroxy-1,2,3-propanetricarboxylate
2',3',4',9'-tétrahydro-N,N-diméthyl-4-butyl-2'-(2-phényléthènecarbonyl)-spiro[cyclohexane-1,1'(1'H)-pyrido[3,4-b]indol]-4-amine, 2-hydroxy-1,2,3-propanetricarboxylate,

éventuellement également en mélange.

**10.** Procédé de fabrication de dérivés de cyclohexane spirocycliques selon l'une quelconque des revendications 1 à 9, **caractérisé en ce qu'**un réactif de formule générale E

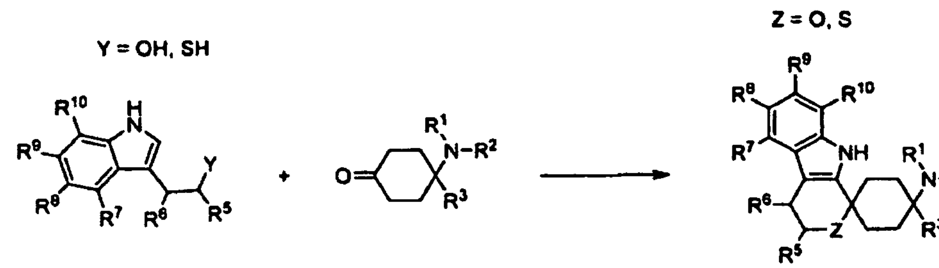


F E G

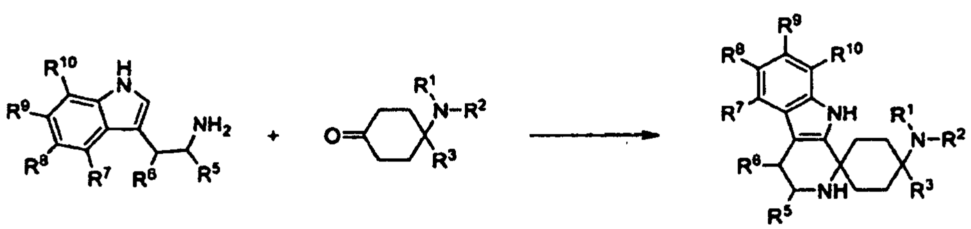

H E I

est mis en réaction avec ajout d'un acide, ou son ester triméthylsilylique, par exemple l'ester triméthylsilylique de l'acide trifluorométhanesulfonique, l'acide trifluorométhanesulfonique, l'acide acétique, l'acide phosphorique, l'acide méthanesulfonique ou l'acide trifluoroacétique, dans un solvant approprié, par exemple le dichloroéthane, le dichlorométhane, le chloroforme, l'acétonitrile, l'éther diéthylique ou le nitrométhane, avec un réactif de formule générale F ou H, les radicaux $R^1$ à $R^3$ et $R^5$ à $R^{10}$ ayant les significations indiquées dans la revendication 1.

**11.** Procédé de fabrication de dérivés de cyclohexane spirocycliques selon la revendication 1, dans lesquels X signifie $NR^{17}$ et $R^{17}$ signifie $COR^{12}$ ou $SO_2R^{12}$, **caractérisé en ce qu'**un dérivé de cyclohexane spirocyclique, dans lequel X signifie NH, est mis en réaction avec ajout d'une base, par exemple de triéthylamine, avec un anhydride ou un chlorure d'acide, de préférence sous un rayonnement micro-onde.

**12.** Procédé de fabrication de dérivés de cyclohexane spirocycliques selon la revendication 1, dans lesquels X signifie SO ou $SO_2$, **caractérisé en ce qu'**un dérivé de cyclohexane spirocyclique, dans lequel X signifie S, est oxydé à l'aide d'un oxydant, par exemple $H_2O_2$.

**13.** Médicament contenant au moins un dérivé de cyclohexane spirocyclique selon l'une quelconque des revendications 1 à 9, et contenant éventuellement des additifs et/ou adjuvants appropriés et/ou éventuellement d'autres agents actifs.

**14.** Dérivé de cyclohexane spirocyclique selon l'une quelconque des revendications 1 à 9, destiné à être utilisé pour le traitement de la douleur, notamment de la douleur aigue, neuropathique ou chronique, des états d'anxiété, du stress et des syndromes associés avec le stress, des dépressions, de l'épilepsie, de la maladie d'Alzheimer, de la démence sénile, des dysfonctionnements cognitifs généraux, des difficultés d'apprentissage et de mémorisation (en tant que nootropique), des phénomènes de sevrage, de l'abus de et/ou de la dépendance à l'alcool et/ou aux drogues et/ou aux médicaments, des dysfonctionnements sexuels, des maladies cardiovasculaires, de l'hypotension, de l'hypertension, des acouphènes, du prurit, de la migraine, de la surdité, du manque de motilité intestinale, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthésique ou pour la co-administration lors du traitement avec un analgésique opioïde ou avec un anesthésique, pour la diurèse ou l'antinatriurèse, l'anxiolyse, pour la modulation de l'activité motrice, pour la modulation de la distribution des neurotransmetteurs et le traitement des maladies neurodégénératives associées, pour le traitement des phénomènes de sevrage et/ou pour la réduction du potentiel d'accoutumance des opioïdes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004043967 A


### In der Beschreibung aufgeführte Nicht-Patentliteratur


- **MEUNIER et al.** *Nature,* 1995, vol. 377, 532-535
- **REINSCHEID et al.** *Science,* 1995, vol. 270, 792-794
- **MOGIL et al.** *Neuroscience,* 1996, vol. 75, 333-337
- **JENCK et al.** *Proc. Natl. Acad. Sci. USA,* 1997, vol. 94, 14854-14858
- **KING et al.** *Neurosci. Lett.,* 1997, vol. 223, 113-116
- **ABDULLA ; SMITH.** *J. Neurosci.,* 1998, vol. 18, 9685-9694
- **MANABE et al.** *Nature,* 1997, vol. 394, 577-581
- **NISHI et al.** *EMBO J.,* 1997, vol. 16, 1858-1864
- **VON CALO et al.** *Br.J. Pharmacol.,* 2000, vol. 129, 1261-1283
- *Exp. Op. Dug Disc.,* 2007, vol. 2, 145
- **KATRITZKY et al.** *Synthesis,* 1989, 66-69
- **JIRKOVSKY et al.** *J. Heterocycl. Chem.,* 1975, vol. 12, 937-940
- **BECK et al.** *J. Chem. Soc. Perkin,* 1992, vol. 1, 813-822
- **SHINADA et al.** *Tetrahedron Lett.,* 1996, vol. 39, 7099-7102
- **GARDEN et al.** *Tetrahedron,* 2002, vol. 58, 8399-8412
- **LEDNICER et al.** *J. Med. Chem.,* 1980, vol. 23, 424-430
- **BANDINI et al.** *J. Org. Chem.,* 2002, vol. 67 (15), 5386-5389
- **DAVIS et al.** *J.Med.Chem.,* 1992, vol. 35 (1), 177-184
- **YAMAGISHI et al.** *J.Med.Chem.,* 1992, vol. 35 (11), 2085-2094
- **GLEAVE et al.** *Bioorg.Med.Chem.Lett.,* 1998, vol. 8 (10), 1231-1236
- **SANDMEYER.** *Helv.Chim.Acta,* 1919, vol. 2, 239
- **KATZ E.** *J. Med. Chem.,* 1988, vol. 31 (6), 1244-1250
- **BAC et al.** *Tetrahedron Lett.,* 1988, vol. 29, 2819
- **MA et al.** *J. Org. Chem.,* 2001, vol. 66, 4525
- **KATO et al.** *J. Fluorine Chem.,* 1999, vol. 99 (1), 5-8
- **S. J. GARDEN ; R. B. DA SILVA ; A. C. PINTO.** *Tetrahedron,* 2002, vol. 58, 8399-8412
- **VON ARDATI et al.** *Mol. Pharmacol.,* 1997, vol. 51, 816-824